# EUROPEAN PATENT APPLICATION

(11) **EP 2 650 362 A2**
(43) Date of publication of application: **16.10.2013**
(21) Application number: 11838278.7
(22) Date of filing: 01.11.2011
(51) Int. Cl.: C12N 7/08, A61K 39/00

(54) **NOVEL VACCINES AGAINST THE A/H1N1 PANDEMIC FLU VIRUS**

(30) Priority: 02.11.2010 US 409303 P; 31.10.2011 MX 2011011561
(71) Applicant: Zepeda López, Hector Manuel, C.P. 53110 Naucalpan, Estado de Mexico (MX)
(72) Inventor: Zepeda López, Hector Manuel, C.P. 53110 Naucalpan, Estado de Mexico (MX)
(74) Representative: Patentanwälte Bals & Vogel
(86) International application number: PCT/MX2011/000132
(87) International publication number: WO 2012/060678

(57) **Abstract**

The invention relates to influenza virus vaccines, and in particular to an influenza virus which has at least its hemagglutinin gene comprising a D222 mutation, and a oseltamivir resistant neuraminidase gene. In some embodiments the influenza virus vaccine further comprises a matrix protein comprising a mutation that increases specific immune response in humans. The virus is useful for production of vaccines against hypervirulent and oseltamivir-resistant influenza.

## Description

### TECHNICAL FIELD OF THE INVENTION

The present invention relates to to vaccines against influenza virus, and in particular to vaccines against hypervirulent strains of pandemic A/H1N1 influenza virus. The invention provides influenza virus strains useful in the production of influenza vaccine carrying specific mutations in the HA and NA segments.

### BACKGROUND OF THE INVENTION

Influenza is caused by an RNA virus of the orthomyxoviridae family. There are three types of these viruses and they cause three different types of influenza: type A, B and C. Influenza virus type A viruses infect mammals (humans, pigs, ferrets, horses) and birds. This is very important to mankind, as this is the type of virus that has caused worldwide pandemics. Influenza virus type B (also known simply as influenza B) infects only humans. It occasionally causes local outbreaks of flu. Influenza C viruses also infect only humans. They infect most people when they are young and rarely cause serious illness.

Influenza A viruses infect a wide variety of mammals, including man, horses, pigs, ferrets and birds. The main human pathogen, associated with epidemics and pandemics. There are at least 15 known hemagglutinin (H) serotypes and 9 known neuraminidase (N) serotypes. Pigs and birds are believed to be particularly important reservoirs, generating pools of genetically and antigenically diverse viruses which get transferred back to the human population via close contact between humans and animals. Influenza B viruses infect mammals only and cause disease, but generally not as severe as A types. Unlike influenza A viruses, influenza B viruses do not have distinguishable serotypes. Influenza C viruses also infect mammals only, but rarely cause disease. They are genetically and morphologically distinct from A and B types.

In contrast to many other viruses, the influenza genome is composed of RNA rather than DNA. The genome comprises eight segments, and only virus particles containing all eight segments are viable. (Steinhauer DA, Skehel JJ., Genetics of influenza viruses. Ann Rev Genet. 2002 36: 305-332).

There are 4 antigens present in the influenza virus, the hemagglutinin (HA), neuraminidase (NA), nucleocapsid (NA), the matrix (M) and the nucleocapsid proteins (NP). The NP is a type-specific antigen which occurs in 3 forms, A, B and C, which provides the basis for the classification of human influenza viruses. The matrix protein (M protein) surrounds the nucleocapsid and makes up 35-45% of the particle mass. Two surface glycoproteins are seen on the surface as rod-shaped projections. The hemagglutinin (HA) is made up of 2 subunits, HA1 and HA2. HA mediates the attachment of the virus to the cellular receptor. Neuraminidase (NA) molecules are present in lesser quantities in the envelope. Circulating human strains are notorious for their tendency to accumulate mutations from one year to the next and cause recurrent epidemics.

Influenza virus is one of a rare few viruses that has its genome in separate segments (eight). The segmented nature of the genome also allows for the exchange of entire genes between different viral strains which increases the potential for recombinants to form (by interchange of gene segments if two different viruses infect the same cell). That is, if a cell is infected with two different strains of influenza, it is possible for the segments to re-assort to create new viruses that have segments from each original virus. This may contribute to the rapid development of new flu strains in nature. New genomes can be assembled as a result of mixing between two viruses. The process can also be duplicated in the laboratory (used for making vaccine strains). Avian and human strains recombining in pigs in the Far East may permit virulent human strains to evolve.

The genetic plasticity of influenza viruses also has serious potential implications regarding vaccine design, pathogenicity, and the capacity for novel viruses to emerge from natural reservoirs and cause global pandemics.

Pandemic (H1N1) 2009 virus has evolved worldwide, shifting from an initial mixed clade pattern to the predominance of one clade (clade 7) during the course of the pandemic. The virus constituting this clade was therefore responsible for most of the pandemic burden worldwide. The mutation S203T in the hemagglutinin (HA) protein sequence is specific to clade 7 isolates. (Valli MB et al., Evolutionary pattern of pandemic influenza (H1N1) 2009 virus in the late phases of the 2009 pandemic. PLoS Curr Influenza. 2010 March 3: RRN1149).

Among clade 7 isolates comprising S203T mutation, a change in the hemagglutinin HA1 gene sequence at the amino acid corresponding to position 222 (225 in H3 numbering), where aspartic acid (D) is changed to glycine (G), is associated with severe clinical outcomes. The D222G mutation was noted in H1N1 pdm global isolates in GenBank at different time points during April-September, 2009. (Chen GW, Shih SR (2009) Genomic signatures of influenza A pandemic (H1N1) 2009 virus. Emerg Infect Dis).

The Influenza vaccine is an annual vaccine to protect against the highly variable influenza virus. Each injected seasonal influenza vaccine contains three influenza viruses: one A (H3N2) virus, the 2009 pandemic H1N1 virus, and one influenza B virus. (www.cdc.gov/vaccines/pubs/vis/downloads/vis-flu.pdf). However, the pandemic flu vaccine is only weakly effective against influenza H1N1 viruses carrying the D222G mutation in the HA protein.

Therefore, viral strains and virus derived antigens that generate a robust antibody titer against D222G variants of H1N1, are urgently needed.

### SUMMARY OF THE INVENTION

The present invention relates to methods and compositions for preparing vaccines against hypervirulent and/or oseltamivir-resistant versions of the pandemic influenza A H1N1 virus. vaccines compositions comprising mutations in the D222 residue of HA and S275 residue ofNA are provided.

This invention provides an influenza virus vaccine comprising a influenza virus which has at least: a hemagglutinin (HA) gene segment comprising an amino acid sequence of SEQ ID NO: 34 derived from virus 1 isolate of pandemic influenza A H1/N1 influenza virus, and a neuraminidase (NA) gene segment comprising an amino acid sequence of SEQ ID NO: 39 derived from virus 1 isolate of pandemic influenza A H1/N1 influenza virus.

In one embodiment, the HA gene segment comprises a amino acid sequence encoded by the nucleotide sequence of segment 4 of virus 1 (SEQ ID NO: 4). In some versions the HA gene segment comprises a clade 7 specific HA sequence of pandemic influenza A H1/N1 influenza virus.

In one embodiment, the HA gene segment comprises a mutation at D222 residue of wild type pandemic influenza A H1/N1 influenza virus. In one embodiment, the HA gene segment comprises a D222E mutation. In some embodiments, the HA gene segment comprises a S203T mutation relative to wild type pandemic influenza A H1/NI influenza virus.

The invention relates to vaccines wherein the HA gene segment further comprises one or more of P83S and I321V mutations.

In one embodiment, the NA gene segment comprises a amino acid sequence encoded by the nucleotide sequence of segment 6 of virus 1 (SEQ ID NO: 6). The NA gene segment comprises a Y275H mutation relative to wild type pandemic influenza A H1/N1 influenza virus.

The invention relates to vaccines wherein the NA gene segment further comprises one or more of I106V, D248N mutations.

The vaccine of the invention may further comprise: c) a matrix (MA) gene segment encoding an M1 peptide comprising an amino acid sequence of SEQ ID NO: 50 derived from virus I isolate of pandemic influenza A H1/N1 influenza virus.

In one embodiment, the MA gene segment comprises a amino acid sequence encoded by the nucleotide sequence of segment 7 of virus 1 (SEQ ID NO: 7).

In some embodiments, the matrix (MA) gene segment encoding an M2 peptide comprising an amino acid sequence of SEQ ID NO: 51 derived from virus 1 isolate of pandemic influenza A H1/N1 influenza virus. In some embodiments, the M1 peptide comprises a A198P mutation.

The invention relates to a method for immunizing a patient against influenza virus comprising the step of administering an influenza vaccine disclosed herein to the patient.

The invention relates to a method for immunizing a patient against a hypervirulent influenza virus comprising the step of administering an influenza vaccine disclosed herein to the patient.

The invention relates to a method for immunizing a patient against a oseltamivir-resistant influenza virus comprising the step of administering an influenza vaccine disclosed herein to the patient.

The invention relates to a vaccine disclosed herein, wherein the vaccine is a reassortant vaccine. In some embodiments, the reassortant influenza virus is obtained by classical reassortment.

The invention relates to a vaccine disclosed herein, which is a live attenuated vaccine or an inactivated vaccine. In some embodiments, the vaccine is formulated for oral or intranasal administration.

The invention relates to a method of preparing a vaccine for immunization of a subject against a hypervirulent influenza virus strain, comprising the step of mixing an influenza virus disclosed herein with a carrier, and optionally an adjuvant.

These and other aspects will become apparent from the following description of the preferred embodiment taken in conjunction with the following drawings, although variations and modifications therein may be affected without departing from the spirit and scope of the novel concepts of the disclosure.

### BRIEF DESCRIPTION OF THE DRAWINGS

The following drawings form part of the present specification and are included to further demonstrate certain aspects of the present disclosure, the inventions of which can be better understood by reference to one or more of these drawings in combination with the detailed description of specific embodiments presented herein. The patent or application file contains at least one drawing executed in color. Copies of this patent or patent application publication with color drawing(s) will be provided by the Office upon request and payment of the necessary fee.

FIGURE 1 shows examples of viral load quantification by real time PCR.

FIGURE 2 shows sample arrangement for AH1N1 real time PCR detection.

FIGURES 3A and 3B show graphical views of Light Cycler 2.0 real time thermocycler interface showing fluorescence curves. Fig. 3A) Clinical Sample. Fig. 3B) RNAseP (RP) detection.

FIGURE 4A shows the adaptation of the influenza virus HZFLUJAL in VERO cell line. The virus was able to replicate efficiently until 4 subcultures in VERO cells. FIGURE 4B shows the stages for adaptation of the influenza virus HZFLUJAL in MDCK cell line. The Influenza virus HZFLUJA was able to replicate efficiently after 3 subcultures in MDCK cells.

FIGURE 5 shows total antibodies against influenza p/AH1N1 detected by ELISA.

FIGURE 6A shows 1gM antibodies against influenza p/AH1N1 detected in serum by ELISA.

FIGURE 6B shows IgG antibodies against influenza p/AH1N1 detected in serum by ELISA.

FIGURE 7A shows IgG_{2A} antibodies against Influenza p/AH1N1 detected in serum by ELISA. FIGURE 7B shows IgG_{2A} dose-response against Influenza p/AH1N1 detected in serum by ELISA.

FIGURE 8A shows IgA antibodies against Influenza p/AH1N1 detected in respiratory mucosa by ELISA. FIGURE 8B shows IgA dose-response against Influenza p/AH1N1 detected in respiratory mucosa by ELISA.

### DETAILED DESCRIPTION OF THE INVENTION

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way.

Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control. The following references provide one of skill with a general definition of many of the terms used in this invention: Singleton et al., Dictionary of Microbiology and Molecular Biology (2nd Ed. 1993); The Cambridge Dictionary of Science and Technology (Walker ed., Cambridge University Press. 1990); The Glossary of Genetics, 5th ed., R. Rieger et al. (eds.), Springer Verlag (1991); and Hale & Margham, The Harper Collins Dictionary of Biology (1991).

Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, e.g., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

Influenza A viruses contain genomes composed of eight separate segments of negative-sense RNA as shown in Table I below. (Steinhauer DA, Skehel JJ., Genetics of influenza viruses. Ann Rev Genet. 2002 36: 305-332). Circulating human strains are notorious for their tendency to accumulate mutations from one year to the next and cause recurrent epidemics. However, the segmented nature of the genome also allows for the exchange of entire genes between different viral strains. The genetic plasticity of influenza viruses also has serious potential implications regarding vaccine design, pathogenicity, and the capacity for novel viruses to emerge from natural reservoirs and cause global pandemics.

| Table 1. Influenza virus genome consists of s/s (-)sense RNA in 8 segments | | | |
|---|---|---|---|
| Segment: | Size(nt) | Polypeptide(s) | Function |
| 1 | 2341 | PB2 | Transcriptase: cap binding |
| 2 | 2341 | PB1 | Transcriptase: elongation |
| 3 | 2233 | PA | Transcriptase: protease (?) |
| 4 | 1778 | HA | Hemagglutinin |
| 5 | 1565 | NP | Nucleoprotein: RNA binding |
| 6 | 1413 | NA | Neuraminidase: release of virus |
| 7 | 1027 | M1 | Matrix protein: component of virion |
| | | M2 | Integral membrane protein |
| 8 | 890 | NS1 | Non-structural: nuclear |
| | | NS2 | Non-structural: nucleus + cytoplasm |

A total of 950 viruses were isolated by cell culture, from which, 193 Neuraminidase (NA) and 197 Hemaglutinin (HA) partial gene sequences were obtained through capillary sequencing. Since HA and NA are the most variable sequences in AH1N1, an *in silico* analysis (phylogenetic analysis and BLAST) showed that all 386 (HA and NA sequences) could be grouped in 20 clusters, therefore; the complete genome of one representative member of each cluster was pyrosequenced in a 454 Titanium Roche Pyrosequencing equipment (ROCHE). A total of 20 complete AH 1N1 genomes were obtained, each AH1N1 consists of 8 genes.

Sequencing data showed four viral isolates with three different mutations: (i) a pAH1N1 virus possessing mutations in NA (oseltamivir^{R}), HA (Hypervirulence) and Matrix segments (Virus 1); (ii) a pAH1N1 virus possessing mutations in NA and Matrix segments (Virus 2); (iii) a pAH1NI virus possessing mutations in matrix (MA) segment (Virus 3); and (iv) a pAH1N1 virus possessing mutations in neuraminidase (NA) segment (Virus 4).

A change in the hemagglutinin HA1 gene sequence at the amino acid corresponding to position 222 (225 in H3 numbering), where aspartic acid (D) is changed to glycine (G), is associated with severe clinical outcomes. The D222G mutation was noted in H1N1pdm global isolates in GenBank at different time points during April-September, 2009. (Chen GW, Shih SR (2009) Genomic signatures of influenza A pandemic (H1N1) 2009 virus. Emerg Infect Dis).

The preferential binding of influenza virus to sialic acid-α2,3-galactose (α2,3 receptor) or sialic acid-α2,6-galactose (α2,6 receptors) may determine its tropism as α2,3 and α2,6 receptors are dominant on lower and upper respiratory cells respectively (Shinya K, Ebina M, Yamada S, Ono M, Kasai N, Kawaoka Y. Avian flu: influenza virus receptors in the human airway. Nature. 2006; 440(7083):435-6).

Recent glycan microarray analysis suggested that the hemagglutinin (HA) D222G substitution could cause a shift from α2,6 receptors to the mixed α2,3/α2,6 receptors specificity which might increase binding to α2,3 receptors and contribute to severity of disease. (Stevens J, Blixt O, Glaser L, Taubenberger JK, Palese P, Paulson JC, et al. Glycan microarray analysis of the hemagglutinins from modern and pandemic influenza viruses reveals different receptor specificities. J Mol Biol. 2006;355(5):1143-55). This position seems to influence receptor binding specificity, and the D to G difference between two 1918 virus variants (*Id*.) correlates to a shift from α2-6-linked sialic acid preference to a dual α2-3/α2-6 specificity, i.e. it conceivably could make the virus more prone to infect deeper in the airways where cells expressing α2-3-receptors are more abundant, thus causing hyper-virulence.

However, no distinct phylogenetic clusterings of the severe cases with D222G substitution have been observed (Mak GC et al., Association of D222G substitution in hemagglutinin of 2009 pandemic influenza A (H1N1) with severe disease. Eurosurveillance, Volume 15, Issue 14, 08 April 2010)

All of the viruses that were found to contain changes in HA1 position 222 belong to a sub-clade of pandemic H 1N1 viruses characterized by the HA1 substitution S203T (characteristic of clade 7), which is also surrounded by an amino acid pocket which exposes an antigenic region specific for this hyper-virulent and the rest of the AH1N1 strains. This sub-clade forms the great majority of pandemic viruses in Europe and the rest of northern hemisphere. Regarding the southern hemisphere, this sub-clade is quite well-spread as well.

Based on currently available data shared with WHO, the prevalence of D222G substitution is less than 1.8% (52 detections among more than 2755 HA sequences). Of 364 fatal cases analysed to date, viruses from 26 cases (7.1%) had the D222G substitution. The clinical information about potential underlying medical conditions in these cases is limited. The pandemic (H1N1) 2009 viruses with D222G substitution have been antigenically similar to the A/California/7/2009 (H1N1) virus, the WHO-recommended vaccine virus.

Among the same above mentioned viruses A (H1N1)2009 (i) a Gly222 mutation in the hemagglutinin (HA) and (ii) a Tyr275 mutation in the neuraminidase (NA) associated with a resistance to Oseltamivir (McKimm-Breschkin JL. Resistance of influenza viruses to neuraminidase inhibitors-a review. Antiviral Res 2000; 47:1-17; Collins PJ et al. Structural basis for oseltamivir resistance of influenza viruses. Vaccine 27 (2009) 6317-6323) was found. Since both mutations are related to important phenotypes (hyper-virulence and Oseltamivir resistance), and antigenic regions are associated with those mutations, the present vaccine candidate can efficiently recognize both the mutant strains and the AH1NI wild type.

Three of the D222G variant viruses carry the H275Y substitution in the neuraminidase (NA) associated with oseltamivir resistance. WHO Report. Preliminary review of D222G amino acid substitution in the hemagglutinin of pandemic influenza A (H1N1) 2009 viruses. 28 December 2009 (www.who.int/csr/resources/publications/swineflu/cp165_2009_2812_review_d222g_amino_aci d_substitution_in_ha_h1n1_viruses.pdf)

The currently available pandemic vaccine also recognizes the hyper-virulent and oseltamivir resistance mutants, however, although the mutation is situated in the receptor binding cavity and not in the antigenic site of the H1 hemagglutinin, a preliminary antigenic characterization performed at the Swedish Institute for Infectious Disease Control and at our laboratory, showed a significant reduced reactivity of the mutant viruses to antiserum against the current pandemic vaccine in comparison to wild type pandemic AH1N1 strain. Thus, the current vaccine is not as efficient as the present vaccine candidate at recognizing these new mutant strains, which are spreading rapidly all over the world.

A mutation in the 3' end of segment 7 (Matrix segment) of the viral genome shows similarity (60%) to Influenza B M segment. This mutation has been previously reported to increase specific immune response in humans and it might confer protection against Influenza B as well. However, cell-mediated immunity, mainly against virus matrix and nucleoprotein antigens, does not protect against infection, but is crucial for clearance of virus and recovery from illness.

| **Table 2.** Genome sequence of pAH1N1 virus possessing mutations in NA (oseltamivir^{R}), HA(Hypervirulence) and Matrix segments (Virus 1) |
|---|
| Segment 1-VIRUS 1 (SEQ ID NO: 1) |
| TATGGAGAGAATAAAAGAACTGAGAGATCTAATGTCGCAGTCCCGCACTCGCGAGA TACTCACTAAGACCACTGTGGACCATATGGCCATAATCAAAAAGTACACATCAGGA AGGCAAGAGAAGAACCCCGCACTCAGAATGAAGTGGATGATGGCAATGAGATACCC AATTACAGCAGACAAGAGAATAATGGACATGATTCCAGAGAGGAATGAACAAGGA CAAACCCTCTGGAGCAAAACAAACGATGCTGGATCAGACCGAGTGATGGTATCACC TCTGGCCGTAACATGGTGGAATAGGAATGGCCCAACAACAAGTACAGTTCATTACC CTAAGGTATATAAAACTTATTTCGAAAAGGTCGAAAGGTTGAAACATGGTACCTTCG GCCCTGTCCACTTCAGAAATCAAGTTAAAATAAGGAGGAGAGTTGATACAAACCCT GGCCATGCAGATCTCAGTGCCAAGGAGGCACAGGATGTGATTATGGAAGTTGTTTTC CCAAATGAAGTGGGGGCAAGAATACTGACATCAGAGTCACAGCTGGCAATAACAAA AGAGAAGAAAGAAGAGCTCCAGGATTGTAAAATTGCTCCCTTGATGGTGGCGTACA TGCTAGAAAGAGAATTGGTCCGTAAAACAAGGTTTCTCCCAGTAGCCGGCGGAACA GGCAGTGTTTATATTGAAGTGTTGCACTTAACCCAAGGGACGTGCTGGGAGCAGATG TACACTCCAGGAGGAGAAGTGAGAAATGATGATGTTGACCAAAGTTTGATTATCGC TGCTAGAAACATAGTAAGAAGAGCAGCAGTGTCAGCAGACCCATTAGCATCTCTCTT GGAAATGTGCCACAGCACACAGATTGGAGGAGTAAGGATGGTGGACATCCTTAGAC AGAATCCAACTGAGGAACAAGCCGTAGACATATGCAAGGCAGCAATAGGGTTGAGG ATTAGCTCATCTTTCAGTTTTGGTGGGTTCACTTTCAAAAGGACAAGCGGATCATCA GTCAAGAAAGAAGAAGAAGTGCTAACGGGCAACCTCCAAACACTGAAAATAAGAG TACATGAAGGGTATGAAGAATTCACAATGGTTGGGAGAAGAGCAACAGCTATTCTC AGAAAGGCAACCAGGAGATTGATCCAGTTGATAGTAAGCGGGAGAGACGAGCAGT CAATTGCTGAGGCAATAATTGTGGCCATGGTATTCTCACAAGAGGATTGCATGATCA AGGCAGTTAGGGGCGATCTGAACTTTGTCAATAGGGCAAACCAGCGACTGAACCCC ATGCACCAACTCTTGAGGCATTTCCAAAAAGATGCAAAAGTGCTTTTCCAGAACTGG GGAATTGAATCCATCGACAATGTGATGGGAATGATCGGAATACTGCCCGACATGAC CCCAAGCACGGAGATGTCGCTGAGAGGGATAAGAGTCAGCAAAATGGGAGTAGAT GAATACTCCAGCACGGAGAGAGTGGTAGTGAGTATTGACCGATTTTTAAGGGTTAG AGATCAAAGAGGGAACGTACTATTGTCTCCCGAAGAAGTCAGTGAAACGCAAGGAA CTGAGAAGTTGACAATAACTTATTCGTCATCAATGATGTGGGAGATCAATGGCCCTG AGTCAGTGCTAGTCAACACTTATCAATGGATAATCAGGAACTGGGAAATTGTGAAA ATTCAATGGTCACAAGATCCCACAATGTTATACAACAAAATGGAATTTGAACCATTT CAGTCTCTTGTCCCTAAGGCAACCAGAAGCCGGTACAGTGGATTCGTAAGGACACTG TTCCAGCAAATGCGGGATGTGCTTGGGACATTTGACACTGTCCAAATAATAAAACTT |
| CTCCCCTTTGCTGCTGCTCCACCAGAACAGAGTAGGATGCAATTTTCCTCATTGACTG TGAATGTGAGAGGATCAGGGTTGAGGATACTGGTAAGAGGCAATTCTCCAGTATTC AATTACAACAAGGCAACCAAACGACTTACAGTTCTTGGAAAGGATGCAGGTGCATT GACTGAAGATCCAGATGAAGGCACATCTGGGGTGGAGTCTGCTGTCCTGAGAGGAT TTCTCATTTTGGGCAAAGAAGACAAGAGATATGGCCCAGCATTAAGCATCAATGAA CTGAGCAATCTTGCAAAAGGAGAGAAAGCTAATGTGCTAATTGGGCAAGGGGACGT AGTGTTGGTAATGAAACGAAAACGGACTCTAGCATACTTACTGACAGCCAGACAG CGACCAAAAGAATTCGGATGGCCATCAATTAGTGTCGAATTGTT |
| Segment 2-VIRUS 1 (SEQ ID NO: 2) |
| TTGAATGGATGTCAATCCGACTCTACTTTTCCTAAAAATTCCAGCGCAAAATGCCAT AAGCACCACATTCCCTTATACTGGAGATCCTCCATACAGCCATGGAACAGGAACAG GATACACCATGGACACAGTAAACAGAACACACCAATACTCAGAAAGGGAAGTG GACGACAAACACAGAGACTGGTGCACCCCAGCTCAACCCGATTGATGGACCACTAC CTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATG GAAGTTGTTCAACAAACAAGGGTAGATAAACTAACTCAAGGTCGCCAGACTTATGA TTGGACATTAAACAGAAATCAACCGGCAGCAACTGCATTGGCCAACACCATAGAAG TCTTTAGATCGAATGGCCTAACAGCTAATGAGTCAGGAAGGCTAATAGATTTCTTAA AGGATGTAATGGAATCAATGAACAAAGAGGAAATAGAGATAACAACCCACTTTCAA AGAAAAAGGAGAGTAAGAGACAACATGACCAAGAAGATGGTCACGCAAAGAACAA TAGGGAAGAAAAAACAAAGACTGAATAAGAGAGGCTATCTAATAAGAGCACTGAC ATTAAATACGATGACCAAAGATGCAGAGAGAGGCAAGTTAAAAAGAAGGGCTATC GCAACACCTGGGATGCAGATTAGAGGTTTCGTATACTTTGTTGAAACTTTAGCTAGG AGCATTTGCGAAAAGCTTGAACAGTCTGGGCTCCCAGTAGGGGGCAATGAAAAGAA GGCCAAACTGGCAAATGTTGTGAGAAAGATGATGACTAATTCACAAGACACAGAGA TTTCTTTCACAATCACTGGGGACAACACTAAGTGGAATGAAAATCAAAATCCTCGAA TGTTCCTGGCGATGATTACATATATCACCAGAAATCAACCCGAGTGGTTCAGAAACA TCCTGAGCATGGCACCCATAATGTTCTCAAACAAAATGGCAAGACTAGGGAAAGGG TACATGTTCGAGAGTAAAAGAATGAAGATTCGAACACAAATACCAGCAGAAATGCT AGCAAGCATTGACCTGAAGTACTTCAATGAATCAACAAAGAAGAAAATTGAGAAAA TAAGGCCTCTTCTAATAGATGGCACAGCATCACTGAGTCCTGGGATGATGATGGGCA TGTTCAACATGCTAAGTACGGTCTTGGGAGTCTCGATACTGAATCTTGGACAAAAGA AATACACCAAGACAATATACTGGTGGGATGGGCTCCAATCATCCGACGATTTTGCTC TCATAGTGAATGCACCAAACCATGAGGGAATACAAGCAGGAGTGGACAGATTCTAC AGGACCTGCAAGTTAGTGGGAATCAACATGAGCAAAAAGAAGTCCTATATAAATAA GACAGGGACATTTGAATTCACAAGCTTTTTTATCGCTATGGATTTGTGGCTAATTTT AGCATGGAGCTACCCAGCTTTGGAGTGTCTGGAGTAAATGAATCAGCTGACATGAG TATTGGAGTAACAGTGATAAAGAACAACATGATAAACAATGACCTTGGACCTGCAA CGGCCCAGATGGCTCTTCAATTGTTCATCAAAGACTACAGATACACATATAGGTGCC |
| ATAGGGGAGACACACAAATTCAGACGAGAAGATCATTTGAGTTAAAGAAGCTGTGG GATCAAACCCAATCAAAGGTAGGGCTATTAGTATCAGATGGAGGACCAAACTTATA CAATATACGGAATCTTCACATTCCTGAAGTCTGCTTAAAATGGGAGCTAATGGATGA TGATTATCGGGGAAGACTTTGTAATCCCCTGAATCCCTTTGTCAGTCATAAAGAGAT TGATTCTGTAAACAATGCTGTGGTAATGCCAGCCCATGGTCCAGCCAAAAGCATGGA ATATGATGCCGTTGCAACTACACATTCCTGGATTCCCAAGAGGAATCGTTCTATTCT CAACACAAGCCAAAGGGGAATTCTTGAGGATGAACAGATGTACCAGAAGTGCTGCA ATCTATTCGAGAAATTTTTCCCTAGCAGTTCATATAGGAGACCGGTTGGAATTTCTA GCATGGTGGAGGCCATGGTGTCTAGGGCCCGGATTGATGCCAGGGTCGACTTCGAG TCTGGACGGATCAAGAAAGAAGAGTTCTCTGAGATCATGAAGATCTGTTCCACCATT GAAGAACTCAGACGGCAAAAATAATGAATTTAACTTGTCCTTCATGAAA |
| Segment 3-VIRUS 1 (SEQ ID NO: 3) |
| TCCAAAATGGAAGACTTTGTGCGACAATGCTTCAATCCAATGATCGTCGAGCTTGCG GAAAAGGCAATGAAAGAATATGGGGAAGATCCGAAAATCGAAACTAACAAGTTTG CTGCAATATGCACACATTTGGAAGTTTGTTTCATGTATTCGGATTTCCATTTCATCGA CGAACGGGGTGAATCAATAATTGTAGAATCTGGTGACCCGAATGCACTATTGAAGC ACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGAT TACAAAGAGAACCGGTTCATTGAAATTGGAGTAACACGGAGGGAAGTCCACATATA TTACCTAGAGAAAGCCAACAAAATAAAATCTGAGAAGACACACATTCACATCTTTTC ATTCACTGGAGAGGAGATGGCCACCAAAGCGGACTACACCCTTGACGAAGAGAGCA GGGCAAGAATCAAAACTAGGCTTTTCACTATAAGACAAGAAATGGCCAGTAGGAGT CTATGGGATTCCTTTCGTCAGTCCGAAAGAGGCGAAGAGACAATTGAAGAAAAATT TGAGATTACAGGAACTATGCGCAAGCTTGCCGACCAAAGTCTCCCACCGAACTTCTC CAGCCTTGAAAACTTTAGAGCCTATGTAGATGGATTCGAGCCGAACGGCTGCATTGA GGGCAAGCTTTCCCAAATGTCAAAAGAAGTGAACGCCAAAATTGAACCATTCTTGA GGACGACACCACGCCCCCTCAGATTGCCTGATGGGCCTCTTTGCCATCAGCGGTCAA AGTTCCTGCTGATGGATGCTCTGAAATTAAGTATTGAAGACCCGAGTCACGAGGGG GAGGGAATACCACTATATGATGCAATCAAATGCATGAAGACATTCTTTGGCTGGAA AGAGCCTAACATAGTCAAACCACATGAGAAAGGCATAAATCCCAATTACCTCATGG CTTGGAAGCAGGTGCTAGCAGAGCTACAGGACATTGAAAATGAAGAGAAGATCCCA AGGACAAAGAACATGAAGAGAACAAGCCAATTGAAGTGGGCACTCGGTGAAAATA TGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATT CAATAAGGCATGTGAATTGACTGATTCAAGCTGGATAGAACTTGATGAAATAGGAG AAGATGTTGCCCCGATTGAACATATCGCAAGCATGAGGAGGAACTATTTTACAGCA GAAGTGTCCCACTGCAGGGCTACTGAATACATAATGAAGGGAGTGTACATAAATAC GGCCTTGCTCAATGCATCCTGTGCAGCCATGGATGACTTTCAGCTGATCCCAATGAT AAGCAAATGTAGGACCAAAGAAGGAAGACGGAAAACAAACCTGTATGGGTTCATTA |
| TAAAAGGAAGGTCTCATTTGAGAAATGATACTGATGTGGTGAACTTTGTAAGTATGG AGTTCTCACTCACTGACCCGAGACTGGAGCCACACAAATGGGAAAAATACTGTGTTC TTGAAATAGGAGACATGCTCTTGAGGACTGCGATAGGCCAAGTGTCGAGGCCCATG TTCCTATATGTGAGAACCAATGGAACCTCCAAGATCAAGATGAAATGGGGCATGGA ACTGAGGCGCTGCCTTCTTCAGTCTCTTCAGCAGATTGAGAGCATGATTGAGGCCGA GTCTTCTGTCAAAGAGAAAGACATGACCAAGGAATTCTTTGAAAACAAATCGGAAA CATGGCCAATCGGAGAGTCACCCAGGGGAGTGGAGGAAGGCTCTATTGGGAAAGTG TGCAGGACCTTACTGGCAAAATCTGTATTCAACAGTCTATATGCGTCTCCACAACTT GAGGGGTTTTCGGCTGAATCGAGAAAATTGCTTCTCATTGTTCAGGCACTTAGGGAC AACCTGGAACCTGGAACCTTCGATCTTGGGGGGCTATATGAAGCAATCGAGGAGTG CCTGATTAATGATCCCTGGGTTTTGCTTAATGCATCTTGGTTCAACTCCTTCCTCACA CATGCACTGAAGTAGTTGTGGCAATGCTAC |
| Segment 4-VIRUS 1 (SEQ ID NO: 4) |
| AGCAAAAGCAGGGGAAAACAAAAGCAACAAAAATGAAGGCAATACTAGTAGTTCT GCTATACACATTTGCAACCGCAAATGCAGACACATTATGTATAGGTTATCATGCGAA CAATTCAACAGACACTGTAGACACAGTACTAGAAAAGAATGTAACAGTAACACACT CTGTTAACCTTCTAGAAGACAAGCATAACGGGAAACTATGCAAACTAAGAGGGGTA GCCCCATTGCATTTGGGTAAATGTAACATTGCTGGCTGGATCCTGGGAAATCCAGAG TGTGAATCACTCTCCACAGCAAGCTCATGGTCCTACATTGTGGAAACATCTAGTTCA GACAATGGAACGTGTTACCCAGGAGATTTCATCGATTATGAGGAGCTAAGAGAGCA ATTGAGCTCAGTGTCATCATTTGAAAGGTTTGAGATATTCCCCAAGACAAGTTCATG GCCCGATCATGACTCGAACAAAGGTGTAACGGCAGCATGTCCTCATGCTGGAGCAA AAAGCTTCTACAAAAATTTAATATGGCTAGTTAAAAAAGGAAATTCATACCCAAAG CTCAGCAAATCCTACATTAATGATAAAGGGAAAGAAGTCCTCGTGCTATGGGGCATT CACCATCCATCTACTAGTGCTGACCAACAAAGTCTCTATCAGAATGCAGATGCATAT GTTTTTGTGGGGACATCAAGATACAGCAAGAAGTTCAAGCCGGAAATAGCAATAAG ACCCAAAGTGAGGGAACGAGAAGGAGAATGAACTATTACTGGACACTAGTAGAG CCGGGAGACAAAATAACATTCGAAGCAACTGGAAATCTAGTGGTACCGAGATATGC ATTCGCAATGGAAAGAAATGCTGGATCTGGTATTATCATTTCAGATACACCAGTCCA CGATTGCAATACAACTTGTCAGACACCCAAAGGTGCTATAAACACCAGCCTCCCATT TCAGAATATACATCCGATCACAATTGGAAAATGTCCAAAATATGTAAAAAGCACAA AATTGAGACTGGCCACAGGATTGAGGAATGTCCCGTCTATTCAATCTAGAGGCCTAT TTGGGGCCATTGCCGGTTTCATTGAAGGGGGGTGGACAGGGATGGTAGATGGATGG TACGGTTATCACCATCAAAATGAGCAGGGGTCAGGATATGCAGCCGACCTGAAGAG CACACAGAATGCCATTGACGAGATTACTAACAAAGTAAATTCTGTTATTGAAAAGAT GAATACACAGTTCACAGCAGTAGGTAAAGAGTTCAACCACCTGGAAAAAAGAATAG AGAATTTAAATAAAAAAGTTGATGATGGTTTCCTGGACATTTGGACTTACAATGCCG AACTGTTGGTTCTATTGGAAAATGAAAGAACTTTGGACTACCACGATTCAAATGTGA AGAACTTATATGAAAAGGTAAGAAGCCAGTTAAAAAACAATGCCAAGGAAATTGGA |
| AACGGCTGCTTTGAATTTACCACAAATGCGATAACACGTGCATGGAAAGTGTCAAA AATGGACTTATGACTACCCAAAATACTCAGAGGAAGCAAAATTAAACAGAGAAGA AATAGATGGGGTAAAGCTGGAATCAACAAGGATTTACCAGATTTTGGCGATCTATTC AACTGTCGCCAGTTCATTGGTACTGGTAGTCTCCCTGGGGGCAATCAGTTTCTGGAT GTGCTCTAATGGGTCTCTACAGTGTAGAATATGTATTTAACATTAGGATTTCAGAAG C |
| Segment 5-VIRUS 1 (SEQ ID NO: 5) |
| ATCAATCACTGAGTGACATCGAAGCCATGGCGTCTCAAGGCACCAAACGATCATAT GAACAAATGGAGACTGGTGGGGAGCGCCAGGATGCCACAGAAATCAGAGCATCTGT CGGAAGAATGATTGGTGGAATCGGGAGATTCTACATCCAAATGTGCACTGAACTCA AACTCAGTGATTATGATGGACGACTAATCCAGAATAGCATAACAATAGAGAGGATG GTGCTTTCTGCTTTTGATGAGAGAAGAAATAAATACCTAGAAGAGCATCCCAGTGCT GGGAAGGACCCTAAGAAAACAGGAGGACCCATATATAGAAGAATAGACGGAAAGT GGATGAGAGAACTCATCCTTTATGACAAAGAAGAAATAAGGAGAGTTTGGCGCCAA GCAAACAATGGCGAAGATGCAACAGCAGGTCTTACTCATATCATGATTTGGCATTCC AACCTGAATGATGCCACATATCAGAGAACAAGAGCGCTTGTTCGCACCGGAATGGA TCCCAGAATGTGCTCTCTAATGCAAGGTTCAACACTTCCCAGAAGGTCTGGTGCCGC AGGTGCTGCGGTGAAAGGAGTTGGAACAATAGCAATGGAGTTAATCAGAATGATCA AACGTGGAATCAATGACCGAAATTTCTGGAGGGGTGAAAATGGACGAAGGACAAG GGTTGCTTATGAAAGAATGTGCAATATCCTCAAAGGAAAATTTCAAACAGCTGCCCA GAGGGCAATGATGGATCAAGTAAGAGAAAGTCGAAACCCAGGAAACGCTGAGATT GAAGACCTCATTTTCCTGGCACGGTCAGCACTCATTCTGAGGGGATCAGTTGCACAT AAATCCTGCCTGCCTGCTTGTGTGTATGGGCTTGCAGTAGCAAGTGGGCATGACTTT GAAAGGGAAGGGTACTCACTGGTCGGGATAGACCCATTCAAATTACTCCAAAACAG CCAAGTGGTCAGCCTGATGAGACCAAATGAAAACCCAGCTCACAAGAGTCAATTGG TGTGGATGGCATGCCACTCTGCTGCATTTGAAGATTTAAGAGTATCAAGTTTCATAA GAGGAAAGAAAGTGATTCCAAGAGGAAAGCTTTCCACAAGAGGGGTCCAGATTGCT TCAAATGAGAATGTGGAAACCATGGACTCCAATACCCTGGAACTAAGAAGCAGATA CTGGGCCATAAGGACCAGGAGTGGAGGAAATACCAATCAACAAAGGCATCCGCA GGCCAGATCAGTGTGCAGCCTACATTCTCAGTGCAGCGAAATCTCCCTTTTGAAAGA GCAACCGTTATGGCAGCATTCAGCGGGAACAATGAAGGACGGACATCCGACATGCG AACAGAAGTTATAAGAATGATGGAAAGTGCAAAGCCAGAAGATTTGTCCTTCCAGG GGCGGGGAGTCTTCGAGCTCTCGGACGAAAAGGCAACGAACCCGATCGTGCCTTCC TTTGACATGAGTAATGAAGGGTCTTATTTCTTCGGAGACAATGCAGAGGAGTATGAC AGTTGAGGAAAAATACCCTTGTTTCTACT |
| Segment 6-VIRUS 1 (SEQ ID NO: 6) |
| ATGAATCCAAACCAAAAGATAATAACCATTGGTTCGGTCTGTATGACGATTGGAATG GCTAACTTAATATTACAAATTGGAAACATAATCTCAATATGGATTAGCCACTCAATT |
| CAACTTGGGAATCAAAATCAGATTGAAACATGCAATCAAAGCGTCATTACTTATGA AAACAACACTTGGGTAAATCAGACATATGTTAACATCAGCAACACCAACTTTGCTGC TGGACAGTCAGTGGTTTCCGTGAAATTAGCGGGCAATTCCTCTCTCTGCCCTGTTAGT GGATGGGCTATATACAGTAAAGACAACAGTATAAGAATCGGTTCCAAGGGGGATGT GTTTGTCATAAGGGAACCATTCATATCATGCTCCCCCTTGGAATGCAGAACCTTCTTC TTGACTCAAGGGGCCTTGCTAAATGACAAACATTCCAATGGAACCATTAAAGACAG GAGCCCATATCGAACCCTAATGAGCTGTCCTATTGGTGAAGTTCCCTCTCCATACAA CTCAAGATTTGAGTCAGTCGCTTGGTCAGCAAGTGCTTGTCATGATGGCATCAATTG GCTAACAATTGGAATTTCTGGCCCAGACAATGGGGCAGTGGCTGTGTTAAAGTACA ACGGCATAATAACAGACACTATCAAGAGTTGGAGAAACAATATATTGAGAACACAA GAGTCTGAATGTGCATGTGTAAATGGTTCTTGCTTTACTGTAATGACCGATGGACCA AGTGATGGACAGGCCTCATACAAGATCTTCAGAATAGAAAAGGGAAAGATAGTCAA ATCAGTCGAAATGAATGCCCCTAATTATTACTATGAGGAATGCTCCTGTTATCCTGA TTCTAGTGAAATCACATGTGTGTGCAGGGATAACTGGCATGGCTCGAATCGACCGTG GGTGTCTTTCAACCAGAATCTGGAATATCAGATAGGATACATATGCAGTGGGATTTT CGGAGACAATCCACGCCCTAATGATAAGACAGGCAGTTGTGGTCCAGTATCGTCTA ATGGAGCAAATGGAGTAAAAGGATTTTCATTCAAATACGGCAATGGTGTTTGGATA GGGAGAACTAAAAGCATTAGTTCAAGAAACGGTTTTGAGATGATTTGGGATCCGAA CGGATGGACTGGGACAGACAATAACTTCTCAATAAAGCAAGATATCGTAGGAATAA ATGAGTGGTCAGGATATAGCGGGAGTTTTGTTCAGCATCCAGAACTAACAGGGCTG GATTGTATAAGACCTTGCTTCTGGGTTGAACTAATCAGAGGGCGACCCAAAGAGAA CACAATCTGGACTAGCGGGAGCAGCATATCCTTTTGTGGTGTAAACAGTGACACTGT GGGTTGGTCTTGGCCAGACGGTGCTGAGTTGCCATTTACCATTGACAAGTAA |
| Segment 7-VIRUS 1 (SEQ ID NO: 7) |
| ATGAGTCTTCTAACCGAGGTCGAAACGTACGTTCTTTCTATCATCCCGTCAGGCCCC CTCAAAGCCGAGATCGCGCAGAGACTGGAAAGTGTCTTTGCAGGAAAGAACACAGA TCTTGAGGCTCTCATGGAATGGCTAAAGACAAGACCAATCTTGTCACCTCTGACTAA GGGAATTTTAGGATTTGTGTTCACGCTCACCGTGCCCAGTGAGCGAGGACTGCAGCG TAGACGCTTTGTCCAAAATGCCCTAAATGGGAATGGGGACCCGAACAACATGGATA GAGCAGTTAAACTATACAAGAAGCTCAAAAGAGAAATAACGTTCCATGGGGCCAAG GAGGTGTCACTAAGCTATTCAACTGGTGCACTTGCCAGTTGCATGGGCCTCATATAC AACAGGATGGGAACAGTGACCACAGAAGCTGCTTTTGGTCTAGTGTGTGCCACTTGT GAACAGATTGCTGATTCACAGCATCGGTCTCACAGACAGATGGCTACTACCACCAAT CCACTAATCAGGCATGAAAACAGAATGGTGCTGGCTAGCACTACGGCAAAGGCTAT GGAACAGATGGCTGGATCGAGTGAACAGAAAGGAGAAGACGTCCAAAAGCTGGCA GAAGAGCTGCAAAGCAACATTGGAGTATTGAGATCTCTTGGAGCAAGTCAAAAGAG TGGGGAAGGAATTGCAAAGGATGTAATGGAAGTGCTAAAGCAGAGCTCTATGGGAA ATTCAGCTCTTGTGAAGAAATATCTATGCTCGAACCATTTCAGATTCTTTCAATTTGT TCTTTTATCTTATCAGCTCTCCATTTCATGGCTTGGACAAGGCATTATCAAAAAGAG |
| GAGAGAAAAGAAAAGGTCCAAACAAAGAGACAAACAGAGAGGTATCAATTTGACA CAGTTACCAAAAAGAAATCCAGGCCAAAGAAACAAAGGAAGTACTCTCTGACAACA AGGGTGACCACAAGGGGCTTTCTGCCGGCGGAGGCCATGGAGGTTGCTAATCAGAC TAGGCAGATGGTACATGCAATGAGAACTATTGGGACTCATCCTAGCTCCAGTGCTGG TCTGAAAGATGACCTTCTTGAAAATTTGCAGGCCTACCAGAAGCGAATGGGAGTGC AGATGCAGCGATTCAAGTCCTCTCGTCATTGCAGCAATATCATTGGGATCTTGCACC TGATATTGTGGATTACTGATCGTCTTTTTTCAAATGTATTTATCGTCGCTTTAAATA CGGTTTGAAAAGAGGGCCTTCTACGGAAGGAGTGCCTGAGTCCATGAGGAAGAAT ATCAACAGGAACAGCAGAGTGCTGTGGATGTTGACGATGGTCATTTTGTCAACATAG AGCTAGAGTAA |
| Segment 8-VIRUS 1 (SEQ ID NO: 8) |
| AACATAATGGACTCCAACACCATGTCAAGCTTTCAGGTAGACTGTTTCCTTTGGCAT ATCCGCAAGCGATTTGCAGACAATGGATTGGGTGATGCCCCATTCCTTGATCGGCTC CGCCGAGATCAAAAGTCCTTAAAAGGAAGAGGCAACACCCTTGGCCTCGATATCGA AACAGCCACTCTTGTTGGGAAACAAATCGTGGAATGGATCTTGAAAGAGGAATCCA GCGAGACACTTAGAATGACAATTGCATCTGTACCTACTTCGCGCTACCTTTCTGACA TGACCCTCGAGGAAATGTCACGAGACTGGTTCATGCTCATGCCTAGGCAAAAGATA ATAGGCCCTCTTTGCGTGCGATTGGACCAGGCGATCATGGAAAAGAACATAGTACT GAAAGCGAACTTCAGTGTAATCTTTAACCGATTAGAGACCTTGATACTACTAAGGGC TTTCACTGAGGAGGGAGCAATAGTTGGAGAAATTTCACCATTACCTTCTCTTCCAGG ACATACTTATGAGGATGTCAAAAATGCAATTGGGGTCCTCATCGGAGGACTTGAATG GAATGGTAACACGGTTCGAGTCTCTGAAAATATACAGAGATTCGCTTGGAGAAACT GTGATGAGAATGGGAGACCTTCACTACCTCCAGAGCAGAAATGAAAAGTGGCGAGA GCAATTGGGACAGAAATTTGAGGAAATAAGGTGGTTAATTGAAGAAATGCGGCACA GATTGAAAGCGACAGAGAATAGTTTCGAACAAATAACATTTATGCAAGCCTTACAA CTACTGCTTGAAGTAGAACAAGAGATAAGAGCTTTCTCGTTTCAGCTTATTTAACTG ATAA |

| **Table 3.** Genome sequence of pAH1N1 virus possessing mutations in NA and Matrix segments (Virus 2) |
|---|
| Segment 1-VIRUS 2 (SEQ ID NO: 9) |
| TATGGAGAGAATAAAAGAACTGAGAGATCTAATGTCGCAGTCCCGCACTCGCGAGA TACTCACTAAGACCACTGTGGACCATATGGCCATAATCAAAAAGTACACATCAGGA AGGCAAGAGAAGAACCCCGCACTCAGAATGAAGTGGATGATGGCAATGAGATACCC AATTACAGCAGACAAGAGAATAATGGACATGATTCCAGAGAGGAATGAACAAGGA CAAACCCTCTGGAGCAAAACAAACGATGCTGGATCAGACCGAGTGATGGTATCACC |
| TCTGGCCGTAACATGGTGGAATAGGAATGGCCCAACAACAAGTACAGTTCATTACC CTAAGGTATATAAAACTTATTTCGAAAAGGTCGAAAGGTTGAAACATGGTACCTTCG GCCCTGTCCACTTCAGAAATCAAGTTAAAATAAGGAGGAGAGTTGATACAAACCCT GGCCATGCAGATCTCAGTGCCAAGGAGGCACAGGATGTGATTATGGAAGTTGTTTTC CCAAATGAAGTGGGGGCAAGAATACTGACATCAGAGTCACAGCTGGCAATAACAAA AGAGAAGAAAGAAGAGCTCCAGGATTGTAAAATTGCTCCCTTGATGGTGGCGTACA TGCTAGAAAGAGAATTGGTCCGTAAAACAAGGTTTCTCCCAGTAGCCGGCGGAACA GGCAGTGTTTATATTGAAGTGTTGCACTTAACCCAAGGGACGTGCTGGGAGCAGATG TACACTCCAGGAGGAGAAGTGAGAAATGATGATGTTGACCAAAGTTTGATTATCGC TGCTAGAAACATAGTAAGAAGAGCAGCAGTGTCAGCAGACCCATTAGCATCTCTCTT GGAAATGTGCCACAGCACACAGATTGGAGGAGTAAGGATGGTGGACATCCTTAGAC AGAATCCAACTGAGGAACAAGCCGTAGACATATGCAAGGCAGCAATAGGGTTGAGG ATTAGCTCATCTTTCAGTTTTGGTGGGTTCACTTTCAAAAGGACAAGCGGATCATCA GTCAAGAAAGAAGAAGAAGTGCTAACGGGCAACCTCCAAACACTGAAAATAAGAG TACATGAAGGGTATGAAGAATTCACAATGGTTGGGAGAAGAGCAACAGCTATTCTC AGAAAGGCAACCAGGAGATTGATCCAGTTGATAGTAAGCGGGAGAGACGAGCAGT CAATTGCTGAGGCAATAATTGTGGCCATGGTATTCTCACAAGAGGATTGCATGATCA AGGCAGTTAGGGGCGATCTGAACTTTGTCAATAGGGCAAACCAGCGACTGAACCCC ATGCACCAACTCTTGAGGCATTTCCAAAAAGATGCAAAAGTGCTTTTCCAGAACTGG GGAATTGAATCCATCGACAATGTGATGGGAATGATCGGAATACTGCCCGACATGAC CCCAAGCACGGAGATGTCGCTGAGAGGGATAAGAGTCAGCAAAATGGGAGTAGAT GAATACTCCAGCACGGAGAGAGTGGTAGTGAGTATTGACCGATTTTTAAGGGTTAG AGATCAAAGAGGGAACGTACTATTGTCTCCCGAAGAAGTCAGTGAAACGCAAGGAA CTGAGAAGTTGACAATAACTTATTCGTCATCAATGATGTGGGAGATCAATGGCCCTG AGTCAGTGCTAGTCAACACTTATCAATGGATAATCAGGAACTGGGAAATTGTGAAA ATTCAATGGTCACAAGATCCCACAATGTTATACAACAAAATGGAATTTGAACCATTT CAGTCTCTTGTCCCTAAGGCAACCAGAAGCCGGTACAGTGGATTCGTAAGGACACTG TTCCAGCAAATGCGGGATGTGCTTGGGACATTTGACACTGTCCAAATAATAAAACTT CTCCCCTTTGCTGCTGCTCCACCAGAACAGAGTAGGATGCAATTTTCCTCATTGACTG TGAATGTGAGAGGATCAGGGTTGAGGATACTGGTAAGAGGCAATTCTCCAGTATTC AATTACAACAAGGCAACCAAACGACTTACAGTTCTTGGAAAGGATGCAGGTGCATT GACTGAAGATCCAGATGAAGGCACATCTGGGGTGGAGTCTGCTGTCCTGAGAGGAT TTCTCATTTTGGGCAAAGAAGACAAGAGATATGGCCCAGCATTAAGCATCAATGAA CTGAGCAATCTTGCAAAAGGAGAGAAAGCTAATGTGCTAATTGGGCAAGGGGACGT AGTGTTGGTAATGAAACGAAAACGGGACTCTAGCATACTTACTGACAGCCAGACAG CGACCAAAAGAATTCGGATGGCCATCAATTAGTGTCGAATTGTT |
| Segment 2-VIRUS 2 (SEQ ID NO: 10) |
| TTGAATGGATGTCAATCCGACTCTACTTTTCCTAAAAATTCCAGCGCAAAATGCCAT AAGCACCACATTCCCTTATACTGGAGATCCTCCATACAGCCATGGAACAGGAACAG |
| GATACACCATGGACACAGTAAACAGAACACACCAATACTCAGAAAAGGGAAAGTG GACGACAAACACAGAGACTGGTGCACCCCAGCTCAACCCGATTGATGGACCACTAC CTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATG GAAGTTGTTCAACAAACAAGGGTAGATAAACTAACTCAAGGTCGCCAGACTTATGA TTGGACATTAAACAGAAATCAACCGGCAGCAACTGCATTGGCCAACACCATAGAAG TCTTTAGATCGAATGGCCTAACAGCTAATGAGTCAGGAAGGCTAATAGATTTCTTAA AGGATGTAATGGAATCAATGAACAAAGAGGAAATAGAGATAACAACCCACTTTCAA AGAAAAAGGAGAGTAAGAGACAACATGACCAAGAAGATGGTCACGCAAAGAACAA TAGGGAAGAAAAAACAAAGACTGAATAAGAGAGGCTATCTAATAAGAGCACTGAC ATTAAATACGATGACCAAAGATGCAGAGAGAGGCAAGTTAAAAAGAAGGCTATC GCAACACCTGGGATGCAGATTAGAGGTTTCGTATACTTTGTTGAAACTTTAGCTAGG AGCATTTGCGAAAAGCTTGAACAGTCTGGGCTCCCAGTAGGGGGCAATGAAAAGAA GGCCAAACTGGCAAATGTTGTGAGAAAGATGATGACTAATTCACAAGACACAGAGA TTTCTTTCACAATCACTGGGGACAACACTAAGTGGAATGAAAATCAAAATCCTCGAA TGTTCCTGGCGATGATTACATATATCACCAGAAATCAACCCGAGTGGTTCAGAAACA TCCTGAGCATGGCACCCATAATGTTCTCAAACAAAATGGCAAGACTAGGGAAAGGG TACATGTTCGAGAGTAAAAGAATGAAGATTCGAACACAAATACCAGCAGAAATGCT AGCAAGCATTGACCTGAAGTACTTCAATGAATCAACAAAGAAGAAAATTGAGAAAA TAAGGCCTCTTCTAATAGATGGCACAGCATCACTGAGTCCTGGGATGATGATGGGCA TGTTCAACATGCTAAGTACGGTCTTGGGAGTCTCGATACTGAATCTTGGACAAAAGA AATACACCAAGACAATATACTGGTGGGATGGGCTCCAATCATCCGACGATTTTGCTC TCATAGTGAATGCACCAAACCATGAGGGAATACAAGCAGGAGTGGACAGATTCTAC AGGACCTGCAAGTTAGTGGGAATCAACATGAGCAAAAAGAAGTCCTATATAAATAA GACAGGGACATTTGAATTCACAAGCTTTTTTATCGCTATGGATTTGTGGCTAATTTT AGCATGGAGCTACCCAGCTTTGGAGTGTCTGGAGTAAATGAATCAGCTGACATGAG TATTGGAGTAACAGTGATAAAGAACAACATGATAAACAATGACCTTGGACCTGCAA CGGCCCAGATGGCTCTTCAATTGTTCATCAAAGACTACAGATACACATATAGGTGCC ATAGGGGAGACACACAAATTCAGACGAGAAGATCATTTGAGTTAAAGAAGCTGTGG GATCAAACCCAATCAAAGGTAGGGCTATTAGTATCAGATGGAGGACCAAACTTATA CAATATACGGAATCTTCACATTCCTGAAGTCTGCTTAAAATGGGAGCTAATGGATGA TGATTATCGGGGAAGACTTTGTAATCCCCTGAATCCCTTTGTCAGTCATAAAGAGAT TGATTCTGTAAACAATGCTGTGGTAATGCCAGCCCATGGTCCAGCCAAAAGCATGGA ATATGATGCCGTTGCAACTACACATTCCTGGATTCCCAAGAGGAATCGTTCTATTCT CAACACAAGCCAAAGGGGAATTCTTGAGGATGAACAGATGTACCAGAAGTGCTGCA ATCTATTCGAGAAATTTTTCCCTAGCAGTTCATATAGGAGACCGGTTGGAATTTCTA GCATGGTGGAGGCCATGGTGTCTAGGGCCCGGATTGATGCCAGGGTCGACTTCGAG TCTGGACGGATCAAGAAAGAAGAGTTCTCTGAGATCATGAAGATCTGTTCCACCATT GAAGAACTCAGACGGCAAAAATAATGAATTTAACTTGTCCTTCATGAAA |
| Segment 3-VIRUS 2 (SEQ ID NO: 11) |
| TCCAAAATGGAAGACTTTGTGCGACAATGCTTCAATCCAATGATCGTCGAGCTTGCG GAAAAGGCAATGAAAGAATATGGGGAAGATCCGAAAATCGAAACTAACAAGTTTG CTGCAATATGCACACATTTGGAAGTTTGTTTCATGTATTCGGATTTCCATTTCATCGA CGAACGGGGTGAATCAATAATTGTAGAATCTGGTGACCCGAATGCACTATTGAAGC ACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGAT TACAAAGAGAACCGGTTCATTGAAATTGGAGTAACACGGAGGGAAGTCCACATATA TTACCTAGAGAAAGCCAACAAAATAAAATCTGAGAAGACACACATTCACATCTTTTC ATTCACTGGAGAGGAGATGGCCACCAAAGCGGACTACACCCTTGACGAAGAGAGCA GGGCAAGAATCAAAACTAGGCTTTTCACTATAAGACAAGAAATGGCCAGTAGGAGT CTATGGGATTCCTTTCGTCAGTCCGAAAGAGGCGAAGAGACAATTGAAGAAAAATT TGAGATTACAGGAACTATGCGCAAGCTTGCCGACCAAAGTCTCCCACCGAACTTCTC CAGCCTTGAAAACTTTAGAGCCTATGTAGATGGATTCGAGCCGAACGGCTGCATTGA GGGCAAGCTTTCCCAAATGTCAAAAGAAGTGAACGCCAAAATTGAACCATTCTTGA GGACGACACCACGCCCCCTCAGATTGCCTGATGGGCCTCTTTGCCATCAGCGGTCAA AGTTCCTGCTGATGGATGCTCTGAAATTAAGTATTGAAGACCCGAGTCACGAGGGG GAGGGAATACCACTATATGATGCAATCAAATGCATGAAGACATTCTTTGGCTGGAA AGAGCCTAACATAGTCAAACCACATGAGAAAGGCATAAATCCCAATTACCTCATGG CTTGGAAGCAGGTGCTAGCAGAGCTACAGGACATTGAAAATGAAGAGAAGATCCCA AGGACAAAGAACATGAAGAGAACAAGCCAATTGAAGTGGGCACTCGGTGAAAATA TGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATT CAATAAGGCATGTGAATTGACTGATTCAAGCTGGATAGAACTTGATGAAATAGGAG AAGATGTTGCCCCGATTGAACATATCGCAAGCATGAGGAGGAACTATTTTACAGCA GAAGTGTCCCACTGCAGGGCTACTGAATACATAATGAAGGGAGTGTACATAAATAC GGCCTTGCTCAATGCATCCTGTGCAGCCATGGATGACTTTCAGCTGATCCCAATGAT AAGCAAATGTAGGACCAAAGAAGGAAGACGGAAAACAAACCTGTATGGGTTCATTA TAAAAGGAAGGTCTCATTTGAGAAATGATACTGATGTGGTGAACTTTGTAAGTATGG AGTTCTCACTCACTGACCCGAGACTGGAGCCACACAAATGGGAAAAATACTGTGTTC TTGAAATAGGAGACATGCTCTTGAGGACTGCGATAGGCCAAGTGTCGAGGCCCATG TTCCTATATGTGAGAACCAATGGAACCTCCAAGATCAAGATGAAATGGGGCATGGA ACTGAGGCGCTGCCTTCTTCAGTCTCTTCAGCAGATTGAGAGCATGATTGAGGCCGA GTCTTCTGTCAAAGAGAAAGACATGACCAAGGAATTCTTTGAAAACAAATCGGAAA CATGGCCAATCGGAGAGTCACCCAGGGGAGTGGAGGAAGGCTCTATTGGGAAAGTG TGCAGGACCTTACTGGCAAAATCTGTATTCAACAGTCTATATGCGTCTCCACAACTT GAGGGGTTTTCGGCTGAATCGAGAAAATTGCTTCTCATTGTTCAGGCACTTAGGGAC AACCTGGAACCTGGAACCTTCGATCTTGGGGGGCTATATGAAGCAATCGAGGAGTG CCTGATTAATGATCCCTGGGTTTTGCTTAATGCATCTTGGTTCAACTCCTTCCTCACA |
| CATGCACTGAAGTAGTTGTGGCAATGCTAC |
| Segment 4-VIRUS 2 (SEQ ID NO: 12) |
| AAAAGCAACAAAAGATGAAGGCAATACTAGTAGTTCTGCTATATACATTTGCAACC GCAAATGCAGACACATTATGTATAGGTTATCATGCGAACAATTCAACAGACACTGTA GACACAGTACTAGAAAAGAATGTAACAGTAACACACTCTGTTAACCTTCTAGAAGA CAAGCATAACGGAAACTATGCAAACTAAGAGGGGTAGCCCCATTGCATTTGGGTA AATGTAACATTGCTGGCTGGATCCTGGGAAATCCAGAGTGTGAATCACTCTCCACAG CAAGCTCATGGTCCTACATTGTGGAAACATCTAGTTCAGACAATGGAACGTGTTACC CAGGAGATTTCATCGATTATGAGGAGCTAAGAGAGCAATTGAGCTCAGTGTCATCAT TTGAAAGGTTTGAGATATTCCCCAAGACAAGTTCATGGCCCAATCATGACTCGAACA AAGGTGTAACGGCAGCATGTCCTCATGCTGGAGCAAAAAGCTTCTACAAAAATTTA ATATGGCTAGTTAAAAAGGAAATTCATACCCAAAGCTCAGCAAATCCTACATTAAT GATAAAGGGAAAGAAGTCCTCGTGCTATGGGGCATTCACCATCCATCTACTAGTGCT GACCAACAAAGTCTCTATCAGAATGCAGATGCATATGTTTTTGTGGGGTCATCAAGA TACAGCAAGAAGTTCAAGCCGGAAATAGCAATAAGACCCAAAGTGAGGGATCAAG AAGGGAGAATGAACTATTACTGGACACTAGTAGAGCCGGGAGACAAAATAACATTC GAAGCAACTGGAAATCTAGTGGTACCGAGATATGCATTCGCAATGGAAAGAAATGC TGGATCTGGTATTATCATTTCAGATACACCAGTCCACGATTGCAATACAACTTGTCA GACACCCAAGGGTGCTATAAACACCAGCCTCCCATTTCAGAATATACATCCGATCAC AATTGGAAAATGTCCAAAATATGTAAAAAGCACAAAATTGAGACTGGCCACAGGAT TGAGGAATGTCCCGTCTATTCAATCTAGAGGCCTATTTGGGGCCATTGCCGGTTTCA TTGAAGGGGGGTGGACAGGGATGGTAGATGGATGGTACGGTTATCACCATCAAAAT GAGCAGGGGTCAGGATATGCAGCCGACCTGAAGAGCACACAGAATGCCATTGACGA GATTACTAACAAAGTAAATTCTGTTATTGAAAAGATGAATACACAGTTCACAGCAGT AGGTAAAGAGTTCAACCACCTGGAAAAAAGAATAGAGAATTTAAATAAAAAAGTTG ATGATGGTTTCCTGGACATTTGGACTTACAATGCCGAACTGTTGGTTCTATTGGAAA ATGAAAGAACTTTGGACTACCACGATTCAAATGTGAAGAACTTATATGAAAAGGTA AGAAGCCAGCTAAAAAACAATGCCAAGGAAATTGGAAACGGCTGCTTTGAATTTTA CCACAAATGCGATAACACGTGCATGGAAAGTGTCAAAAATGGGACTTATGACTACC CAAAATACTCAGAGGAAGCAAAATTAAACAGAGAAGAAATAGATGGGGTAAAGCT GGAATCAACAAGGATTTACCAGATTTTGGCGATCTATTCAACTGTCGCCAGTTCATT GGTACTGGTAGTCTCCCTGGGGGCAATCAGTTTCTGGATGTGCTCTAATGGGTCTCT ACAGTGTAGAATATGTATTTAACTTTAGGATTTCAGAAGCAT |
| Segment 5-VIRUS 2 (SEQ ID NO: 13) |
| ATCAATCACTGAGTGACATCGAAGCCATGGCGTCTCAAGGCACCAAACGATCATAT GAACAAATGGAGACTGGTGGGGAGCGCCAGGATGCCACAGAAATCAGAGCATCTGT CGGAAGAATGATTGGTGGAATCGGGAGATTCTACATCCAAATGTGCACTGAACTCA AACTCAGTGATTATGATGGACGACTAATCCAGAATAGCATAACAATAGAGAGGATG |
| GTGCTTTCTGCTTTTGATGAGAGAAGAAATAAATACCTAGAAGAGCATCCCAGTGCT GGGAAGGACCCTAAGAAAACAGGAGGACCCATATATAGAAGAATAGACGGAAAGT GGATGAGAGAACTCATCCTTTATGACAAAGAAGAAATAAGGAGAGTTTGGCGCCAA GCAAACAATGGCGAAGATGCAACAGCAGGTCTTACTCATATCATGATTTGGCATTCC AACCTGAATGATGCCACATATCAGAGAACAAGAGCGCTTGTTCGCACCGGAATGGA TCCCAGAATGTGCTCTCTAATGCAAGGTTCAACACTTCCCAGAAGGTCTGGTGCCGC AGGTGCTGCGGTGAAAGGAGTTGGAACAATAGCAATGGAGTTAATCAGAATGATCA AACGTGGAATCAATGACCGAAATTTCTGGAGGGGTGAAAATGGACGAAGGACAAG GGTTGCTTATGAAAGAATGTGCAATATCCTCAAAGGAAAATTTCAAACAGCTGCCCA GAGGGCAATGATGGATCAAGTAAGAGAAAGTCGAAACCCAGGAAACGCTGAGATT GAAGACCTCATTTTCCTGGCACGGTCAGCACTCATTCTGAGGGGATCAGTTGCACAT AAATCCTGCCTGCCTGCTTGTGTGTATGGGCTTGCAGTAGCAAGTGGGCATGACTTT GAAAGGGAAGGGTACTCACTGGTCGGGATAGACCCATTCAAATTACTCCAAAACAG CCAAGTGGTCAGCCTGATGAGACCAAATGAAAACCCAGCTCACAAGAGTCAATTGG TGTGGATGGCATGCCACTCTGCTGCATTTGAAGATTTAAGAGTATCAAGTTTCATAA GAGGAAAGAAAGTGATTCCAAGAGGAAAGCTTTCCACAAGAGGGGTCCAGATTGCT TCAAATGAGAATGTGGAAACCATGGACTCCAATACCCTGGAACTAAGAAGCAGATA CTGGGCCATAAGGACCAGGAGTGGAGGAAATACCAATCAACAAAAGGCATCCGCA GGCCAGATCAGTGTGCAGCCTACATTCTCAGTGCAGCGAAATCTCCCTTTTGAAAGA GCAACCGTTATGGCAGCATTCAGCGGGAACAATGAAGGACGGACATCCGACATGCG AACAGAAGTTATAAGAATGATGGAAAGTGCAAAGCCAGAAGATTTGTCCTTCCAGG GGCGGGGAGTCTTCGAGCTCTCGGACGAAAAGGCAACGAACCCGATCGTGCCTTCC TTTGACATGAGTAATGAAGGGTCTTATTTCTTCGGAGACAATGCAGAGGAGTATGAC AGTTGAGGAAAAATACCCTTGTTTCTACT |
| Segment 6-VIRUS 2 (SEQ ID NO: 14) |
| ATGAATCCAAACCAAAAGATAATAACCATTGGTTCGGTCTGTATGACGATTGGAATG GCTAACTTAATATTACAAATTGGAAACATAATCTCAATATGGATTAGCCACTCAATT CAACTTGGGAATCAAAATCAGATTGAAACATGCAATCAAAGCGTCATTACTTATGA AAACAACACTTGGGTAAATCAGACATATGTTAACATCAGCAACACCAACTTTGCTGC TGGACAGTCAGTGGTTTCCGTGAAATTAGCGGGCAATTCCTCTCTCTGCCCTGTTAGT GGATGGGCTATATACAGTAAAGACAACAGTATAAGAATCGGTTCCAAGGGGGATGT GTTTGTCATAAGGGAACCATTCATATCATGCTCCCCCTTGGAATGCAGAACCTTCTTC TTGACTCAAGGGGCCTTGCTAAATGACAAACATTCCAATGGAACCATTAAAGACAG GAGCCCATATCGAACCCTAATGAGCTGTCCTATTGGTGAAGTTCCCTCTCCATACAA CTCAAGATTTGAGTCAGTCGCTTGGTCAGCAAGTGCTTGTCATGATGGCATCAATTG GCTAACAATTGGAATTTCTGGCCCAGACAATGGGGCAGTGGCTGTGTTAAAGTACA ACGGCATAATAACAGACACTATCAAGAGTTGGAGAAACAATATATTGAGAACACAA GAGTCTGAATGTGCATGTGTAAATGGTTCTTGCTTTACTGTAATGACCGATGGACCA AGTGATGGACAGGCCTCATACAAGATCTTCAGAATAGAAAAGGGAAAGATAGTCAA |
| ATCAGTCGAAATGAATGCCCCTAATTATTACTATGAGGAATGCTCCTGTTATCCTGA TTCTAGTGAAATCACATGTGTGTGCAGGGATAACTGGCATGGCTCGAATCGACCGTG GGTGTCTTTCAACCAGAATCTGGAATATCAGATAGGATACATATGCAGTGGGATTTT CGGAGACAATCCACGCCCTAATGATAAGACAGGCAGTTGTGGTCCAGTATCGTCTA ATGGAGCAAATGGAGTAAAAGGATTTTCATTCAAATACGGCAATGGTGTTTGGATA GGGAGAACTAAAAGCATTAGTTCAAGAAACGGTTTTGAGATGATTTGGGATCCGAA CGGATGGACTGGGACAGACAATAACTTCTCAATAAAGCAAGATATCGTAGGAATAA ATGAGTGGTCAGGATATAGCGGGAGTTTTGTTCAGCATCCAGAACTAACAGGGCTG GATTGTATAAGACCTTGCTTCTGGGTTGAACTAATCAGAGGGCGACCCAAAGAGAA CACAATCTGGACTAGCGGGAGCAGCATATCCTTTTGTGGTGTAAACAGTGACACTGT GGGTTGGTCTTGGCCAGACGGTGCTGAGTTGCCATTTACCATTGACAAGTAA |
| Segment 7-VIRUS 2 (SEQ ID NO: 15) |
| ATGAGTCTTCTAACCGAGGTCGAAACGTACGTTCTTTCTATCATCCCGTCAGGCCCC CTCAAAGCCGAGATCGCGCAGAGACTGGAAAGTGTCTTTGCAGGAAAGAACACAGA TCTTGAGGCTCTCATGGAATGGCTAAAGACAAGACCAATCTTGTCACCTCTGACTAA GGGAATTTTAGGATTTGTGTTCACGCTCACCGTGCCCAGTGAGCGAGGACTGCAGCG TAGACGCTTTGTCCAAAATGCCCTAAATGGGAATGGGGACCCGAACAACATGGATA GAGCAGTTAAACTATACAAGAAGCTCAAAAGAGAAATAACGTTCCATGGGGCCAAG GAGGTGTCACTAAGCTATTCAACTGGTGCACTTGCCAGTTGCATGGGCCTCATATAC AACAGGATGGGAACAGTGACCACAGAAGCTGCTTTTGGTCTAGTGTGTGCCACTTGT GAACAGATTGCTGATTCACAGCATCGGTCTCACAGACAGATGGCTACTACCACCAAT CCACTAATCAGGCATGAAAACAGAATGGTGCTGGCTAGCACTACGGCAAAGGCTAT GGAACAGATGGCTGGATCGAGTGAACAGAAAGGAGAAGACGTCCAAAAGCTGGCA GAAGAGCTGCAAAGCAACATTGGAGTATTGAGATCTCTTGGAGCAAGTCAAAAGAG TGGGGAAGGAATTGCAAAGGATGTAATGGAAGTGCTAAAGCAGAGCTCTATGGGAA ATTCAGCTCTTGTGAAGAAATATCTATGCTCGAACCATTTCAGATTCTTTCAATTTGT TCTTTTATCTTATCAGCTCTCCATTTCATGGCTTGGACAAGGCATTATCAAAAAAGAG GAGAGAAAAGAAAAGGTCCAAACAAAGAGACAAACAGAGAGGTATCAATTTGACA CAGTTACCAAAAAGAAATCCAGGCCAAAGAAACAAGGAAGTACTCTCTGACAACA AGGGTGACCACAAGGGGCTTTCTGCCGGCGGAGGCCATGGAGGTTGCTAATCAGAC TAGGCAGATGGTACATGCAATGAGAACTATTGGGACTCATCCTAGCTCCAGTGCTGG TCTGAAAGATGACCTTCTTGAAAATTTGCAGGCCTACCAGAAGCGAATGGGAGTGC AGATGCAGCGATTCAAGTCCTCTCGTCATTGCAGCAATATCATTGGGATCTTGCACC TGATATTGTGGATTACTGATCGTCTTTTTTTCAAATGTATTTATCGTCGCTTTAAATA CGGTTTGAAAAGAGGGCCTTCTACGGAAGGAGTGCCTGAGTCCATGAGGGAAGAAT ATCAACAGGAACAGCAGAGTGCTGTGGATGTTGACGATGGTCATTTTGTCAACATAG AGCTAGAGTAA |
| Segment 8-VIRUS 2 (SEQ ID NO: 16) |
| AACATAATGGACTCCAACACCATGTCAAGCTTTCAGGTAGACTGTTTCCTTTGGCAT ATCCGCAAGCGATTTGCAGACAATGGATTGGGTGATGCCCCATTCCTTGATCGGCTC CGCCGAGATCAAAAGTCCTTAAAAGGAAGAGGCAACACCCTTGGCCTCGATATCGA AACAGCCACTCTTGTTGGGAAACAAATCGTGGAATGGATCTTGAAAGAGGAATCCA GCGAGACACTTAGAATGACAATTGCATCTGTACCTACTTCGCGCTACCTTTCTGACA TGACCCTCGAGGAAATGTCACGAGACTGGTTCATGCTCATGCCTAGGCAAAAGATA ATAGGCCCTCTTTGCGTGCGATTGGACCAGGCGATCATGGAAAAGAACATAGTACT GAAAGCGAACTTCAGTGTAATCTTTAACCGATTAGAGACCTTGATACTACTAAGGGC TTTCACTGAGGAGGGAGCAATAGTTGGAGAAATTTCACCATTACCTTCTCTTCCAGG ACATACTTATGAGGATGTCAAAAATGCAATTGGGGTCCTCATCGGAGGACTTGAATG GAATGGTAACACGGTTCGAGTCTCTGAAAATATACAGAGATTCGCTTGGAGAAACT GTGATGAGAATGGGAGACCTTCACTACCTCCAGAGCAGAAATGAAAAGTGGCGAGA GCAATTGGGACAGAAATTTGAGGAAATAAGGTGGTTAATTGAAGAAATGCGGCACA GATTGAAAGCGACAGAGAATAGTTTCGAACAAATAACATTTATGCAAGCCTTACAA CTACTGCTTGAAGTAGAACAAGAGATAAGAGCTTTCTCGTTTCAGCTTATTTAACTG ATAA |

| **Table 4.** Genome sequence of pAH1N1 virus possessing mutations in matrix (MA) segment (Virus 3) |
|---|
| Segment 1-VIRUS 3 (SEQ ID NO: 17) |
| TATGGAGAGAATAAAAGAACTGAGAGATCTAATGTCGCAGTCCCGCACTCGCGAGA TACTCACTAAGACCACTGTGGACCATATGGCCATAATCAAAAAGTACACATCAGGA AGGCAAGAGAAGAACCCCGCACTCAGAATGAAGTGGATGATGGCAATGAGATACCC AATTACAGCAGACAAGAGAATAATGGACATGATTCCAGAGAGGAATGAACAAGGA CAAACCCTCTGGAGCAAAACAAACGATGCTGGATCAGACCGAGTGATGGTATCACC TCTGGCCGTAACATGGTGGAATAGGAATGGCCCAACAACAAGTACAGTTCATTACC CTAAGGTATATAAAACTTATTTCGAAAAGGTCGAAAGGTTGAAACATGGTACCTTCG GCCCTGTCCACTTCAGAAATCAAGTTAAAATAAGGAGGAGAGTTGATACAAACCCT GGCCATGCAGATCTCAGTGCCAAGGAGGCACAGGATGTGATTATGGAAGTTGTTTTC CCAAATGAAGTGGGGGCAAGAATACTGACATCAGAGTCACAGCTGGCAATAACAAA AGAGAAGAAAGAAGAGCTCCAGGATTGTAAAATTGCTCCCTTGATGGTGGCGTACA TGCTAGAAAGAGAATTGGTCCGTAAAACAAGGTTTCTCCCAGTAGCCGGCGGAACA GGCAGTGTTTATATTGAAGTGTTGCACTTAACCCAAGGGACGTGCTGGGAGCAGATG TACACTCCAGGAGGAGAAGTGAGAAATGATGATGTTGACCAAAGTTTGATTATCGC TGCTAGAAACATAGTAAGAAGAGCAGCAGTGTCAGCAGACCCATTAGCATCTCTCTT GGAAATGTGCCACAGCACACAGATTGGAGGAGTAAGGATGGTGGACATCCTTAGAC |
| AGAATCCAACTGAGGAACAAGCCGTAGACATATGCAAGGCAGCAATAGGGTTGAGG ATTAGCTCATCTTTCAGTTTTGGTGGGTTCACTTTCAAAAGGACAAGCGGATCATCA GTCAAGAAAGAAGAAGTGCTAACGGCAACCTCCAAACACTGAAAATAAGAG TACATGAAGGGTATGAAGAATTCACAATGGTTGGGAGAAGAGCAACAGCTATTCTC AGAAAGGCAACCAGGAGATTGATCCAGTTGATAGTAAGCGGGAGAGACGAGCAGT CAATTGCTGAGGCAATAATTGTGGCCATGGTATTCTCACAAGAGGATTGCATGATCA AGGCAGTTAGGGGCGATCTGAACTTTGTCAATAGGGCAAACCAGCGACTGAACCCC ATGCACCAACTCTTGAGGCATTTCCAAAAAGATGCAAAAGTGCTTTTCCAGAACTGG GGAATTGAATCCATCGACAATGTGATGGGAATGATCGGAATACTGCCCGACATGAC CCCAAGCACGGAGATGTCGCTGAGAGGGATAAGAGTCAGCAAAATGGGAGTAGAT GAATACTCCAGCACGGAGAGAGTGGTAGTGAGTATTGACCGATTTTTAAGGTTAG AGATCAAAGAGGGAACGTACTATTGTCTCCCGAAGAAGTCAGTGAAACGCAAGGAA CTGAGAAGTTGACAATAACTTATTCGTCATCAATGATGTGGGAGATCAATGGCCCTG AGTCAGTGCTAGTCAACACTTATCAATGGATAATCAGGAACTGGGAAATTGTGAAA ATTCAATGGTCACAAGATCCCACAATGTTATACAACAAAATGGAATTTGAACCATTT CAGTCTCTTGTCCCTAAGGCAACCAGAAGCCGGTACAGTGGATTCGTAAGGACACTG TTCCAGCAAATGCGGGATGTGCTTGGGACATTTGACACTGTCCAAATAATAAAACTT CTCCCCTTTGCTGCTGCTCCACCAGAACAGAGTAGGATGCAATTTTCCTCATTGACTG TGAATGTGAGAGGATCAGGGTTGAGGATACTGGTAAGAGGCAATTCTCCAGTATTC AATTACAACAAGGCAACCAAACGACTTACAGTTCTTGGAAAGGATGCAGGTGCATT GACTGAAGATCCAGATGAAGGCACATCTGGGGTGGAGTCTGCTGTCCTGAGAGGAT TTCTCATTTTGGGCAAAGAAGACAAGAGATATGGCCCAGCATTAAGCATCAATGAA CTGAGCAATCTTGCAAAAGGAGAGAAAGCTAATGTGCTAATTGGGCAAGGGGACGT AGTGTTGGTAATGAAACGAAAACGGGACTCTAGCATACTTACTGACAGCCAGACAG CGACCAAAAGAATTCGGATGGCCATCAATTAGTGTCGAATTGTT |
| Segment 2-VIRUS 3 (SEQ ID NO: 18) |
| TTGAATGGATGTCAATCCGACTCTACTTTTCCTAAAAATTCCAGCGCAAAATGCCAT AAGCACCACATTCCCTTATACTGGAGATCCTCCATACAGCCATGGAACAGGAACAG GATACACCATGGACACAGTAAACAGAACACACCAATACTCAGAAAAGGGAAAGTG GACGACAAACACAGAGACTGGTGCACCCCAGCTCAACCCGATTGATGGACCACTAC CTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATG GAAGTTGTTCAACAAACAAGGGTAGATAAACTAACTCAAGGTCGCCAGACTTATGA TTGGACATTAAACAGAAATCAACCGGCAGCAACTGCATTGGCCAACACCATAGAAG TCTTTAGATCGAATGGCCTAACAGCTAATGAGTCAGGAAGGCTAATAGATTTCTTAA AGGATGTAATGGAATCAATGAACAAAGAGGAAATAGAGATAACAACCCACTTTCAA AGAAAAAGGAGAGTAAGAGACAACATGACCAAGAAGATGGTCACGCAAAGAACAA TAGGGAAGAAAAAACAAAGACTGAATAAGAGAGGCTATCTAATAAGAGCACTGAC ATTAAATACGATGACCAAAGATGCAGAGAGAGGCAAGTTAAAAAGAAGGGCTATC GCAACACCTGGGATGCAGATTAGAGGTTTCGTATACTTTGTTGAAACTTTAGCTAGG |
| AGCATTTGCGAAAAGCTTGAACAGTCTGGGCTCCCAGTAGGGGGCAATGAAAAGAA GGCCAAACTGGCAAATGTTGTGAGAAAGATGATGACTAATTCACAAGACACAGAGA TTTCTTTCACAATCACTGGGGACAACACTAAGTGGAATGAAAATCAAAATCCTCGAA TGTTCCTGGCGATGATTACATATATCACCAGAAATCAACCCGAGTGGTTCAGAAACA TCCTGAGCATGGCACCCATAATGTTCTCAAACAAAATGGCAAGACTAGGGAAAGGG TACATGTTCGAGAGTAAAAGAATGAAGATTCGAACACAAATACCAGCAGAAATGCT AGCAAGCATTGACCTGAAGTACTTCAATGAATCAACAAAGAAGAAAATTGAGAAAA TAAGGCCTCTTCTAATAGATGGCACAGCATCACTGAGTCCTGGGATGATGATGGGCA TGTTCAACATGCTAAGTACGGTCTTGGGAGTCTCGATACTGAATCTTGGACAAAAGA AATACACCAAGACAATATACTGGTGGGATGGGCTCCAATCATCCGACGATTTTGCTC TCATAGTGAATGCACCAAACCATGAGGGAATACAAGCAGGAGTGGACAGATTCTAC AGGACCTGCAAGTTAGTGGGAATCAACATGAGCAAAAAGAAGTCCTATATAAATAA GACAGGGACATTTGAATTCACAAGCTTTTTTATCGCTATGGATTTGTGGCTAATTTT AGCATGGAGCTACCCAGCTTTGGAGTGTCTGGAGTAAATGAATCAGCTGACATGAG TATTGGAGTAACAGTGATAAAGAACAACATGATAAACAATGACCTTGGACCTGCAA CGGCCCAGATGGCTCTTCAATTGTTCATCAAAGACTACAGATACACATATAGGTGCC ATAGGGGAGACACACAAATTCAGACGAGAAGATCATTTGAGTTAAAGAAGCTGTGG GATCAAACCCAATCAAAGGTAGGGCTATTAGTATCAGATGGAGGACCAAACTTATA CAATATACGGAATCTTCACATTCCTGAAGTCTGCTTAAAATGGGAGCTAATGGATGA TGATTATCGGGGAAGACTTTGTAATCCCCTGAATCCCTTTGTCAGTCATAAAGAGAT TGATTCTGTAAACAATGCTGTGGTAATGCCAGCCCATGGTCCAGCCAAAAGCATGGA ATATGATGCCGTTGCAACTACACATTCCTGGATTCCCAAGAGGAATCGTTCTATTCT CAACACAAGCCAAAGGGGAATTCTTGAGGATGAACAGATGTACCAGAAGTGCTGCA ATCTATTCGAGAAATTTTTCCCTAGCAGTTCATATAGGAGACCGGTTGGAATTTCTA GCATGGTGGAGGCCATGGTGTCTAGGGCCCGGATTGATGCCAGGGTCGACTTCGAG TCTGGACGGATCAAGAAAGAAGAGTTCTCTGAGATCATGAAGATCTGTTCCACCATT GAAGAACTCAGACGGCAAAAATAATGAATTTAACTTGTCCTTCATGAAA |
| Segment 3-VIRUS 3 (SEQ ID NO: 19) |
| TCCAAAATGGAAGACTTTGTGCGACAATGCTTCAATCCAATGATCGTCGAGCTTGCG GAAAAGGCAATGAAAGAATATGGGGAAGATCCGAAAATCGAAACTAACAAGTTTG CTGCAATATGCACACATTTGGAAGTTGTTTCATGTATTCGGATTTCCATTTCATCGA CGAACGGGGTGAATCAATAATTGTAGAATCTGGTGACCCGAATGCACTATTGAAGC ACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGAT TACAAAGAGAACCGGTTCATTGAAATTGGAGTAACACGGAGGGAAGTCCACATATA TTACCTAGAGAAAGCCAACAAAATAAAATCTGAGAAGACACACATTCACATCTTTTC ATTCACTGGAGAGGAGATGGCCACCAAAGCGGACTACACCCTTGACGAAGAGAGCA GGGCAAGAATCAAAACTAGGCTTTTCACTATAAGACAAGAAATGGCCAGTAGGAGT CTATGGGATTCCTTTCGTCAGTCCGAAAGAGGCGAAGAGACAATTGAAGAAAAATT TGAGATTACAGGAACTATGCGCAAGCTTGCCGACCAAAGTCTCCCACCGAACTTCTC |
| CAGCCTTGAAAACTTTAGAGCCTATGTAGATGGATTCGAGCCGAACGGCTGCATTGA GGGCAAGCTTTCCCAAATGTCAAAAGAAGTGAACGCCAAAATTGAACCATTCTTGA GGACGACACCACGCCCCCTCAGATTGCCTGATGGGCCTCTTTGCCATCAGCGGTCAA AGTTCCTGCTGATGGATGCTCTGAAATTAAGTATTGAAGACCCGAGTCACGAGGGG GAGGGAATACCACTATATGATGCAATCAAATGCATGAAGACATTCTTTGGCTGGAA AGAGCCTAACATAGTCAAACCACATGAGAAAGGCATAAATCCCAATTACCTCATGG CTTGGAAGCAGGTGCTAGCAGAGCTACAGGACATTGAAAATGAAGAGAAGATCCCA AGGACAAAGAACATGAAGAGAACAAGCCAATTGAAGTGGCACTCGGTGAAAATA TGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATT CAATAAGGCATGTGAATTGACTGATTCAAGCTGGATAGAACTTGATGAAATAGGAG AAGATGTTGCCCCGATTGAACATATCGCAAGCATGAGGAGGAACTATTTTACAGCA GAAGTGTCCCACTGCAGGGCTACTGAATACATAATGAAGGGAGTGTACATAAATAC GGCCTTGCTCAATGCATCCTGTGCAGCCATGGATGACTTTCAGCTGATCCCAATGAT AAGCAAATGTAGGACCAAAGAAGGAAGACGGAAAACAAACCTGTATGGGTTCATTA TAAAAGGAAGGTCTCATTTGAGAAATGATACTGATGTGGTGAACTTTGTAAGTATGG AGTTCTCACTCACTGACCCGAGACTGGAGCCACACAAATGGGAAAAATACTGTGTTC TTGAAATAGGAGACATGCTCTTGAGGACTGCGATAGGCCAAGTGTCGAGGCCCATG TTCCTATATGTGAGAACCAATGGAACCTCCAAGATCAAGATGAAATGGGGCATGGA ACTGAGGCGCTGCCTTCTTCAGTCTCTTCAGCAGATTGAGAGCATGATTGAGGCCGA GTCTTCTGTCAAAGAGAAAGACATGACCAAGGAATTCTTTGAAAACAAATCGGAAA CATGGCCAATCGGAGAGTCACCCAGGGGAGTGGAGGAAGGCTCTATTGGGAAAGTG TGCAGGACCTTACTGGCAAAATCTGTATTCAACAGTCTATATGCGTCTCCACAACTT GAGGGGTTTTCGGCTGAATCGAGAAAATTGCTTCTCATTGTTCAGGCACTTAGGGAC AACCTGGAACCTGGAACCTTCGATCTTGGGGGGCTATATGAAGCAATCGAGGAGTG CCTGATTAATGATCCCTGGGTTTTGCTTAATGCATCTTGGTTCAACTCCTTCCTCACA CATGCACTGAAGTAGTTGTGGCAATGCTAC |
| Segment 4-VIRUS 3 (SEQ ID NO: 20) |
| AAAAGCAACAAAAGATGAAGGCAATACTAGTAGTTCTGCTATATACATTTGCAACC GCAAATGCAGACACATTATGTATAGGTTATCATGCGAACAATTCAACAGACACTGTA GACACAGTACTAGAAAAGAATGTAACAGTAACACACTCTGTTAACCTTCTAGAAGA CAAGCATAACGGGAAACTATGCAAACTAAGAGGGGTAGCCCCATTGCATTTGGGTA AATGTAACATTGCTGGCTGGATCCTGGGAAATCCAGAGTGTGAATCACTCTCCACAG CAAGCTCATGGTCCTACATTGTGGAAACATCTAGTTCAGACAATGGAACGTGTTACC CAGGAGATTTCATCGATTATGAGGAGCTAAGAGAGCAATTGAGCTCAGTGTCATCAT TTGAAAGGTTTGAGATATTCCCCAAGACAAGTTCATGGCCCAATCATGACTCGAACA AAGGTGTAACGGCAGCATGTCCTCATGCTGGAGCAAAAAGCTTCTACAAAAATTTA ATATGGCTAGTTAAAAAAGGAAATTCATACCCAAAGCTCAGCAAATCCTACATTAAT GATAAAGGGAAAGAAGTCCTCGTGCTATGGGGCATTCACCATCCATCTACTAGTGCT GACCAACAAAGTCTCTATCAGAATGCAGATGCATATGTTTTTGTGGGGTCATCAAGA |
| TACAGCAAGAAGTTCAAGCCGGAAATAGCAATAAGACCCAAAGTGAGGGATCAAG AAGGGAGAATGAACTATTACTGGACACTAGTAGAGCCGGGAGACAAAATAACATTC GAAGCAACTGGAAATCTAGTGGTACCGAGATATGCATTCGCAATGGAAAGAAATGC TGGATCTGGTATTATCATTTCAGATACACCAGTCCACGATTGCAATACAACTTGTCA GACACCCAAGGGTGCTATAAACACCAGCCTCCCATTTCAGAATATACATCCGATCAC AATTGGAAAATGTCCAAAATATGTAAAAAGCACAAAATTGAGACTGGCCACAGGAT TGAGGAATGTCCCGTCTATTCAATCTAGAGGCCTATTTGGGGCCATTGCCGGTTTCA TTGAAGGGGGGTGGACAGGGATGGTAGATGGATGGTACGGTTATCACCATCAAAAT GAGCAGGGGTCAGGATATGCAGCCGACCTGAAGAGCACACAGAATGCCATTGACGA GATTACTAACAAAGTAAATTCTGTTATTGAAAAGATGAATACACAGTTCACAGCAGT AGGTAAAGAGTTCAACCACCTGGAAAAAAGAATAGAGAATTTAAATAAAAAAGTTG ATGATGGTTTCCTGGACATTTGGACTTACAATGCCGAACTGTTGGTTCTATTGGAAA ATGAAAGAACTTTGGACTACCACGATTCAAATGTGAAGAACTTATATGAAAAGGTA AGAAGCCAGCTAAAAAACAATGCCAAGGAAATTGGAAACGGCTGCTTTGAATTTTA CCACAAATGCGATAACACGTGCATGGAAAGTGTCAAAAATGGGACTTATGACTACC CAAAATACTCAGAGGAAGCAAAATTAAACAGAGAAGAAATAGATGGGGTAAAGCT GGAATCAACAAGGATTTACCAGATTTTGGCGATCTATTCAACTGTCGCCAGTTCATT GGTACTGGTAGTCTCCCTGGGGGCAATCAGTTTCTGGATGTGCTCTAATGGGTCTCT ACAGTGTAGAATATGTATTTAACTTTAGGATTTCAGAAGCAT |
| Segment 5-VIRUS 3 (SEQ ID NO: 21) |
| ATCAATCACTGAGTGACATCGAAGCCATGGCGTCTCAAGGCACCAAACGATCATAT GAACAAATGGAGACTGGTGGGGAGCGCCAGGATGCCACAGAAATCAGAGCATCTGT CGGAAGAATGATTGGTGGAATCGGGAGATTCTACATCCAAATGTGCACTGAACTCA AACTCAGTGATTATGATGGACGACTAATCCAGAATAGCATAACAATAGAGAGGATG GTGCTTTCTGCTTTTGATGAGAGAAGAAATAAATACCTAGAAGAGCATCCCAGTGCT GGGAAGGACCCTAAGAAAACAGGAGGACCCATATATAGAAGAATAGACGGAAAGT GGATGAGAGAACTCATCCTTTATGACAAAGAAGAAATAAGGAGAGTTTGGCGCCAA GCAAACAATGGCGAAGATGCAACAGCAGGTCTTACTCATATCATGATTTGGCATTCC AACCTGAATGATGCCACATATCAGAGAACAAGAGCGCTTGTTCGCACCGGAATGGA TCCCAGAATGTGCTCTCTAATGCAAGGTTCAACACTTCCCAGAAGGTCTGGTGCCGC AGGTGCTGCGGTGAAAGGAGTTGGAACAATAGCAATGGAGTTAATCAGAATGATCA AACGTGGAATCAATGACCGAAATTTCTGGAGGGGTGAAAATGGACGAAGGACAAG GGTTGCTTATGAAAGAATGTGCAATATCCTCAAAGGAAAATTTCAAACAGCTGCCCA GAGGGCAATGATGGATCAAGTAAGAGAAAGTCGAAACCCAGGAAACGCTGAGATT GAAGACCTCATTTTCCTGGCACGGTCAGCACTCATTCTGAGGGGATCAGTTGCACAT AAATCCTGCCTGCCTGCTTGTGTGTATGGGCTTGCAGTAGCAAGTGGGCATGACTTT GAAAGGGAAGGGTACTCACTGGTCGGATAGACCCATTCAAATTACTCCAAAACAG CCAAGTGGTCAGCCTGATGAGACCAAATGAAAACCCAGCTCACAAGAGTCAATTGG TGTGGATGGCATGCCACTCTGCTGCATTTGAAGATTTAAGAGTATCAAGTTTCATAA GAGGAAAGAAAGTGATTCCAAGAGGAAAGCTTTCCACAAGAGGGGTCCAGATTGCT |
| TCAAATGAGAATGTGGAAACCATGGACTCCAATACCCTGGAACTAAGAAGCAGATA CTGGGCCATAAGGACCAGGAGTGGAGGAAATACCAATCAACAAAAGGCATCCGCA GGCCAGATCAGTGTGCAGCCTACATTCTCAGTGCAGCGAAATCTCCCTTTTGAAAGA GCAACCGTTATGGCAGCATTCAGCGGGAACAATGAAGGACGGACATCCGACATGCG AACAGAAGTTATAAGAATGATGGAAAGTGCAAAGCCAGAAGATTTGTCCTTCCAGG GGCGGGGAGTCTTCGAGCTCTCGGACGAAAAGGCAACGAACCCGATCGTGCCTTCC TTTGACATGAGTAATGAAGGGTCTTATTTCTTCGGAGACAATGCAGAGGAGTATGAC AGTTGAGGAAAAATACCCTTGTTTCTACT |
| Segment 6-VIRUS 3 (SEQ ID NO: 22) |
| TGATGAATCCAAACCAAAAGATAATAACCATTGGTTCGGTCTGTATGACAATTGGAA TGGCTAACTTAATGTTACAAATTGGAAACATAATCTCAATATGGATTAGCCACTCAA TTCAACTTGGGAATCAAAATCAGATTGAAACATGCAATCAAAGCGTCATTACTTATG AAAACAACACTTGGGTAAATCAGACATATGTTAACATCAGCAACACCAACTTTGCTG CTGGACAGTCAGTGGTTTCCGTGAAATTAGCGGGCAATTCCTCTCTCTGCCCTGTTA GTGGATGGGCTATATACAGTAAAGACAACAGTGTAAGAATCGGTTCCAAGGGGGAT GTGTTTGTCATAAGGGAACCATTCATATCATGCTCCCCCTTGGAATGCAGAACCTTC TTCTTGACTCAAGGGGCCTTGCTAAATGACAAACATTCCAATGGAACCATTAAAGAC AGGAGCCCATATCGAACCCTAATGAGCTGTCCTATTGGTGAAGTTCCCTCTCCATAC AACTCAAGATTTGAGTCAGTCGCTTGGTCAGCAAGTGCTTGTCATGATGGCATCAAT TGGCTAACAATTGGAATTTCTGGCCCAGACAATGGGGCAGTGGCTGTGTTAAAGTAC AACGGCATAATAACAGACACTATCAAGAGTTGGAGAAACAATATATTGAGAACACA AGAGTCTGAATGTGCATGTGTAAATGGTTCTTGCTTTACTGTAATGACCGATGGACC AAGTAATGAACAGGCCTCATACAAGATCTTCAGAATAGAAAAGGGAAAGATAGTCA AATCAGTCGAAATGAATGCCCCTAATTATCACTATGAGGAATGCTCCTGTTATCCTG ATTCTAGTGAAATCACATGTGTGTGCAGGGATAACTGGCATGGCTCGAATCGACCGT GGGTGTCTTTCAACCAGAATCTGGAATATCAGATAGGATACATATGCAGTGGGATTT TCGGAGACAATCCACGCCCTAATGATAAGACAGGCAGTTGTGGTCCAGTATCGTCTA ATGGAGCAAATGGAGTAAAAGGATTTTCATTCAAATACGGCAATGGTGTTTGGATA GGGAGAACTAAAAGCATTAGTTCAAGAAACGGTTTTGAGATGATTTGGGATCCGAA CGGATGGACTGGGACAGACAATAACTTCTCAATAAAGCAAGATATCGTAGGAATAA ATGAGTGGTCAGGATATAGCGGGAGTTTTGTTCAGCATCCAGAACTAACAGGGCTG GATTGTATAAGACCTTGCTTCTGGGTTGAACTAATCAGAGGGCGACCCAAAGAGAA CACAATCTGGACTAGCGGGAGCAGCATATCCTTTTGTGGTGTAAACAGTGACACTGT GGGTTGGTCTTGGCCAGACGGTGCTGAGTTGCCATTTACCATTGACAAGTAACA |
| Segment 7-VIRUS 3 (SEQ ID NO: 23) |
| ATGAGTCTTCTAACCGAGGTCGAAACGTACGTTCTTTCTATCATCCCGTCAGGCCCC CTCAAAGCCGAGATCGCGCAGAGACTGGAAAGTGTCTTTGCAGGAAAGAACACAGA TCTTGAGGCTCTCATGGAATGGCTAAAGACAAGACCAATCTTGTCACCTCTGACTAA GGGAATTTTAGGATTTGTGTTCACGCTCACCGTGCCCAGTGAGCGAGGACTGCAGCG |
| TAGACGCTTTGTCCAAAATGCCCTAAATGGAATGGGGACCCGAACAACATGGATA GAGCAGTTAAACTATACAAGAAGCTCAAAAGAGAAATAACGTTCCATGGGGCCAAG GAGGTGTCACTAAGCTATTCAACTGGTGCACTTGCCAGTTGCATGGGCCTCATATAC AACAGGATGGGAACAGTGACCACAGAAGCTGCTTTTGGTCTAGTGTGTGCCACTTGT GAACAGATTGCTGATTCACAGCATCGGTCTCACAGACAGATGGCTACTACCACCAAT CCACTAATCAGGCATGAAAACAGAATGGTGCTGGCTAGCACTACGGCAAAGGCTAT GGAACAGATGGCTGGATCGAGTGAACAGAAAGGAGAAGACGTCCAAAAGCTGGCA GAAGAGCTGCAAAGCAACATTGGAGTATTGAGATCTCTTGGAGCAAGTCAAAAGAG TGGGGAAGGAATTGCAAAGGATGTAATGGAAGTGCTAAAGCAGAGCTCTATGGGAA ATTCAGCTCTTGTGAAGAAATATCTATGCTCGAACCATTTCAGATTCTTTCAATTTGT TCTTTTATCTTATCAGCTCTCCATTTCATGGCTTGGACAAGGCATTATCAAAAAAGAG GAGAGAAAAGAAAAGGTCCAAACAAAGAGACAAACAGAGAGGTATCAATTTGACA CAGTTACCAAAAAGAAATCCAGGCCAAAGAAACAAAGGAAGTACTCTCTGACAACA AGGGTGACCACAAGGGGCTTTCTGCCGGCGGAGGCCATGGAGGTTGCTAATCAGAC TAGGCAGATGGTACATGCAATGAGAACTATTGGGACTCATCCTAGCTCCAGTGCTGG TCTGAAAGATGACCTTCTTGAAAATTTGCAGGCCTACCAGAAGCGAATGGGAGTGC AGATGCAGCGATTCAAGTCCTCTCGTCATTGCAGCAATATCATTGGGATCTTGCACC TGATATTGTGGATTACTGATCGTCTTTTTTCAAATGTATTTATCGTCGCTTTAAATA CGGTTTGAAAAGAGGGCCTTCTACGGAAGGAGTGCCTGAGTCCATGAGGAAGAAT ATCAACAGGAACAGCAGAGTGCTGTGGATGTTGACGATGGTCATTTTGTCAACATAG AGCTAGAGTAA |
| Segment 8-VIRUS 3 (SEQ ID NO: 24) |
| AACATAATGGACTCCAACACCATGTCAAGCTTTCAGGTAGACTGTTTCCTTTGGCAT ATCCGCAAGCGATTTGCAGACAATGGATTGGGTGATGCCCCATTCCTTGATCGGCTC CGCCGAGATCAAAAGTCCTTAAAAGGAAGAGGCAACACCCTTGGCCTCGATATCGA AACAGCCACTCTTGTTGGGAAACAAATCGTGGAATGGATCTTGAAAGAGGAATCCA GCGAGACACTTAGAATGACAATTGCATCTGTACCTACTTCGCGCTACCTTTCTGACA TGACCCTCGAGGAAATGTCACGAGACTGGTTCATGCTCATGCCTAGGCAAAAGATA ATAGGCCCTCTTTGCGTGCGATTGGACCAGGCGATCATGGAAAAGAACATAGTACT GAAAGCGAACTTCAGTGTAATCTTTAACCGATTAGAGACCTTGATACTACTAAGGGC TTTCACTGAGGAGGGAGCAATAGTTGGAGAAATTTCACCATTACCTTCTCTTCCAGG ACATACTTATGAGGATGTCAAAAATGCAATTGGGGTCCTCATCGGAGGACTTGAATG GAATGGTAACACGGTTCGAGTCTCTGAAAATATACAGAGATTCGCTTGGAGAAACT GTGATGAGAATGGGAGACCTTCACTACCTCCAGAGCAGAAATGAAAAGTGGCGAGA GCAATTGGGACAGAAATTTGAGGAAATAAGGTGGTTAATTGAAGAAATGCGGCACA GATTGAAAGCGACAGAGAATAGTTTCGAACAAATAACATTTATGCAAGCCTTACAA CTACTGCTTGAAGTAGAACAAGAGATAAGAGCTTTCTCGTTTCAGCTTATTTAACTG ATAA |

| **Table 5.** Genome sequence of pAH1N1 virus possessing mutations in NA segment (Virus 4) |
|---|
| Segment 1-VIRUS 4 (SEQ ID NO: 25) |
| TATGGAGAGAATAAAAGAACTGAGAGATCTAATGTCGCAGTCCCGCACTCGCGAGA TACTCACTAAGACCACTGTGGACCATATGGCCATAATCAAAAAGTACACATCAGGA AGGCAAGAGAAGAACCCCGCACTCAGAATGAAGTGGATGATGGCAATGAGATACCC AATTACAGCAGACAAGAGAATAATGGACATGATTCCAGAGAGGAATGAACAAGGA CAAACCCTCTGGAGCAAAACAAACGATGCTGGATCAGACCGAGTGATGGTATCACC TCTGGCCGTAACATGGTGGAATAGGAATGGCCCAACAACAAGTACAGTTCATTACC CTAAGGTATATAAAACTTATTTCGAAAAGGTCGAAAGGTTGAAACATGGTACCTTCG GCCCTGTCCACTTCAGAAATCAAGTTAAAATAAGGAGGAGAGTTGATACAAACCCT GGCCATGCAGATCTCAGTGCCAAGGAGGCACAGGATGTGATTATGGAAGTTGTTTTC CCAAATGAAGTGGGGGCAAGAATACTGACATCAGAGTCACAGCTGGCAATAACAAA AGAGAAGAAAGAAGAGCTCCAGGATTGTAAAATTGCTCCCTTGATGGTGGCGTACA TGCTAGAAAGAGAATTGGTCCGTAAAACAAGGTTTCTCCCAGTAGCCGGCGGAACA GGCAGTGTTTATATTGAAGTGTTGCACTTAACCCAAGGGACGTGCTGGGAGCAGATG TACACTCCAGGAGGAGAAGTGAGAAATGATGATGTTGACCAAAGTTTGATTATCGC TGCTAGAAACATAGTAAGAAGAGCAGCAGTGTCAGCAGACCCATTAGCATCTCTCTT GGAAATGTGCCACAGCACACAGATTGGAGGAGTAAGGATGGTGGACATCCTTAGAC AGAATCCAACTGAGGAACAAGCCGTAGACATATGCAAGGCAGCAATAGGGTTGAGG ATTAGCTCATCTTTCAGTTTTGGTGGGTTCACTTTCAAAAGGACAAGCGGATCATCA GTCAAGAAAGAAGAAGAAGTGCTAACGGGCAACCTCCAAACACTGAAAATAAGAG TACATGAAGGGTATGAAGAATTCACAATGGTTGGGAGAAGAGCAACAGCTATTCTC AGAAAGGCAACCAGGAGATTGATCCAGTTGATAGTAAGCGGGAGAGACGAGCAGT CAATTGCTGAGGCAATAATTGTGGCCATGGTATTCTCACAAGAGGATTGCATGATCA AGGCAGTTAGGGGCGATCTGAACTTTGTCAATAGGGCAAACCAGCGACTGAACCCC ATGCACCAACTCTTGAGGCATTTCCAAAAAGATGCAAAAGTGCTTTTCCAGAACTGG GGAATTGAATCCATCGACAATGTGATGGGAATGATCGGAATACTGCCCGACATGAC CCCAAGCACGGAGATGTCGCTGAGAGGATAAGAGTCAGCAAAATGGGAGTAGAT GAATACTCCAGCACGGAGAGAGTGGTAGTGAGTATTGACCGATTTTTAAGGGTTAG AGATCAAAGAGGGAACGTACTATTGTCTCCCGAAGAAGTCAGTGAAACGCAAGGAA CTGAGAAGTTGACAATAACTTATTCGTCATCAATGATGTGGGAGATCAATGGCCCTG AGTCAGTGCTAGTCAACACTTATCAATGGATAATCAGGAACTGGGAAATTGTGAAA ATTCAATGGTCACAAGATCCCACAATGTTATACAACAAAATGGAATTTGAACCATTT CAGTCTCTTGTCCCTAAGGCAACCAGAAGCCGGTACAGTGGATTCGTAAGGACACTG TTCCAGCAAATGCGGGATGTGCTTGGGACATTTGACACTGTCCAAATAATAAAACTT CTCCCCTTTGCTGCTGCTCCACCAGAACAGAGTAGGATGCAATTTTCCTCATTGACTG TGAATGTGAGAGGATCAGGGTTGAGGATACTGGTAAGAGGCAATTCTCCAGTATTC AATTACAACAAGGCAACCAAACGACTTACAGTTCTTGGAAAGGATGCAGGTGCATT GACTGAAGATCCAGATGAAGGCACATCTGGGGTGGAGTCTGCTGTCCTGAGAGGAT |
| TTCTCATTTTGGGCAAAGAAGACAAGAGATATGGCCCAGCATTAAGCATCAATGAA CTGAGCAATCTTGCAAAAGGAGAGAAAGCTAATGTGCTAATTGGGCAAGGGGACGT AGTGTTGGTAATGAAACGAAAACGGACTCTAGCATACTTACTGACAGCCAGACAG CGACCAAAAGAATTCGGATGGCCATCAATTAGTGTCGAATTGTT |
| Segment 2-VIRUS 4 (SEQ ID NO: 26) |
| TTGAATGGATGTCAATCCGACTCTACTTTTCCTAAAATTCCAGCGCAAAATGCCAT AAGCACCACATTCCCTTATACTGGAGATCCTCCATACAGCCATGGAACAGGAACAG GATACACCATGGACACAGTAAACAGAACACACCAATACTCAGAAAGGGAAAGTG GACGACAAACACAGAGACTGGTGCACCCCAGCTCAACCCGATTGATGGACCACTAC CTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATG GAAGTTGTTCAACAAACAAGGGTAGATAAACTAACTCAAGGTCGCCAGACTTATGA TTGGACATTAAACAGAAATCAACCGGCAGCAACTGCATTGGCCAACACCATAGAAG TCTTTAGATCGAATGGCCTAACAGCTAATGAGTCAGGAAGGCTAATAGATTTCTTAA AGGATGTAATGGAATCAATGAACAAAGAGGAAATAGAGATAACAACCCACTTTCAA AGAAAAAGGAGAGTAAGAGACAACATGACCAAGAAGATGGTCACGCAAAGAACAA TAGGGAAGAAAAAACAAAGACTGAATAAGAGAGGCTATCTAATAAGAGCACTGAC ATTAAATACGATGACCAAAGATGCAGAGAGAGGCAAGTTAAAAAGAAGGGCTATC GCAACACCTGGGATGCAGATTAGAGGTTTCGTATACTTTGTTGAAACTTTAGCTAGG AGCATTTGCGAAAAGCTTGAACAGTCTGGGCTCCCAGTAGGGGGCAATGAAAAGAA GGCCAAACTGGCAAATGTTGTGAGAAAGATGATGACTAATTCACAAGACACAGAGA TTTCTTTCACAATCACTGGGGACAACACTAAGTGGAATGAAAATCAAAATCCTCGAA TGTTCCTGGCGATGATTACATATATCACCAGAAATCAACCCGAGTGGTTCAGAAACA TCCTGAGCATGGCACCCATAATGTTCTCAAACAAAATGGCAAGACTAGGGAAAGGG TACATGTTCGAGAGTAAAAGAATGAAGATTCGAACACAAATACCAGCAGAAATGCT AGCAAGCATTGACCTGAAGTACTTCAATGAATCAACAAAGAAGAAAATTGAGAAAA TAAGGCCTCTTCTAATAGATGGCACAGCATCACTGAGTCCTGGGATGATGATGGGCA TGTTCAACATGCTAAGTACGGTCTTGGGAGTCTCGATACTGAATCTTGGACAAAAGA AATACACCAAGACAATATACTGGTGGGATGGGCTCCAATCATCCGACGATTTTGCTC TCATAGTGAATGCACCAAACCATGAGGGAATACAAGCAGGAGTGGACAGATTCTAC AGGACCTGCAAGTTAGTGGGAATCAACATGAGCAAAAAGAAGTCCTATATAAATAA GACAGGGACATTTGAATTCACAAGCTTTTTTATCGCTATGGATTTGTGGCTAATTTT AGCATGGAGCTACCCAGCTTTGGAGTGTCTGGAGTAAATGAATCAGCTGACATGAG TATTGGAGTAACAGTGATAAAGAACAACATGATAAACAATGACCTTGGACCTGCAA CGGCCCAGATGGCTCTTCAATTGTTCATCAAAGACTACAGATACACATATAGGTGCC ATAGGGGAGACACACAAATTCAGACGAGAAGATCATTTGAGTTAAAGAAGCTGTGG GATCAAACCCAATCAAAGGTAGGGCTATTAGTATCAGATGGAGGACCAAACTTATA CAATATACGGAATCTTCACATTCCTGAAGTCTGCTTAAAATGGGAGCTAATGGATGA TGATTATCGGGGAAGACTTTGTAATCCCCTGAATCCCTTTGTCAGTCATAAAGAGAT |
| TGATTCTGTAAACAATGCTGTGGTAATGCCAGCCCATGGTCCAGCCAAAAGCATGGA ATATGATGCCGTTGCAACTACACATTCCTGGATTCCCAAGAGGAATCGTTCTATTCT CAACACAAGCCAAAGGGGAATTCTTGAGGATGAACAGATGTACCAGAAGTGCTGCA ATCTATTCGAGAAATTTTTCCCTAGCAGTTCATATAGGAGACCGGTTGGAATTTCTA GCATGGTGGAGGCCATGGTGTCTAGGGCCCGGATTGATGCCAGGGTCGACTTCGAG TCTGGACGGATCAAGAAAGAAGAGTTCTCTGAGATCATGAAGATCTGTTCCACCATT GAAGAACTCAGACGGCAAAAATAATGAATTTAACTTGTCCTTCATGAAA |
| Segment 3-VIRUS 4 (SEQ ID NO: 27) |
| TCCAAAATGGAAGACTTTGTGCGACAATGCTTCAATCCAATGATCGTCGAGCTTGCG GAAAAGGCAATGAAAGAATATGGGGAAGATCCGAAAATCGAAACTAACAAGTTTG CTGCAATATGCACACATTTGGAAGTTTGTTTCATGTATTCGGATTTCCATTTCATCGA CGAACGGGGTGAATCAATAATTGTAGAATCTGGTGACCCGAATGCACTATTGAAGC ACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGAT TACAAAGAGAACCGGTTCATTGAAATTGGAGTAACACGGAGGGAAGTCCACATATA TTACCTAGAGAAAGCCAACAAAATAAAATCTGAGAAGACACACATTCACATCTTTTC ATTCACTGGAGAGGAGATGGCCACCAAAGCGGACTACACCCTTGACGAAGAGAGCA GGGCAAGAATCAAAACTAGGCTTTTCACTATAAGACAAGAAATGGCCAGTAGGAGT CTATGGGATTCCTTTCGTCAGTCCGAAAGAGGCGAAGAGACAATTGAAGAAAAATT TGAGATTACAGGAACTATGCGCAAGCTTGCCGACCAAAGTCTCCCACCGAACTTCTC CAGCCTTGAAAACTTTAGAGCCTATGTAGATGGATTCGAGCCGAACGGCTGCATTGA GGGCAAGCTTTCCCAAATGTCAAAAGAAGTGAACGCCAAAATTGAACCATTCTTGA GGACGACACCACGCCCCCTCAGATTGCCTGATGGGCCTCTTTGCCATCAGCGGTCAA AGTTCCTGCTGATGGATGCTCTGAAATTAAGTATTGAAGACCCGAGTCACGAGGGG GAGGGAATACCACTATATGATGCAATCAAATGCATGAAGACATTCTTTGGCTGGAA AGAGCCTAACATAGTCAAACCACATGAGAAAGGCATAAATCCCAATTACCTCATGG CTTGGAAGCAGGTGCTAGCAGAGCTACAGGACATTGAAAATGAAGAGAAGATCCCA AGGACAAAGAACATGAAGAGAACAAGCCAATTGAAGTGGGCACTCGGTGAAAATA TGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATT CAATAAGGCATGTGAATTGACTGATTCAAGCTGGATAGAACTTGATGAAATAGGAG AAGATGTTGCCCCGATTGAACATATCGCAAGCATGAGGAGGAACTATTTTACAGCA GAAGTGTCCCACTGCAGGGCTACTGAATACATAATGAAGGGAGTGTACATAAATAC GGCCTTGCTCAATGCATCCTGTGCAGCCATGGATGACTTTCAGCTGATCCCAATGAT AAGCAAATGTAGGACCAAAGAAGGAAGACGGAAAACAAACCTGTATGGGTTCATTA TAAAAGGAAGGTCTCATTTGAGAAATGATACTGATGTGGTGAACTTTGTAAGTATGG AGTTCTCACTCACTGACCCGAGACTGGAGCCACACAAATGGGAAAAATACTGTGTTC TTGAAATAGGAGACATGCTCTTGAGGACTGCGATAGGCCAAGTGTCGAGGCCCATG TTCCTATATGTGAGAACCAATGGAACCTCCAAGATCAAGATGAAATGGGGCATGGA |
| ACTGAGGCGCTGCCTTCTTCAGTCTCTTCAGCAGATTGAGAGCATGATTGAGGCCGA GTCTTCTGTCAAAGAGAAAGACATGACCAAGGAATTCTTTGAAAACAAATCGGAAA CATGGCCAATCGGAGAGTCACCCAGGGGAGTGGAGGAAGGCTCTATTGGGAAAGTG TGCAGGACCTTACTGGCAAAATCTGTATTCAACAGTCTATATGCGTCTCCACAACTT GAGGGGTTTTCGGCTGAATCGAGAAAATTGCTTCTCATTGTTCAGGCACTTAGGGAC AACCTGGAACCTGGAACCTTCGATCTTGGGGGCTATATGAAGCAATCGAGGAGTG CCTGATTAATGATCCCTGGGTTTTGCTTAATGCATCTTGGTTCAACTCCTTCCTCACA CATGCACTGAAGTAGTTGTGGCAATGCTAC |
| Segment 4-VIRUS 4 (SEQ ID NO: 28) |
| AAAAGCAACAAAAGATGAAGGCAATACTAGTAGTTCTGCTATATACATTTGCAACC GCAAATGCAGACACATTATGTATAGGTTATCATGCGAACAATTCAACAGACACTGTA GACACAGTACTAGAAAAGAATGTAACAGTAACACACTCTGTTAACCTTCTAGAAGA CAAGCATAACGGGAAACTATGCAAACTAAGAGGGGTAGCCCCATTGCATTTGGGTA AATGTAACATTGCTGGCTGGATCCTGGGAAATCCAGAGTGTGAATCACTCTCCACAG CAAGCTCATGGTCCTACATTGTGGAAACATCTAGTTCAGACAATGGAACGTGTTACC CAGGAGATTTCATCGATTATGAGGAGCTAAGAGAGCAATTGAGCTCAGTGTCATCAT TTGAAAGGTTTGAGATATTCCCCAAGACAAGTTCATGGCCCAATCATGACTCGAACA AAGGTGTAACGGCAGCATGTCCTCATGCTGGAGCAAAAAGCTTCTACAAAAATTTA ATATGGCTAGTTAAAAAAGGAAATTCATACCCAAAGCTCAGCAAATCCTACATTAAT GATAAAGGGAAAGAAGTCCTCGTGCTATGGGGCATTCACCATCCATCTACTAGTGCT GACCAACAAAGTCTCTATCAGAATGCAGATGCATATGTTTTTGTGGGGTCATCAAGA TACAGCAAGAAGTTCAAGCCGGAAATAGCAATAAGACCCAAAGTGAGGGATCAAG AAGGGAGAATGAACTATTACTGGACACTAGTAGAGCCGGGAGACAAAATAACATTC GAAGCAACTGGAAATCTAGTGGTACCGAGATATGCATTCGCAATGGAAAGAAATGC TGGATCTGGTATTATCATTTCAGATACACCAGTCCACGATTGCAATACAACTTGTCA GACACCCAAGGGTGCTATAAACACCAGCCTCCCATTTCAGAATATACATCCGATCAC AATTGGAAAATGTCCAAAATATGTAAAAAGCACAAAATTGAGACTGGCCACAGGAT TGAGGAATGTCCCGTCTATTCAATCTAGAGGCCTATTTGGGGCCATTGCCGGTTTCA TTGAAGGGGGGTGGACAGGGATGGTAGATGGATGGTACGGTTATCACCATCAAAAT GAGCAGGGGTCAGGATATGCAGCCGACCTGAAGAGCACACAGAATGCCATTGACGA GATTACTAACAAAGTAAATTCTGTTATTGAAAAGATGAATACACAGTTCACAGCAGT AGGTAAAGAGTTCAACCACCTGGAAAAAAGAATAGAGAATTTAAATAAAAAAGTTG ATGATGGTTTCCTGGACATTTGGACTTACAATGCCGAACTGTTGGTTCTATTGGAAA ATGAAAGAACTTTGGACTACCACGATTCAAATGTGAAGAACTTATATGAAAAGGTA AGAAGCCAGCTAAAAAACAATGCCAAGGAAATTGGAAACGGCTGCTTTGAATTTTA CCACAAATGCGATAACACGTGCATGGAAAGTGTCAAAAATGGGACTTATGACTACC CAAAATACTCAGAGGAAGCAAAATTAAACAGAGAAGAAATAGATGGGGTAAAGCT GGAATCAACAAGGATTTACCAGATTTTGGCGATCTATTCAACTGTCGCCAGTTCATT GGTACTGGTAGTCTCCCTGGGGGCAATCAGTTTCTGGATGTGCTCTAATGGGTCTCT |
| ACAGTGTAGAATATGTATTTAACTTTAGGATTTCAGAAGCAT |
| Segment 5-VIRUS 4 (SEQ ID NO: 29) |
| ATCAATCACTGAGTGACATCGAAGCCATGGCGTCTCAAGGCACCAAACGATCATAT GAACAAATGGAGACTGGTGGGGAGCGCCAGGATGCCACAGAAATCAGAGCATCTGT CGGAAGAATGATTGGTGGAATCGGGAGATTCTACATCCAAATGTGCACTGAACTCA AACTCAGTGATTATGATGGACGACTAATCCAGAATAGCATAACAATAGAGAGGATG GTGCTTTCTGCTTTTGATGAGAGAAGAAATAAATACCTAGAAGAGCATCCCAGTGCT GGGAAGGACCCTAAGAAAACAGGAGGACCCATATATAGAAGAATAGACGGAAAGT GGATGAGAGAACTCATCCTTTATGACAAAGAAGAAATAAGGAGAGTTTGGCGCCAA GCAAACAATGGCGAAGATGCAACAGCAGGTCTTACTCATATCATGATTTGGCATTCC AACCTGAATGATGCCACATATCAGAGAACAAGAGCGCTTGTTCGCACCGGAATGGA TCCCAGAATGTGCTCTCTAATGCAAGGTTCAACACTTCCCAGAAGGTCTGGTGCCGC AGGTGCTGCGGTGAAAGGAGTTGGAACAATAGCAATGGAGTTAATCAGAATGATCA AACGTGGAATCAATGACCGAAATTTCTGGAGGGGTGAAAATGGACGAAGGACAAG GGTTGCTTATGAAAGAATGTGCAATATCCTCAAAGGAAAATTTCAAACAGCTGCCCA GAGGGCAATGATGGATCAAGTAAGAGAAAGTCGAAACCCAGGAAACGCTGAGATT GAAGACCTCATTTTCCTGGCACGGTCAGCACTCATTCTGAGGGGATCAGTTGCACAT AAATCCTGCCTGCCTGCTTGTGTGTATGGGCTTGCAGTAGCAAGTGGGCATGACTTT GAAAGGGAAGGGTACTCACTGGTCGGGATAGACCCATTCAAATTACTCCAAAACAG CCAAGTGGTCAGCCTGATGAGACCAAATGAAAACCCAGCTCACAAGAGTCAATTGG TGTGGATGGCATGCCACTCTGCTGCATTTGAAGATTTAAGAGTATCAAGTTTCATAA GAGGAAAGAAAGTGATTCCAAGAGGAAAGCTTTCCACAAGAGGGGTCCAGATTGCT TCAAATGAGAATGTGGAAACCATGGACTCCAATACCCTGGAACTAAGAAGCAGATA CTGGGCCATAAGGACCAGGAGTGGAGGAAATACCAATCAACAAAGGCATCCGCA GGCCAGATCAGTGTGCAGCCTACATTCTCAGTGCAGCGAAATCTCCCTTTTGAAAGA GCAACCGTTATGGCAGCATTCAGCGGGAACAATGAAGGACGGACATCCGACATGCG AACAGAAGTTATAAGAATGATGGAAAGTGCAAAGCCAGAAGATTTGTCCTTCCAGG GGCGGGGAGTCTTCGAGCTCTCGGACGAAAAGGCAACGAACCCGATCGTGCCTTCC TTTGACATGAGTAATGAAGGGTCTTATTTCTTCGGAGACAATGCAGAGGAGTATGAC AGTTGAGGAAAAATACCCTTGTTTCTACT |
| Segment 6-VIRUS 4 (SEQ ID NO: 30) |
| ATGAATCCAAACCAAAAGATAATAACCATTGGTTCGGTCTGTATGACGATTGGAATG GCTAACTTAATATTACAAATTGGAAACATAATCTCAATATGGATTAGCCACTCAATT CAACTTGGGAATCAAAATCAGATTGAAACATGCAATCAAAGCGTCATTACTTATGA AAACAACACTTGGGTAAATCAGACATATGTTAACATCAGCAACACCAACTTTGCTGC TGGACAGTCAGTGGTTTCCGTGAAATTAGCGGGCAATTCCTCTCTCTGCCCTGTTAGT GGATGGGCTATATACAGTAAAGACAACAGTATAAGAATCGGTTCCAAGGGGGATGT GTTTGTCATAAGGGAACCATTCATATCATGCTCCCCCTTGGAATGCAGAACCTTCTTC |
| TTGACTCAAGGGGCCTTGCTAAATGACAAACATTCCAATGGAACCATTAAAGACAG GAGCCCATATCGAACCCTAATGAGCTGTCCTATTGGTGAAGTTCCCTCTCCATACAA CTCAAGATTTGAGTCAGTCGCTTGGTCAGCAAGTGCTTGTCATGATGGCATCAATTG GCTAACAATTGGAATTTCTGGCCCAGACAATGGGGCAGTGGCTGTGTTAAAGTACA ACGGCATAATAACAGACACTATCAAGAGTTGGAGAAACAATATATTGAGAACACAA GAGTCTGAATGTGCATGTGTAAATGGTTCTTGCTTTACTGTAATGACCGATGGACCA AGTGATGGACAGGCCTCATACAAGATCTTCAGAATAGAAAAGGGAAAGATAGTCAA ATCAGTCGAAATGAATGCCCCTAATTATTACTATGAGGAATGCTCCTGTTATCCTGA TTCTAGTGAAATCACATGTGTGTGCAGGGATAACTGGCATGGCTCGAATCGACCGTG GGTGTCTTTCAACCAGAATCTGGAATATCAGATAGGATACATATGCAGTGGGATTTT CGGAGACAATCCACGCCCTAATGATAAGACAGGCAGTTGTGGTCCAGTATCGTCTA ATGGAGCAAATGGAGTAAAAGGATTTTCATTCAAATACGGCAATGGTGTTTGGATA GGGAGAACTAAAAGCATTAGTTCAAGAAACGGTTTTGAGATGATTTGGGATCCGAA CGGATGGACTGGGACAGACAATAACTTCTCAATAAAGCAAGATATCGTAGGAATAA ATGAGTGGTCAGGATATAGCGGGAGTTTTGTTCAGCATCCAGAACTAACAGGGCTG GATTGTATAAGACCTTGCTTCTGGGTTGAACTAATCAGAGGGCGACCCAAAGAGAA CACAATCTGGACTAGCGGGAGCAGCATATCCTTTTGTGGTGTAAACAGTGACACTGT GGGTTGGTCTTGGCCAGACGGTGCTGAGTTGCCATTTACCATTGACAAGTAA |
| Segment 7-VIRUS 4 (SEQ ID NO: 31) |
| TAAAGATGAGTCTTCTAACCGAGGTCGAAACGTACGTTCTTTCTATCATCCCGTCAG GCCCCCTCAAAGCCGAGATCGCGCAGAGACTGGAAAGTGTCTTTGCAGGAAAGAAC ACAGATCTTGAGGCTCTCATGGAATGGCTAAAGACAAGACCAATCTTGTCACCTCTG ACTAAGGGAATTTTAGGATTTGTGTTCACGCTCACCGTGCCCAGTGAGCGAGGACTG CAGCGTAGACGCTTTGTCCAAAATGCCCTAAATGGGAATGGGGACCCGAACAACAT GGATAGAGCAGTTAAACTATACAAGAAGCTCAAAAGAGAAATAACGTTCCATGGGG CCAAGGAGGTGTCACTAAGCTATTCAACTGGTGCACTTGCCAGTTGCATGGGCCTCA TATACAACAGGATGGGAACAGTGACCACAGAAGCTGCTTTTGGTCTAGTGTGTGCCA CTTGTGAACAGATTGCTGATTCACAGCATCGGTCTCACAGACAGATGGCTACTACCA CCAATCCACTAATCAGGCATGAAAACAGAATGGTGCTGGCTAGCACTACGGCAAAG GCTATGGAACAGATGGCTGGATCGAGTGAACAGGCAGCGGAGGCCATGGAGGTTGC TAATCAGACTAGGCAGATGGTACATGCAATGAGAACTATTGGGACTCATCCTAGCTC CAGTGCTGGTCTGAAAGATGACCTTCTTGAAAATTTGCAGGCCTACCAGAAGCGAAT GGGAGTGCAGATGCAGCGATTCAAGTGATCCTCTCGTCATTGCAGCAAATATCATTG GGATCTTGCACCTGATATTGTGGATTACTGATCGTCTTTTTTTCAAATGTATTTATCG TCGCTTTAAATACGGTTTGAAAAGAGGGCCTTCTACGGAAGGAGTGCCTGAGTCCAT GAGGGAAGAATATCAACAGGAACAGCAGAGTGCTGTGGATGTTGACGATGGTCATT TTGTCAACATAGAGCTAGAGTAA |
| Segment 8-VIRUS 4 (SEQ ID NO: 32) |
| AACATAATGGACTCCAACACCATGTCAAGCTTTCAGGTAGACTGTTTCCTTTGGCAT ATCCGCAAGCGATTTGCAGACAATGGATTGGGTGATGCCCCATTCCTTGATCGGCTC CGCCGAGATCAAAAGTCCTTAAAAGGAAGAGGCAACACCCTTGGCCTCGATATCGA AACAGCCACTCTTGTTGGGAAACAAATCGTGGAATGGATCTTGAAAGAGGAATCCA GCGAGACACTTAGAATGACAATTGCATCTGTACCTACTTCGCGCTACCTTTCTGACA TGACCCTCGAGGAAATGTCACGAGACTGGTTCATGCTCATGCCTAGGCAAAAGATA ATAGGCCCTCTTTGCGTGCGATTGGACCAGGCGATCATGGAAAAGAACATAGTACT GAAAGCGAACTTCAGTGTAATCTTTAACCGATTAGAGACCTTGATACTACTAAGGGC TTTCACTGAGGAGGGAGCAATAGTTGGAGAAATTTCACCATTACCTTCTCTTCCAGG ACATACTTATGAGGATGTCAAAAATGCAATTGGGGTCCTCATCGGAGGACTTGAATG GAATGGTAACACGGTTCGAGTCTCTGAAAATATACAGAGATTCGCTTGGAGAAACT GTGATGAGAATGGGAGACCTTCACTACCTCCAGAGCAGAAATGAAAAGTGGCGAGA GCAATTGGGACAGAAATTTGAGGAAATAAGGTGGTTAATTGAAGAAATGCGGCACA GATTGAAAGCGACAGAGAATAGTTTCGAACAAATAACATTTATGCAAGCCTTACAA CTACTGCTTGAAGTAGAACAAGAGATAAGAGCTTTCTCGTTTCAGCTTATTTAACTG ATAA |

The amino acid sequences of the four viral isolates were deduced from the nucleotide sequence determination. Virus 1 carried mutations of interest in HA, NA and MA proteins. Virus 2 carried mutations of interest in NA and MA proteins. Virus 3 carried mutations of interest in NA protein. Virus 4 carried mutations of interest in MA protein.

The HA sequences were analyzed for mutations of interest relative to wild type pandemic influenza A H1N1 sequence. All four isolates carried mutations P83S and I321V that appear to be prevalent in clade 7 of the A/H1N1 pandemic strain.

Virus 1 also has (i) a clade 7 characteristic S203T mutation, and (ii) a D222E mutation at the 222 position of the HA sequence. At positions 222-223 virus I has E-R amino acids in place of the wild type D-Q. An exact match for the HA sequence of Virus 1 is found in Influenza A virus (A/Novgorod/01/2009(H1N1)) HA protein.

| **Segment 4 - HA** |
|---|
| **Wild Type Influenza A/California/07/2009 Sequence (FJ966974)** |
| MKAILVVLLY TFATANADTL CIGYHANNST DTVDTVLEKN VTVTHSVNLL EDKHNGKLCK LRGVAPLHLG KCNIAGWILG NPECESLSTA SSWSYIVET[?] SSDNGTCYPG DFIDYEELRE QLSSVSSFER FEIFPKTSSW PNHDSNKGVT AACPHAGAKS FYKNLIWLVK KGNSYPKLSK SYINDKGKEV LVLWGIHHPS TSADQQSLYQ NADAYVFVG[?] SRYSKKFKPE IAIRPKVR[?][?] EGRMNYYWTL VEPGDKITFE ATGNLVVPRY AFAMERNAGS GIIISDTPVH DCNTTCQTPK GAINTSLPFQ NIHPITIGKC PKYVKSTKLR LATGLRN[?]PS IQSRGLFGAI AGFIEGGWTG MVDGWYGYHH QNEQGSGYAA DLKSTQNAID EITNKVNSVI EKMNTQFTAV GKEFNHLEKR IENLNKKVDD GFLDIWTYNA ELLVLLENER TLDYHDSNVK NLYEKVRSQL KNNAKEIGNG CFEFYHKCDN TCMESVKNGT YDYPKYSEEA KLNREEIDGV KLESTRIYQI LAIYSTVASS LVLVVSLGAI SFWMCSNGSL QCRICI (SEQ ID NO: 33) |

| **Virus 1** |
|---|
| MKAILVVLLY TFATANADTL CIGYHANNST DTVDTVLEKN VTVTHSVNLL EDKHNGKLCK 60 LRGVAPLHLG KCNIAGWILG NPECESLSTA SSWSYIVETS SSDNGTCYPG DFIDYEELRE 120 QLSSVSSFER FEIFPKTSSW PDHDSNKGVT AACPHAGAKS FYKNLIWLVK KGNSYPKLSK 180 SYINDKGKEV LVLWGIHHPS TSADQQSLYQ NADAYVFVG[?] SRYSKKFKPE IAIRPKVR[?]R 240 EGRMNYYWTL VEPGDKITFE ATGNLVVPRY AFAMERNAGS GIIISDTPVH DCNTTCQTPK 300 GAINTSLPFQ NIHPITIGKC PKYVKSTKLR LATGLRNVPS IQSRGLFGAI AGFIEGGWTG 360 MVDGWYGYHH QNEQGSGYAA DLKSTQNAID EITNKVNSVI EKMNTQFTAV GKEFNHLEKR 420 IENLNKKVDD GFLDIWTYNA ELLVLLENER TLDYHDSNVK NLYEKVRSQL KNNAKEIGNG 480 CFEFYHKCDN TCMESVKNGT YDYPKYSEEA KLNREEIDGV KLESTRIYQI LAIYSTVASS 540 LVLVVSLGAI SFWMCSNGSL QCRICI (SEQ ID NO: 34) |
| 566 |

| **Virus 2** |
|---|
| MKAILVVLLY TFATANADTL CIGYHANNST DTVDTVLEKN VTVTHSVNLL EDKHNGKLCK LRGVAPLHLG KCNIAGWILG NPECESLSTA SSWSYIVETS SSDNGTCYPG DFIDYEELRE QLSSVSSFER FEIFPKTSSW PNHDSNKGVT AACPHAGAKS FYKNLIWLVK KGNSYPKLSK SYINDKGKEV LVLWGIHHPS TSADQQSLYQ NADAYVFVG[?] SRYSKKFKPE IAIRPKVR[?] EGRMNYYWTL VEPGDKITFE ATGNLVVPRY AFAMERNAGS GIIISDTPVH DCNTTCQTPK GAINTSLPFQ NIHPITIGKC PKYVKSTKLR LATGLRNVPS IQSRGLFGAI AGFIEGGWTG MVDGWYGYHH QNEQGSGYAA DLKSTQNAID EITNKVNSVI EKMNTQFTAV GKEFNHLEKR IENLNKKVDD GFLDIWTYNA ELLVLLENER TLDYHDSNVK NLYEKVRSQL KNNAKEIGNG CFEFYHKCDN TCMESVKNGT YDYPKYSEEA KLNREEIDGV KLESTRIYQI LAIYSTVASS LVLVVSLGAI SFWMCSNGSL QCRICI (SEQ ID NO: 35) |

| **Virus 3** |
|---|
| MKAILVVLLY TFATANADTL CIGYHANNST DTVDTVLEKN VTVTHSVNLL EDKHNGKLCK LRGVAPLHLG KCNIAGWILG NPECESLSTA SSWSYIVETS SSDNGTCYPG DFIDYEELRE QLSSVSSFER FEIFPKTSSW PNHDSNKGVT AACPHAGAKS FYKNLIWLVK KGNSYPKLSK SYINDKGKEV LVLWGIHHPS TSADQQSLYQ NADAYVFVG[?] SRYSKKFKPE IAIRPKVR[?] EGRMNYYWTL VEPGDKITFE ATGNLVVPRY AFAMERNAGS GIIISDTPVH DCNTTCQTPK GAINTSLPFQ NIHPITIGKC PKYVKSTKLR LATGLRNVPS IQSRGLFGAI AGFIEGGWTG MVDGWYGYHH QNEQGSGYAA DLKSTQNAID EITNKVNSVI EKMNTQFTAV GKEFNHLEKR IENLNKKVDD GFLDIWTYNA ELLVLLENER TLDYHDSNVK NLYEKVRSQL KNNAKEIGNG CFEFYHKCDN TCMESVKNGT YDYPKYSEEA KLNREEIDGV KLESTRIYQI LAIYSTVASS LVLVVSLGAI SFWMCSNGSL QCRICI (SEQ ID NO: 36) |

| **Virus 4** |
|---|
| MKAILVVLLY TFATANADTL CIGYHANNST DTVDTVLEKN VTVTHSVNLL EDKHNGKLCK LRGVAPLHLG KCNIAGWILG NPECESLSTA SSWSYIVETS SSDNGTCYPG DFIDYEELRE QLSSVSSFER FEIFPKTSSW PNHDSNKGVT AACPHAGAKS FYKNLIWLVK KGNSYPKLSK SYINDKGKEV LVLWGIHHPS TSADQQSLYQ NADAYVFVGS SRYSKKFKPE IAIRPKVR[?][?] EGRMNYYWTL VEPGDKITFE ATGNLVVPRY AFAMERNAGS GIIISDTPVH DCNTTCQTPK GAINTSLPFQ NIHPITIGKC PKYVKSTKLR LATGLRNVPS IQSRGLFGAI AGFIEGGWTG MVDGWYGYHH QNEQGSGYAA DLKSTQNAID EITNKVNSVI EKMNTQFTAV GKEFNHLEKR IENLNKKVDD GFLDIWTYNA ELLVLLENER TLDYHDSNVK NLYEKVRSQL KNNAKEIGNG CFEFYHKCDN TCMESVKNGT YDYPKYSEEA KLNREEIDGV KLESTRIYQI LAIYSTVASS LVLVVSLGAI SFWMCSNGSL QCRICI (SEQ ID NO: 37) |

Viruses 1, 2 and 4 carry mutations at residues I106V, D248N, Y275H of the NA segment (6) relative to wild type A/California/07/2009 Sequence. The Y275H mutation is associated with oseltamivir resistance. Exact matches of the NA sequence found in these three isolates are found in other isolates.

The current wild type vaccine is less effective against Virus 1 which carries both HA and NA mutations at these specific sites. (Puzelli et al., Transmission of Hemagglutinin D222G mutant strain of pandemic (H1N1) 2009 virus. Emerging Infectious Diseases 16(5): 863-865 (2010). A vaccine generated by a virus (such as virus 1) carrying both hypervirulence (D222G/E in the HA region) and oseltamivir resistance (Y275H in the NA region) overcomes the lack of efficiency of wild type H1N1 pandemic vaccines and offers protection against both hypervirulent and oseltamivir-resistant viruses.

| **Segment 6 - NA** |
|---|
| **A/California/07/2009 Sequence** |
| MNPNQKIITI GSVCMTIGMA NLILQIGNII SIWISHSIQL GNQNQIETCN 050 QSVITYENNT WVNQTYVNIS NTNFAAGQSV VSVKLAGNSS LCPVSGWAIY 100 SKDNS[?]RIGS KGDVFVIREP FISCSPLECR TFFLTQGALL NDKHSNGTIK 150 DRSPYRTLMS CPIGEVPSPY NSRFESVAWS ASACHDGINW LTIGISGPDN 200 GAVAVLKYNG IITDTIKSWR NNILRTQESE CACVNGSCFT VMTDGPS[?]GQ 250 ASYKIFRIEK GKIVKSVEMN APNY[?]YEECS CYPDSSEITC VCRDNWHGSN 300 RPWVSFNQNL EYQIGYICSG IFGDNPRPND KTGSCGPVSS NGANGVKGFS 350 FKYGNGVWIG RTKSISSRNG FEMIWDPNGW TGTDNNFSIK QDIVGINEWS 400 GYSGSFVQHP ELTGLDCIRP CFWVELIRGR PKENTIWTSG SSISFCGVNS 450 DTVGWSWPDG AELPFTIDK (SEQ ID NO: 38) 469 |

| **Virus 1** |
|---|
| MNPNQKIITI GSVCMTIGMA NLILQIGNII SIWISHSIQL GNQNQIETCN 50 QSVITYENNT WVNQTYVNIS NTNFAAGQSV VSVKLAGNSS LCPVSGWAIY 100 SKDNS[?]RIGS KGDVFVIREP FISCSPLECR TFFLTQGALL NDKHSNGTIK 150 DRSPYRTLMS CPIGEVPSPY NSRFESVAWS ASACHDGINW LTIGISGPDN 200 GAVAVLKYNG IITDTIKSWR NNILRTQESE CACVNGSCFT VMTDGPS[?]GQ 250 ASYKIFRIEK GKIVKSVEMN APNY[?]YEECS CYPDSSEITC VCRDNWHGSN 300 RPWVSFNQNL EYQIGYICSG IFGDNPRPND KTGSCGPVSS NGANGVKGFS 350 FKYGNGVWIG RTKSISSRNG FEMIWDPNGW TGTDNNFSIK QDIVGINEWS 400 GYSGSFVQHP ELTGLDCIRP CFWVELIRGR PKENTIWTSG SSISFCGVNS 450 DTVGWSWPDG AELPFTIDK (SEQ ID NO: 39) 469 |

| **Virus 2** |
|---|
| MNPNQKIITI GSVCMTIGMA NLILQIGNII SIWISHSIQL GNQNQIETCN |
| QSVITYENNT WVNQTYVNIS NTNFAAGQSV VSVKLAGNSS LCPVSGWAIY SKDNS[?]RIGS KGDVFVIREP FISCSPLECR TFFLTQGALL NDKHSNGTIK DRSPYRTLMS CPIGEVPSPY NSRFESVAWS ASACHDGINW LTIGISGPDN GAVAVLKYNG IITDTIKSWR NNILRTQESE CACVNGSCFT VMTDGPS[?]GQ ASYKIFRIEK GKIVKSVEMN APNY[?]YEECS CYPDSSEITC VCRDNWHGSN RPWVSFNQNL EYQIGYICSG IFGDNPRPND KTGSCGPVSS NGANGVKGFS FKYGNGVWIG RTKSISSRNG FEMIWDPNGW TGTDNNFSIK QDIVGINEWS GYSGSFVQHP ELTGLDCIRP CFWVELIRGR PKENTIWTSG SSISFCGVNS DTVGWSWPDG AELPFTIDK (SEQ ID NO: 40) |

| **Virus 3** |
|---|
| MNPNQKIITI GSVCMTIGMA NLMLQIGNII SIWISHSIQL GNQNQIETCN QSVITYENNT WVNQTYVNIS NTNFAAGQSV VSVKLAGNSS LCPVSGWAIY SKDNS[?]RIGS KGDVFVIREP FISCSPLECR TFFLTQGALL NDKHSNGTIK DRSPYRTLMS CPIGEVPSPY NSRFESVAWS ASACHDGINW LTIGISGPDN GAVAVLKYNG IITDTIKSWR NNILRTQESE CACVNGSCFT VMTDGPS[?]EQ ASYKIFRIEK GKIVKSVEMN APNY[?]YEECS CYPDSSEITC VCRDNWHGSN RPWVSFNQNL EYQIGYICSG IFGDNPRPND KTGSCGPVSS NGANGVKGFS FKYGNGVWIG RTKSISSRNG FEMIWDPNGW TGTDNNFSIK QDIVGINEWS GYSGSFVQHP ELTGLDCIRP CFWVELIRGR PKENTIWTSG SSISFCGVNS DTVGWSWPDG AELPFTIDK (SEQ ID NO: 41) |

| **Virus 4** |
|---|
| MNPNQKIITI GSVCMTIGMA NLILQIGNII SIWISHSIQL GNQNQIETCN QSVITYENNT WVNQTYVNIS NTNFAAGQSV VSVKLAGNSS LCPVSGWAIY |
| SKDNS[?]RIGS KGDVFVIREP FISCSPLECR TFFLTQGALL NDKHSNGTIK DRSPYRTLMS CPIGEVPSPY NSRFESVAWS ASACHDGINW LTIGISGPDN GAVAVLKYNG IITDTIKSWR NNILRTQESE CACVNGSCFT VMTDGPS[?]GQ ASYKIFRIEK GKIVKSVEMN APNY[?]YEECS CYPDSSEITC VCRDNWHGSN RPWVSFNQNL EYQIGYICSG IFGDNPRPND KTGSCGPVSS NGANGVKGFS FKYGNGVWIG RTKSISSRNG FEMIWDPNGW TGTDNNFSIK QDIVGINEWS GYSGSFVQHP ELTGLDCIRP CFWVELIRGR PKENTIWTSG SSISFCGVNS DTVGWSWPDG AELPFTIDK (SEQ ID NO: 42) |

Viruses 1, 2 and 3 carry a mutation A198P in the M1 region (segment 7) relative to wild type A/California/07/2009 Sequence. Further, these three isolates lack a residue corresponding to D34 in the M2 region.

This mutation has been reported to increase cell-mediated immunity (but not protect against infection). The mutated region has 60% sequence similarity with influenza B virus MA region and vaccines generated against viral isolates carrying this mutation may generate immunity against influenza B.

Further, the M1 mutation may be used as therapeutic targets for viral clearance and recovery.

| **Segment 7 - MA** |
|---|
| **A/California/07/2009 Sequence** |
| **M1** |
| MSLLTEVETY VLSIIPSGPL KAEIAQRLES VFAGKNTDLE ALMEWLKTRP 050 ILSPLTKGIL GFVFTLTVPS ERGLQRRRFV QNALNGNGDP NNMDRAVKLY 100 KKLKREITFH GAKEVSLSYS TGALASCMGL IYNRMGTVTT EAAFGLVCAT 150 CEQIADSQHR SHRQMATTTN PLIRHENRMV LASTTAKAME QMAGSSEQ[?]A 200 EAMEVANQTR QMVHAMRTIG THPSSSAGLK DDLLENLQAY QKRMGVQMQR 250 FK (SEQ ID NO: 43) 252 |

| **M2** |
|---|
| MSLLTEVETP TRSEWECRCS DSS[?]PLVIAA NIIGILHLIL WITDRLFFKC 50 IYRRFKYGLK RGPSTEGVPE SMREEYQQEQ QSAVDVDDGH FVNIELE 97 (SEQ ID NO: 44) |

| **Virus 1** |
|---|
| **M1** |
| MSLLTEVETY VLSIIPSGPL KAEIAQRLES VFAGKNTDLE ALMEWLKTRP 050 ILSPLTKGIL GFVFTLTVPS ERGLQRRRFV QNALNGNGDP NNMDRAVKLY 100 KKLKREITFH GAKEVSLSYS TGALASCMGL IYNRMGTVTT EAAFGLVCAT 150 CEQIADSQHR SHRQMATTTN PLIRHENRMV LASTTAKAME QMAGSSEQ[?]A 200 EAMEVANQTR QMVHAMRTIG THPSSSAGLK DDLLENLQAY QKRMGVQMQR 250 FK (SEQ ID NO: 45) 252 |

| **M2** |
|---|
| MSLLTEVETP TRSEWECRCS DSS[?]PLVIAA NIIGILHLIL WITDRLFFKC 50 |
| IYRRFKYGLK RGPSTEGVPE SMREEYQQEQ QSAVDVDDGH FVNIELE 96 (SEQ ID NO: 46) |

| **Virus 2** |
|---|
| **M1** |
| MSLLTEVETY VLSIIPSGPL KAEIAQRLES VFAGKNTDLE ALMEWLKTRP 050 ILSPLTKGIL GFVFTLTVPS ERGLQRRRFV QNALNGNGDP NNMDRAVKLY 100 KKLKREITFH GAKEVSLSYS TGALASCMGL IYNRMGTVTT EAAFGLVCAT 150 CEQIADSQHR SHRQMATTTN PLIRHENRMV LASTTAKAME QMAGSSEQ[?]A 200 EAMEVANQTR QMVHAMRTIG THPSSSAGLK DDLLENLQAY QKRMGVQMQR 250 FK (SEQ ID NO: 47) 252 |

| **M2** |
|---|
| MSLLTEVETP TRSEWECRCS DSS[?]PLVIAA NIIGILHLIL WITDRLFFKC 50 IYRRFKYGLK RGPSTEGVPE SMREEYQQEQ QSAVDVDDGH FVNIELE 96 (SEQ ID NO: 48) |

| **Virus 3** |
|---|
| **M1** |
| MSLLTEVETY VLSIIPSGPL KAEIAQRLES VFAGKNTDLE ALMEWLKTRP 050 ILSPLTKGIL GFVFTLTVPS ERGLQRRRFV QNALNGNGDP NNMDRAVKLY 100 KKLKREITFH GAKEVSLSYS TGALASCMGL IYNRMGTVTT EAAFGLVCAT 150 CEQIADSQHR SHRQMATTTN PLIRHENRMV LASTTAKAME QMAGSSEQ[?]A 200 EAMEVANQTR QMVHAMRTIG THPSSSAGLK DDLLENLQAY QKRMGVQMQR 250 FK (SEQ ID NO: 49) 252 |

| **M2** |
|---|
| MSLLTEVETP TRSEWECRCS DSS[?]PLVIAA NIIGILHLIL WITDRLFFKC 50 IYRRFKYGLK RGPSTEGVPE SMREEYQQEQ QSAVDVDDGH FVNIELE 96 (SEQ ID NO: 50) |

| **Virus 4** |
|---|
| **M1** |
| MSLLTEVETY VLSIIPSGPL KAEIAQRLES VFAGKNTDLE ALMEWLKTRP 050 ILSPLTKGIL GFVFTLTVPS ERGLQRRRFV QNALNGNGDP NNMDRAVKLY 100 KKLKREITFH GAKEVSLSYS TGALASCMGL IYNRMGTVTT EAAFGLVCAT 150 CEQIADSQHR SHRQMATTTN PLIRHENRMV LASTTAKAME QMAGSSEQ[?]A 200 EAMEVANQTR QMVHAMRTIG THPSSSAGLK DDLLENLQAY QKRMGVQMQR 250 FK (SEQ ID NO: 51) 252 |

| **M2** |
|---|
| MSLLTEVETP TRSEWECRCS DSS[?]PLVIAA NIIGILHLIL WITDRLFFKC 50 IYRRFKYGLK RGPSTEGVPE SMREEYQQEQ QSAVDVDDGH FVNIELE 97 (SEQ ID NO: 52) |

The methods of the invention use the herein disclosed influenza virus antigens to immunize against influenza virus infection. The specific virus from which the antigens are derived may be the same as or different from the specific virus for which protection is being provided, because cross-protection between different isolates is known to occur with influenza viruses, particularly within the same viral subtypes.

The influenza vaccines currently in use are designated whole virus (WV) vaccine or subvirion (SV) (also called "split" or "purified surface antigen"). The WV vaccine contains intact, inactivated virus, whereas the SV vaccine contains purified virus disrupted with detergents that solubilize the lipid-containing viral envelope, followed by chemical inactivation of residual virus. Attenuated viral vaccines against influenza are also in development. A discussion of methods of preparing conventional vaccine may be found in Wright, P. F. & Webster, R. G., FIELDS VIROLOGY, 4d Ed. (Knipe, D. M. et al. Ed.), 1464-65 (2001),

Where a vaccine includes more than one strain of influenza, the different strains are typically grown separately and are mixed after the viruses have been harvested and antigens have been prepared. Alternatively, different segments of different isolates of the influenza virus may be combined to generate a multipotent vaccine. The influenza virus(es) used in the processes of the invention may be reassortant strains, and/or may have been obtained by reverse genetics techniques. The virus(es) may be attenuated. The virus(es) may be temperature-sensitive. The virus(es) may be cold-adapted. A reassortant strain including the HA and/or NA viral segments from a pathogenic strain and the remaining six or seven segments from a non-pathogenic strain may be used.

The influenza virus antigen used in the immunogenic composition according to the invention may be in the form of a live virus or, preferably, an inactivated virus. Virus inactivation typically involves treatment with a chemical such as formalin or β-propiolactone. Where an inactivated virus is used, the antigen may be a whole virus, a split virus, or viral subunits. Split viruses are obtained by treating virions with detergents (e.g. ethyl ether, polysorbate 80, deoxycholate, tri-N-butyl phosphate, Triton X-100, Triton N101, cetyltrimethylammonium bromide, etc.) to produce subvirion preparations. Subunit vaccines comprise one or both of the influenza surface antigens hemagglutinin and neuraminidase. Influenza antigens can also be presented in the form of virosomes.

Where an antigen is prepared from an influenza virus (i.e. rather than having been produced in a recombinant or synthetic system that does not involve growth of influenza viruses), the virus may be grown either on eggs or in cell culture. Growth in specific pathogen free embryonated eggs is the traditional route by which influenza viruses have been grown for vaccine production, and cell culture is a more recent development. Where cell culture is used then the influenza virus vaccine will typically be grown on mammalian cells, such as MDCK cells, Vero cells or PER.C6 cells. These cell lines are widely available e.g. from the American Type Cell Culture (ATCC) collection, or from the Coriell Cell Repositories. For example, the ATCC supplies various different Vero cells under catalog numbers CCL-81, CCL-81.2, CRL-1586 and CRL-1587, and it supplies MDCK cells under catalog number CCL-34. Growth on avian cell lines, including cell lines derived from hen, e.g. chicken embryo fibroblasts (CEF), is also possible.

Immunogenic and medicament compositions of the invention are suitable for administration to a patient. This can be achieved by various ways, including but not limited to: intradermal injection; transdermal administration; and topical administration. These may be used in conjunction with skin abrasion e.g. by emery paper or by the use of microabrasives.

Immunogenic and medicament compositions of the invention are preferably presented as vaccines.

Compositions of the invention may include an adjuvant. Adjuvants that have been used in influenza vaccines include aluminium salts, chitosan, CpG oligodeoxynucleotides such as CpG 7909, oil-in-water emulsions such as MF59, water-in-oil-in-water emulsions, E.coli heat labile toxin and its detoxified mutants, monophosphoryl lipid A and its 3-*O*-deacylated derivative, pertussis toxin mutants, muramyl dipeptides, etc.

Hemagglutinin (HA) is the main immunogen in inactivated influenza vaccines, and vaccines doses are standardised by reference to HA levels, typically as measured by a single radial immunodiffusion (SRID) assay. Vaccines for intramuscular injection typically contain about 15 µg of HA per strain, although lower doses are also used (e.g. for children, or in pandemic situations) and fractional doses such as 1/2 (i.e. 7.5 µg HA per strain), 1/4 and 1/8 have been used. Compositions of invention will typically include between 0.1 and 8 µg of HA per influenza strain, preferably e.g. about 7.5, about 5, about 3, about 2.5, about 2, about 1.5, about 1, about 0.75, about 0.5, about 0.4, about 0.2µg, etc.

Vaccines for intramuscular injection typically have a volume of 0.5 ml. The compositions may include preservatives such as thiomersal or 2-phenoxyethanol. It is preferred, however, that the compositions should be substantially free from (i.e. less than I µg/ml) mercurial material e.g. thiomersal-free. Vaccines containing no mercury are more preferred.

Where influenza virus has been grown on cell culture then compositions of the invention preferably contain less than 100 pg of residual host cell DNA per dose, although trace amounts of host cell DNA may be present. Contaminating DNA can be removed during vaccine preparation using standard purification procedures e.g. chromatography, etc. Removal of residual host cell DNA can be enhanced by nuclease treatment e.g. by using the Benzonase® DNase. Vaccines containing <100 pg -host cell DNA per 10 µg of hemagglutinin are preferred, as are vaccines containing <100 pg host cell DNA per 0.25 ml volume. Vaccines containing <100 pg host cell DNA per 50 µg of hemagglutinin are more preferred, as are vaccines containing <100 pg host cell DNA per ml volume.

The vaccines of the invention may be delivered as a single dose immunization regimen. Alternatively, they may be delivered as the prime element of a prime--boost regimen (meaning that the first immunization is followed by a second shot of similar antigenicity within a few weeks or months).

Methods for testing the immunogenicity of influenza vaccines are well known in the art. One method involves the following procedure: (a) Just prior to vaccination, a 10 ml venous blood sample is taken from a patient, normally from the arm, for base-line titration of circulating anti-HA antibodies; (b) Immediately thereafter, a patient receives I dose of vaccine which, if administered to the arm, shall be given into the opposite arm from which blood was drawn; (c) Approximately 3 weeks after vaccination, a 10 ml blood sample shall be taken from patients. Sera are separated from the blood samples and stored (if necessary) at -20° C. Sera are assayed for anti-hemagglutinin antibody against the relevant strains, by hemagglutination inhibition (HI) or single radial hemolysis (SRH). Positive and negative sera as well as reference preparations can be obtained from public reference laboratories. Antibody titrations are performed in duplicate, and pre-and post-vaccination sera are titrated simultaneously. The titter assigned to each sample is the geometric mean of two independent determinations (but, for the purposes of calculation, any HI result <10 0 (=undetectable) is expressed as 5 and any negative SRH result is expressed as 4 mm² under standard conditions).

In HI tests, seroconversion corresponds to a ratio of pre- and post-immunization titers of ≧40 and a significant (e.g. at least 4-fold) increase in antibody titer. In SRH tests, seroconversion corresponds to a post-vaccination area ≧25 mm², with at least a 50% in area relative to the pre-vaccination area.

### EXAMPLES

Without intent to limit the scope of the invention, exemplary instruments, apparatus, methods and their related results according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

### EXAMPLE 1: Cell line

VERO cells is a quite well studied cell line, first isolated from African green monkey kidney (*Cercopithecus aethiops*), it has been widely used in microbiology, cell biology and virology studies. It has been licensed in USA for viral vaccine production (rotavirus and polivirus), also it has been tested and in some cases approved for vaccine production against Rabies, reovirus and Japanese encephalitis. We have chosen this cell line for its susceptibility to co-infection, as well as for its ability to grow at industrial scale in fermentors or bioreactors, which are desirable characteristics for vaccine production.

The Vero cell line used, was purchased from ATCC, and kept at -70 °C with Dimetil Sulfoxid (DMSO). Cells were unfrozen according to CORNING instructions.

### EXAMPLE 2: Sterile conditions

All our facilities at BSLIII are carefully treated and monitored to avoid contamination, on regular basis, room, material, reagents, and equipment sterility tests are performed. Cell culture area is regularly cleaned and disinfected every month, and several rounds of disinfection are performed every 24, 48 and 72 hours, using Benzal, 70% ethanol and 5% chlorine. Sterility tests are performed at the end of these rounds, also, all reagents are tested for contamination by culturing an aliquot in thioglycolate liquid media.

### EXAMPLE 3: Cell propagation

Vero cell propagation was carried out under appropriate physical and chemical conditions such as storage in polystyrene cell culture flasks (25 cm², 50cm² and 75cm²). These flasks are also Gamma irradiated in order to reduce hydrophobicity. These containers allow an efficient cell adhesion and therefore monolayer formation. To increase cell proliferation, growth media M199 or DMEM (Dulbecco's Modified Medium) was used and supplemented with 10% bovine fetal serum (BFS) (SIGMA-Aldrich); maintenance of physiological pH was monitored by using Phenol Red. An antimicrobial mixture (penicillin, streptomycin, and amphotericin B) was added, also, in order to inhibit fungal contamination anti PPLO was added.

Cell cultures were incubated at 35°C / 5% CO₂ / 5% humidity. Cell quality and growth was monitored by phase contrast microscopy, this allow us to discard those cultures presenting bacterial or fungal contamination.

Cell subculture of flasks presenting complete confluence is performed as following:

1. Remove and discard culture medium.

2. Briefly rinse the cell layer with 0.25% (w/v) Trypsin- 0.53 mM EDTA solution to remove all traces of serum that contains trypsin inhibitor.

3. Add 2.0 to 3.0 ml of Trypsin-EDTA solution to flask and observe cells under an inverted microscope until cell layer is dispersed (usually within 5 to 15 minutes).

4. Add 6.0 to 8.0 ml of complete growth medium and aspirate cells by gently pipetting.

5. Add appropriate aliquots of the cell suspension to new culture vessels.

6. Incubate cultures at 37°C.

When 80% of confluence was observed, cells were inoculated with 1x10⁶ p AHN1 virus. This level of confluence was optimal for observation of the cytopathogenic effect caused by the influenza virus A/H1N1.

### EXAMPLE 4: Viral Isolation

Viral isolation was done in 25cm² flasks inoculated with VERO cells (80% confluence), for viral isolation, growth media was removed, cells were centrifuged and resuspended three times in order to discard cell debris. From a swab sample obtained from a patient clinically diagnosed with influenza AH1N1 and confirmed by real time PCR, 50 µl were inoculated in the cell culture and incubated for 30 minutes to allow viral attachment to cell surface. After this, M199 supplemented with BFS 1% and incubated at 40°C/40 min.

Inoculated cultures were monitored for presence of cell cytopathic effect by phase contrast microscopy. Simultaneously, viral load was determined by real time PCR. When 1x10⁶ was reached, cultures were frozen at -80°C (DMSO 10%) and stored for further studies.

### EXAMPLE 5: Viral infection confirmation and quantification by real time-RT-PCR.

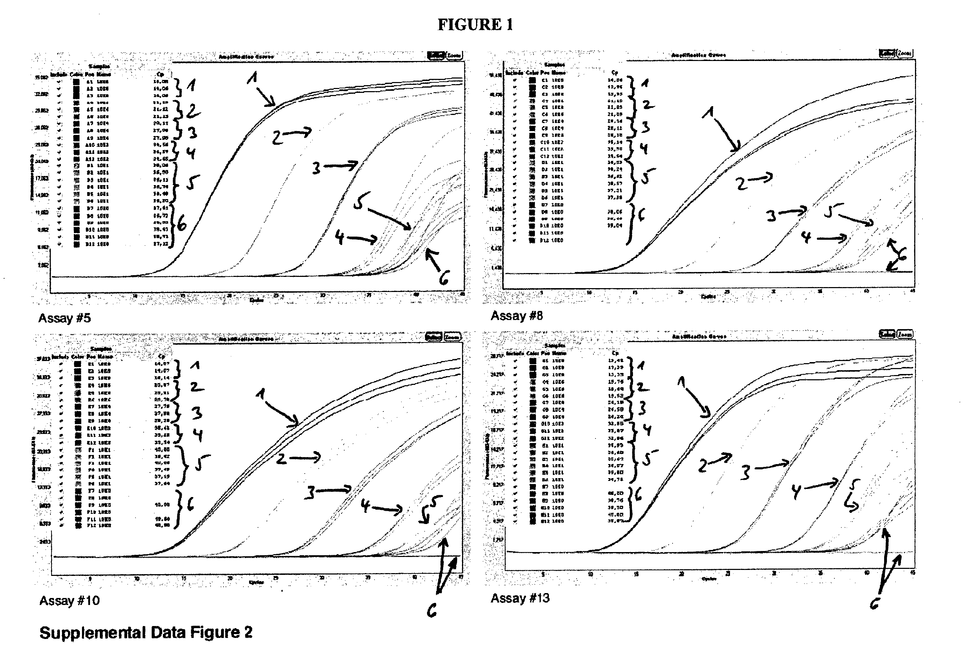

Viral RNA was extracted from VERO cell cultures by using a commercial kit (ROCHE) as described before, after this, RNA was quantified by spectophotometry using a NanoDrop equipment. CDC protocol for AH1N1 detection by Real Time PCR was performed in a Light Cycler 480 thermocycler (ROCHE), including a standard for Influenza in order to quantify (101 a 106 copies of H1 gene cloned in a plasmid). Viral Load determination was based by calculating crossing point format for each sample. Figure 1 shows examples of viral load quantification.

### EXAMPLE 6: Sample selection

A total of 193 out of 3760 positive samples for pAH1N1 for sequencing based on clinical and epidemiological data. Clinical criteria for selection was: severity of infection, drug resistance and atypical clinical setting. Epidemiological criteria was based on location, gender and age. Samples chosen were collected during the 4 outbreak peaks reported from March to November 2009. Samples were resuspended in 2.5 mL of viral transport medium, 500 µl aliquots were dispensed and stored at -70 °C.

### EXAMPLE 7: Nucleic Acid Extraction

Total nucleic acids of clinical samples were isolated and resuspended in d.i. water (100 µ) using MagNA Pure LC Total Nucleic Acid Isolation Kit (Roche) on MagNA Pure LC instrument (Roche) by the External Lysis protocol according to the manufacturer's instructions, in brief: 500 µL of sample was placed in the MagnaPure nucleic acid cartridge and one volume of lysis buffer was added, after this, all parameters were set and initiated. RNA extracted was placed at -70°C for further analysis.

### EXAMPLE 8: Influenza p/AH1N1 real time PCR detection

Detection of pandemic Influenza AH1N1/09 was performed by two methods: Real-time ready Influenza A/H1N1 Detection Set (Roche), using the Real-time ready RNA Virus Master kit (Roche). Thermal cycling was performed on Light Cycler 2.0 instruments (Roche) under the following conditions: 30 min at 50 °C; 15 min at 95 °C; 45 cycles of 15 s at 94 °C; and 30 s at 60 °C.

The second method was the CDC protocol of real-time RT-PCR for influenza A (H1N1), using primers and probes described in the above protocol and one step RT-PCR Invitrogen SuperScript™III Platinum® One-Step Quantitative Kit. Thermal cycling was on Light Cycler 2.0 instruments (Roche) under the following conditions: 50 min at 50 °C; 2 min at 95 °C; 45 cycles of 15 s at 95 °C; and 30 s at 55 °C.

All reactions in both methods were performed according to the manufacturer's instructions using 5 µL of total RNA in a 20 µL total reaction volume. In both detection methods an internal control (human Myostatin gene for Roche method and RnaseP for CDC protocol) was included.

Each sample was tested and real time PCR separately using the following probes: Influenza A (InfA), universal swine (SwFlu A), H 1 swine (SwH1) and an internal control for human nucleic acid RNaseP (RP). Positive and negative controls were introduced.

Reaction mixture contained 0.8 µM of each primer, 0.2 µM of each probe, 1X PCR buffer and 0.5 U of SuperScript III/Platinum Taq mix (Invitrogen).

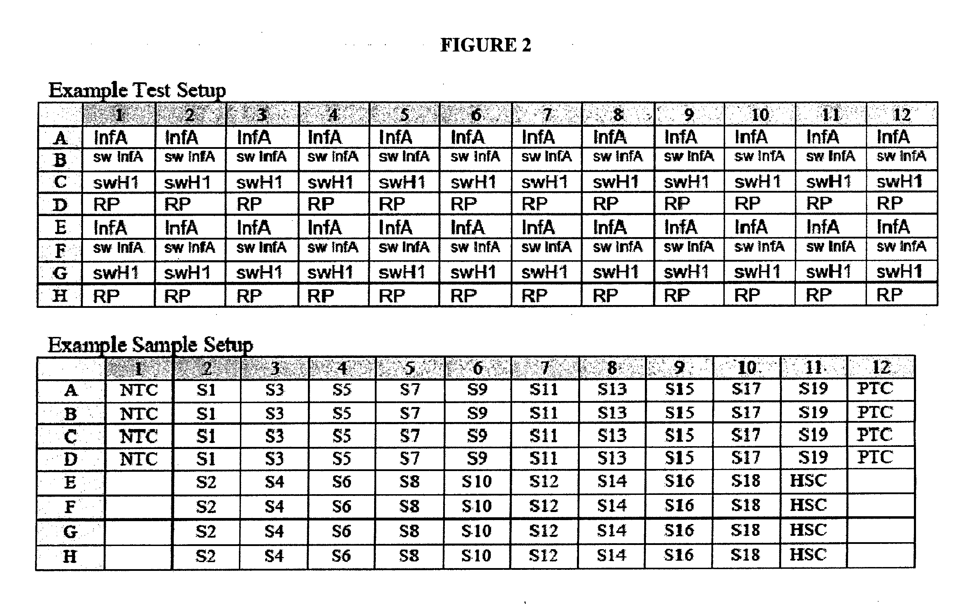

An example of sample arrangement is shown in Figure 2.

Amplification conditions were as follows:

Reverse transcription - 50°C / 30 min

Inactivation - 15°C / 2 min

Extension - 95°C / 15 cycles and 55°C / 30 cycles.

### EXAMPLE 9: Data analysis

Results were analyzed according to WHO criteria as follows:

1.- No fluorescence should be present in the negative control.

2.- Experimental samples should present fluorescence for RP probe.

3.- Human nucleic acid control should not emit fluorescence for InfA, SwFluA and SwH1 probes.

4.- Positive control (A/H1N1 nucleic acid) should emit fluorescence for all primers and probes.

5.- If all criteria are met, the sample is considered positive.

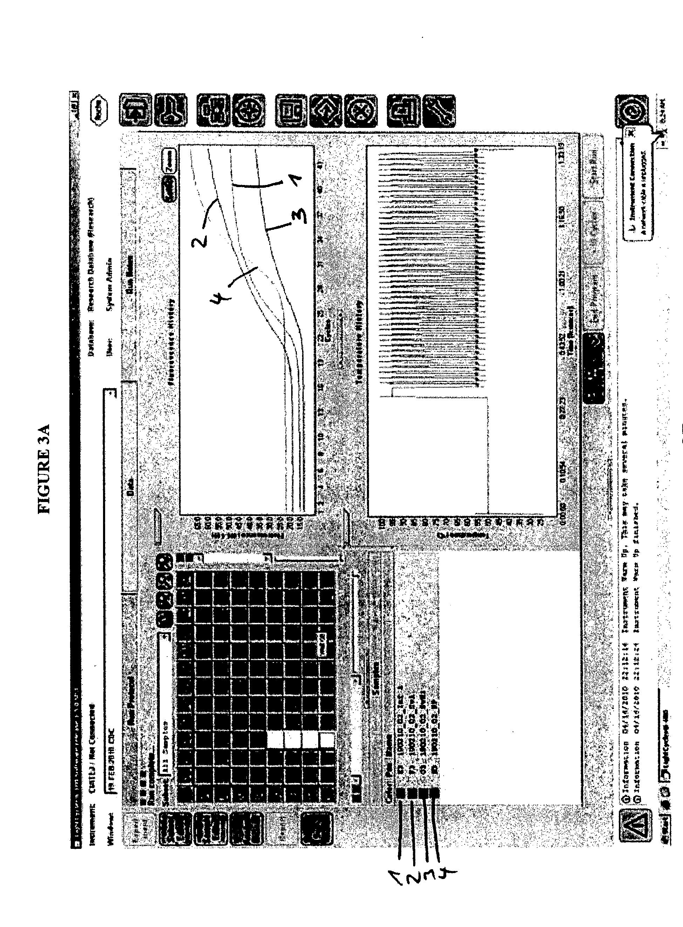

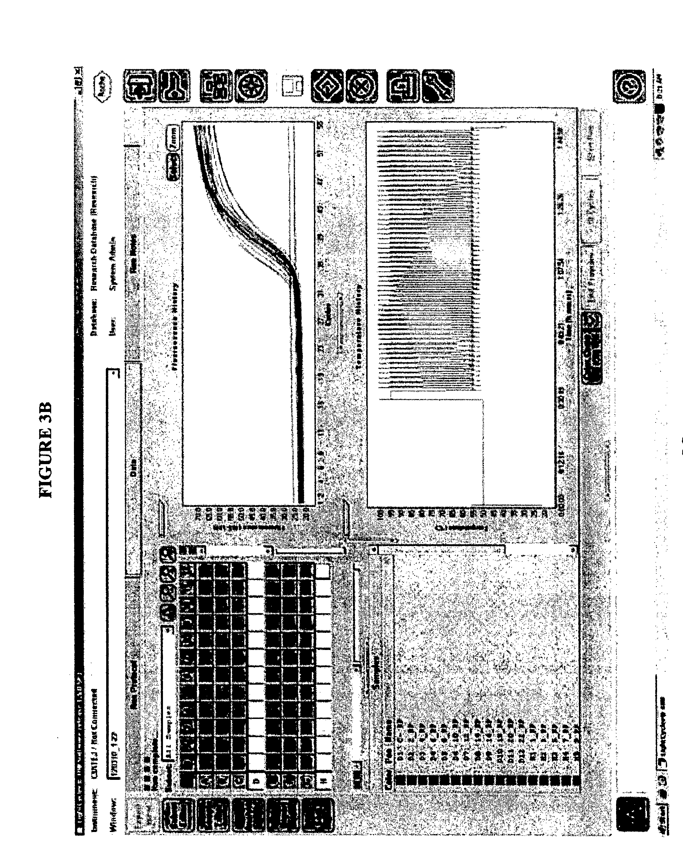

Figures 3A and 3B show graphical views of Light Cycler 2.0 real time thermocycler interface showing fluorescence curves. Fig. 3A) Clinical Sample. Fig. 3B) RNAseP (RP) detection.

### EXAMPLE 10: Sequencing

A total of 950 viruses were isolated by cell culture, from which, 193 Neuraminidase (NA) and 197 Hemagglutinin (HA) partial gene sequences were obtained through capillary sequencing in an ABI 3100 sequencing machine (Applied Biosystems). Since HA and NA are the most variable sequences in AH1N1, an *in silico* analysis (phylogenetic analysis and BLAST) showed that all 386 (HA and NA sequences) could be grouped in 20 clusters, therefore; the complete genome of one representative member of each cluster was pyrosequenced in a 454 Titanium Roche Pyrosequencing equipment (ROCHE).

### EXAMPLE 11: Propagation of influenza virus in chicken embryo

Several attempts were made to propagate the proposed candidates for the influenza vaccine in VERO cells. However, these attempts initially failed because the amount of virus recovered was very low even after 3 passages. Considering this event, one or two passages in SPF (Specific Pathogen Free) chicken embryos were performed. These were inoculated into the amniotic fluid with 0.1 mL of each candidate. 40 other viruses previously isolated during the flu pandemic period were also included, and inoculation was carried out using two eggs for each virus.

After three days at 35°C the amniotic fluid was harvested from each infected embryo, the liquid collected was centrifuged at 3000xg in order to remove cellular debris and the supernatant was analyzed by qRT-PCR and stored at -80°C. All procedures were performed into the BSL III following the standards needed to avoid biosecurity risks.

The viral load after the passage into the embryo was very high for three strains, one of them was the viral candidate. Ten more isolated strains also were detected by the RT-PCR test but showed low viral loads. Therefore they were inoculated again in another SPF chicken embryo but, in this case the inoculation was made into the allantoic fluid. After 72 hrs of incubation following the same conditions described above, the virus was harvested by collecting only the allantoic fluid. A smaller volume was analyzed by using RT-PCR and the rest was stored at -80°C. The results showed that six of the ten viral isolates were able to replicate in high efficiency showing high viral load.

### Certificate of the sequence from Karolinska Institute

Before chicken embryo propagation the viral strain cDNA was sent to the Karolinska Institute to verify the sequence by using a 454 pyrosequencer system ROCHE. The bioinformatics analysis confirmed the sequence previously determined in our laboratory.

### Master Seed Identification

The viral candidate for the influenza vaccine was named HZFLUJAL.

### Adaptation of the influenza virus to grow on VERO cell line

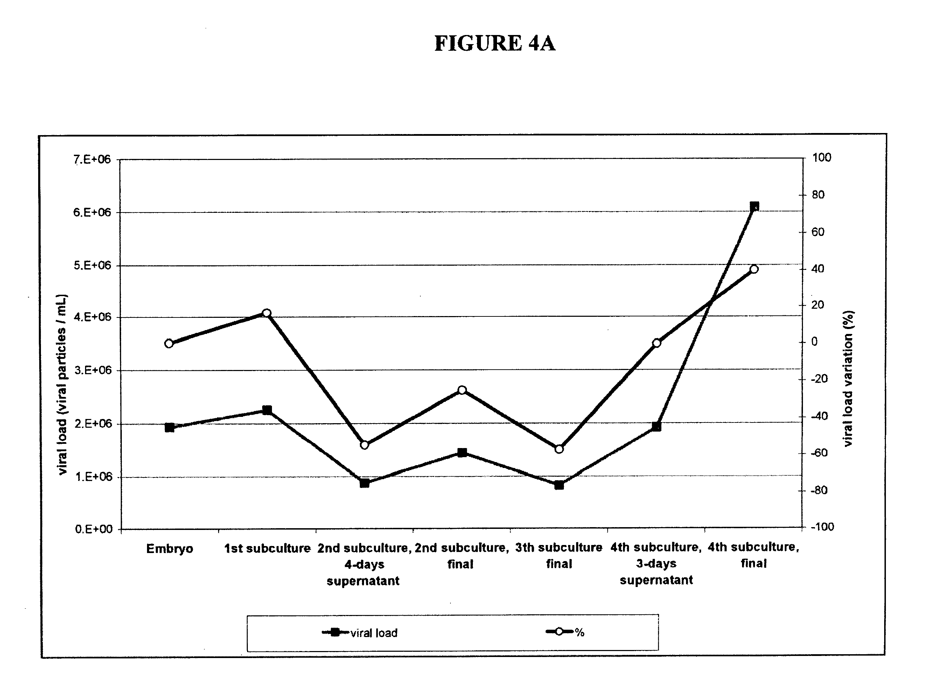

The virus candidate (HZFLUJAL) and two other viruses were harvested from embryo were inoculate on VERO cell line. In order to achieve the adaptation, several passages were performed, collecting the supernatant of the culture after 7 to 10 days of infection. Each supernatant was used to infect a new VERO cell passage. From each passage the viral load was quantified. Figure4A shows the differences among the viral load in the embryo and the respective subcultures of the same virus. In that figure it is clear that viral adaptation to this tissue cell line could be obtained until the four passages.

### Adaptation of the influenza virus to grow on MDCK cell line

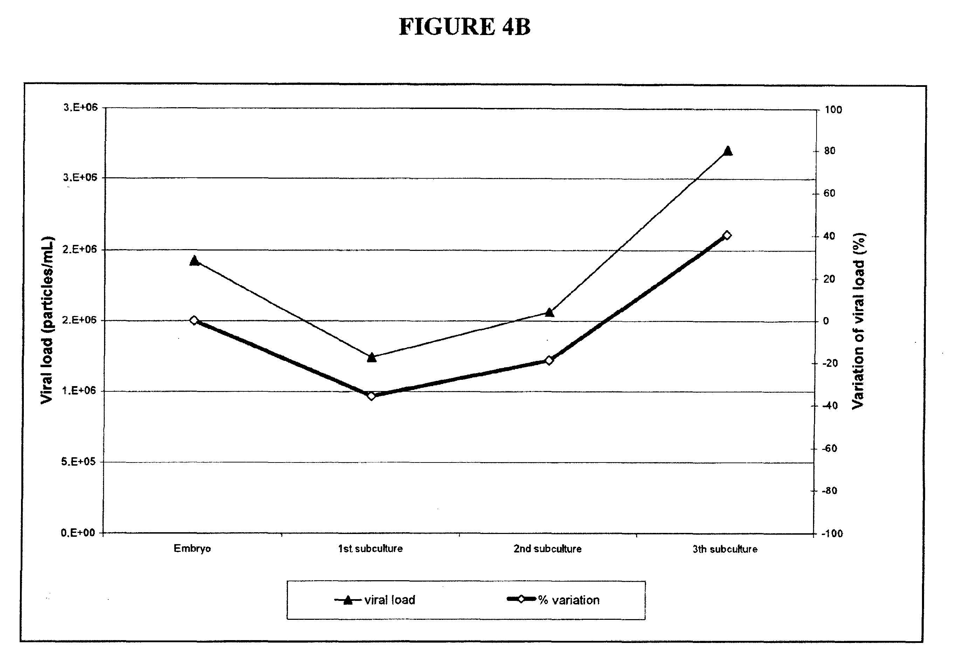

Not only was the VERO cell line evaluated as a substrate for influenza virus, but also MDCK cell line was used to propagate the virus. The same procedures applied to achieve the adaptation to the VERO cells were performed in MDCK cells. Preliminary results show a better adaptation to this cell line, because apparently only three passages were needed to get a viral load larger than that from chicken embryo (Figure 4B).

### Propagation of the virus to get the Master Seed HZFLUJAL

For the propagation of the virus previously adapted to grow on VERO cell line, one cryovial of the fourth passage on VERO cells was defrosted and was used to infect VERO cells on 175 cm² flasks. After 10 days at 33°C in a CO₂ incubator 120 mL of master seed was obtained, which showed a viral load larger than 10⁷ viral particles/mL (approximately 10^{7.7}). 120 cryovials with 1mL of master seed were obtained.

### Quality Control of the Master Seed

Sterility test and hemagglutination test were performed on the master seed. For the first one, 4 cryovials of master seed were defrosted, two were used to inoculate two flasks with 100mL of Fluid Thioglycollate Medium NIH, and the others two cryovials were employed to inoculate DMEM medium without antibiotics. Both media were incubated at 35 °C for 8 to 15 days. Neither growth nor change in the media was seen after the incubation period, therefore it was considered to be sterile (free of bacteria, fungus and protozoa).

Hemagglutination test was carried out to determine the title of hemagglutinin in the master seed. For this assay serial dilutions of 1:2 to 1:1024 of the master seed were made in PBS, pH=7.4, in a 96 well U-shaped microplate. The same volume of human O erythrocyte suspension 0.5% (v/v) in PBS was added to each dilution. The microplate was incubated 2 hours at 4°C, and the results showed a hemagglutination titer of 1:8.

### EXAMPLE 12: Pre-preclinical trials using HZFLUJAL vaccine

### Inactivation of virus for the pre-preclinical trials

For carry out the pre-preclinical trial, one cryovial of master seed was defrosted and used to infect a VERO culture on 80cm² flask after 7 days at 33°C in a CO₂ incubator. The culture was inactivated by formalin at a final concentration of 0.02% (v/v) for 18h at 35 °C. The formalin was eliminated by dialyzing against DMEM four times in 48h. After that, the hemagglutinin titer was quantified, which was once again 1:8. The inactivated viral suspension was adjusted to 10⁶ viral particles per 25µL and used as the vaccine in the groups below.

### Animal model for pre-preclinical trials

Female BALB/c mice from 7 to 8 week were employed for the pre-preclinical trials. Each group was put in a rack with cage Hepa filter covers. All animals were manipulated avoiding unnecessary suffering. When the protocol was completed all mice were euthanized.

### Seroconversion assays in pre-preclinical trials

Seroconversion assays were performed in nine groups of 5 mice each.

Group 1 vaccine with 10⁶ viral particles + transfer factor + sublingual administration

Group 2 vaccine with 10⁶ viral particles + transfer factor + intramuscular administration

Group 3 vaccine with 10⁶ viral particles+ sublingual administration

Group 4 vaccine with 10⁶ viral particles+ intramuscular administration

Group 5 commercial vaccine + sublingual administration

Group 6 commercial vaccine + intramuscular administration

Group 7 DMEM without Bovine fetal serum + sublingual administration

Group 8 DMEM without Bovine fetal serum + intramuscular administration

Group 9 without treatment

### Dose response assays in pre-preclinical trials

Dose response assays were performed in five groups of 3 mice each.

Group A Vaccine with 10⁷ viral particles + transfer factor + sublingual administration

Group B Vaccine with 10⁵ viral particles + transfer factor + sublingual administration

Group C Vaccine with 10⁴ viral particles + transfer factor + sublingual administration

Group D Vaccine with 10³ viral particles + transfer factor + sublingual administration

Group E Vaccine with 10² viral particles + transfer factor + sublingual administration

From the beginning of the immunization protocol all mice were maintained 4 day in an animal experimentation room which has the biosecurity requirements for carrying out the immunological assays. After the adaptation time clinical data such as weight and appearance were recorded, and a blood sample without anticoagulant was extracted from each mouse prior to the immunization. After that each group was inoculated by using the respective administration route. Four samples of blood were obtained from each mouse at 7, 14, 21 and 28 days post-immunization

Serum was obtained by centrifuging the blood samples, and was used for detecting IgG2 antibodies against Influenza A H 1N1 virus. In addition secretory samples were collected from the mice for detecting secreted immunoglobulin A (IgA).

### EXAMPLE 13: Detection of Antibodies against Influenza p/AH1N1 generated by CIATEJ/OPKO/Vaccine

An exemplary vaccine, described herein as the CIATEJ/OPKO/Vaccine, was developed in Vero cells, and tested to the ability to generate antibodies against pandemic influenza AH1N1. The antigen developed by CIATEJ/OPKO in Vero cells was able to induce antibody production when it was employed as vaccine in mice.

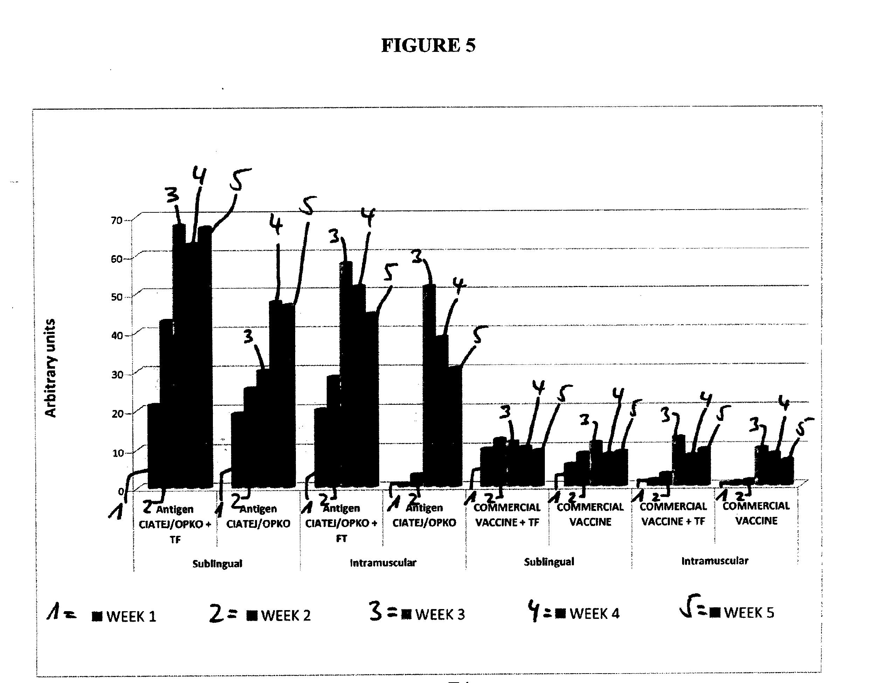

The titer of antibodies induced by the CIATEJ/OPKO antigen was larger than commercial vaccine. FIGURE 5 shows total antibodies against influenza p/AH1N1 detected by ELISA. The CIATEJ/OPKO/Vaccine was developed in Vero cells, TF = Transfer Factor. Immunization was carried out in 7 to 8 weeks old BALB/c female mice. The immunization was sublingual or intramuscular.

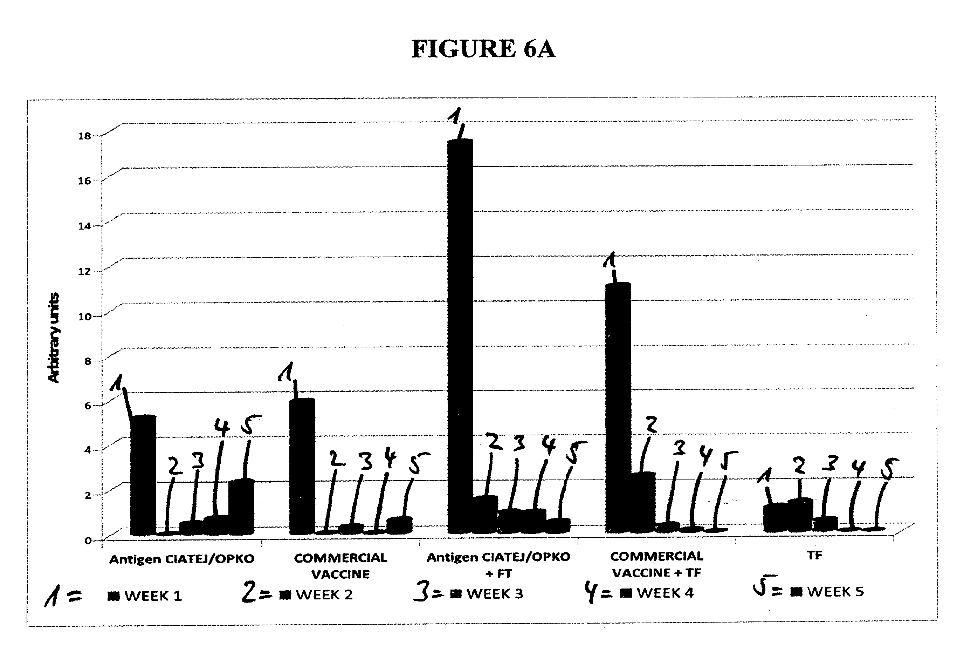

FIGURE 6A shows IgM antibodies against influenza p/AH1N1 detected in serum by ELISA. The CIATEJ/OPKO/Vaccine was developed in Vero cells, TF = Transfer Factor. Immunization was carried out in 7 to 8 weeks old BALB/c female mice. The immunization was intramuscular.

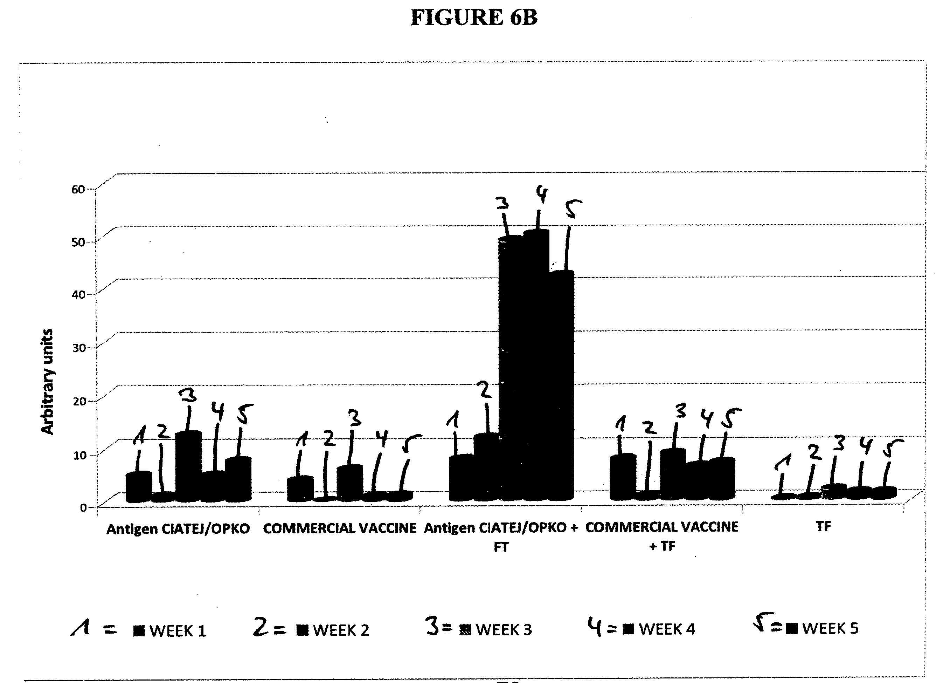

When transfer factor was added to the vaccine CIATEJ/OPKO, it increased the IgG antibody levels five-fold, as the same way transfer factor increased the IgM antibody titers three-fold during the first week of immunization. FIGURE 6B shows IgG antibodies against influenza p/AH1N1 detected in serum by ELISA. The CIATEJ/OPKO/Vaccine was developed in Vero cells, TF = Transfer Factor. Immunization was carried out in 7 to 8 weeks old BALB/c female mice. The immunization was intramuscular.

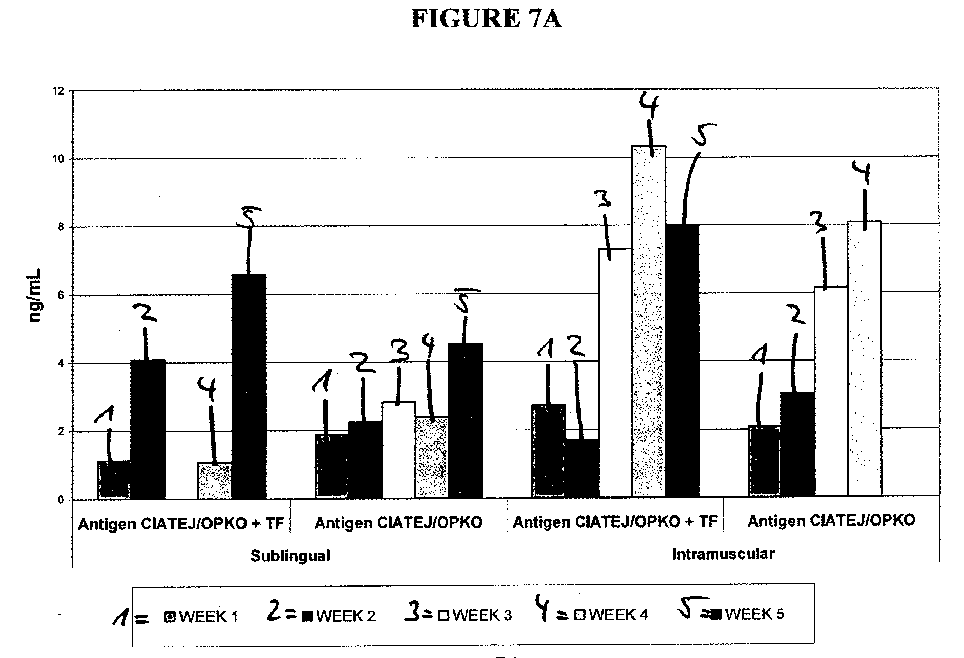

Intramuscular administration of the vaccine CIATEJ/OPKO showed larger induction of IgG_{2A} antibodies than sublingual administration. FIGURE 7A shows IgG_{2A} antibodies against Influenza p/AH1N1 detected in serum by ELISA. The CIATEJ/OPKO/Vaccine was developed in Vero cells, TF = Transfer Factor. Immunization was carried out in 7 to 8 weeks old BALB/c female mice. The immunization was sublingual or intramuscular.

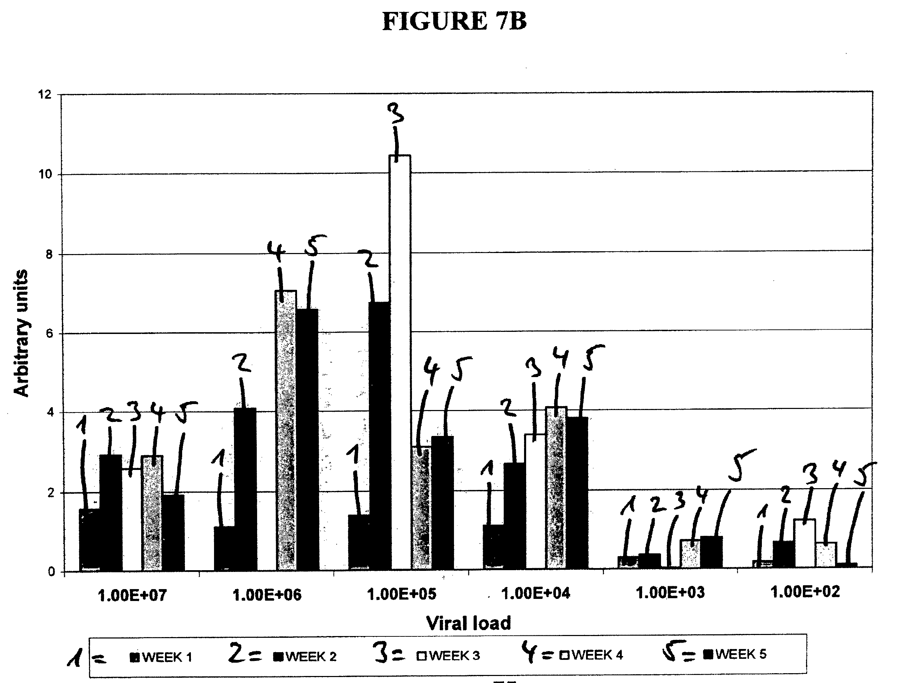

FIGURE 7B shows IgG_{2A} dose-response against Influenza p/AH1N1 detected in serum by ELISA. The CIATEJ/OPKO/Vaccine was developed in Vero cells, TF = Transfer Factor. Immunization was carried out in 7 to 8 weeks old BALB/c female mice. The immunization was sublingual.

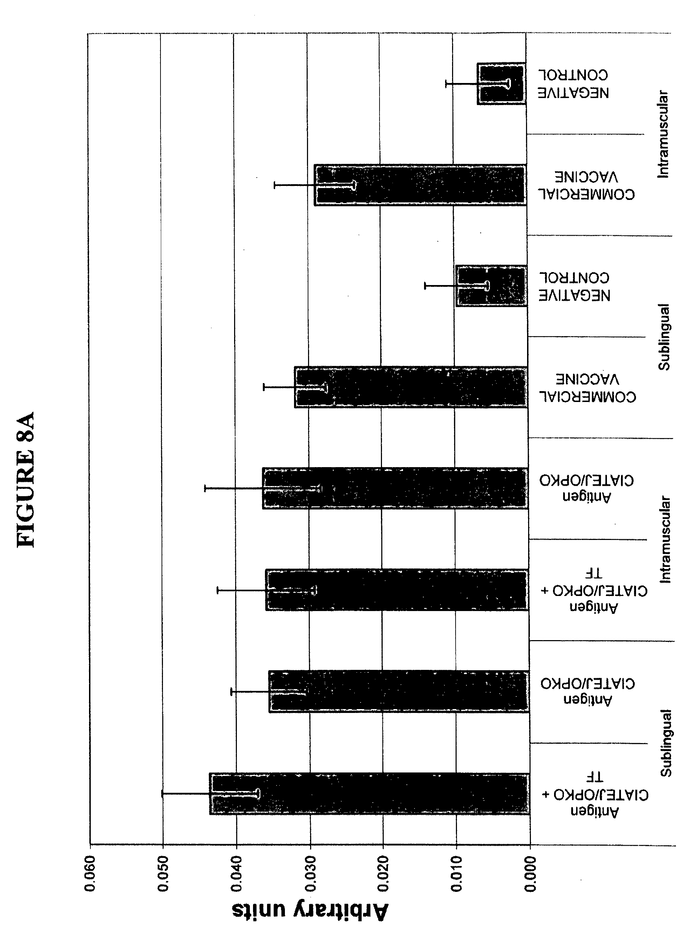

Intramuscular administration of the vaccine CIATEJ/OPKO showed induction of IgA antibodies at comparable levels to sublingual administration. FIGURE 8A shows IgA antibodies against Influenza p/AH1N1 detected in respiratory mucosa by ELISA. The CIATEJ/OPKO/Vaccine was developed in Vero cells, TF = Transfer Factor. Immunization was carried out in 7 to 8 weeks old BALB/c female mice. The immunization was sublingual or intramuscular (day 21 after immunization).

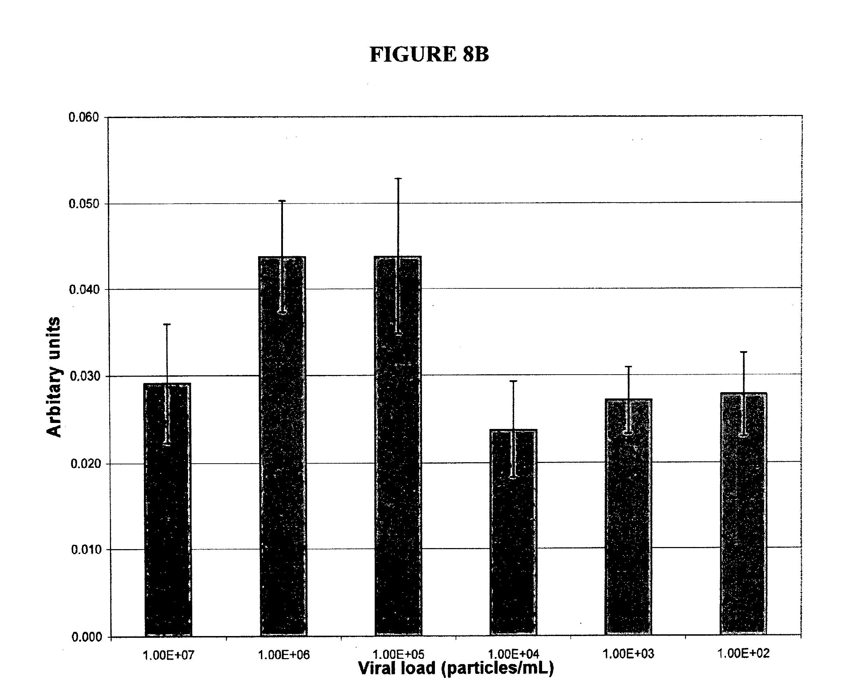

FIGURE 8B shows IgA dose-response against Influenza p/AH1N1 detected in respiratory mucosa by ELISA. The CIATEJ/OPKO/Vaccine was developed in Vero cells, TF = Transfer Factor. Immunization was carried out in 7 to 8 weeks old BALB/c female mice. The immunization was sublingual or intramuscular (day 21 after immunization).

### REFERENCES

Magna Pure LC Total Nucleic Acid Isolation Kit. Version December 2008. Kit for isolation of total viral nucleic acids from mammalian serum, plasma and whole blood. Cat. No.03 038 505 001.

SuperScript III Platinum One Step Quantitative RT-PCR System. Cat. No. 11738-088.

World Health Organization. CDC protocol of Real time for Influenza A (H1N1). April 29 2009.

CENAVECE-InDRE. Lineamientos para la red Nacional de laboratorios de salud pública e institutos nacionales de salud para vigilancia de influenza en Mexico 2009.

Ammerman N., Beier M., and Azad F. 2008 Growth and Maintenance of Vero Cell Lines. Curr Protoc Microbiol. Pp. 1-10.

Erlich H., Müller M., Helen M., Tambyah P., Joukhadar M., Montomoli M., Fisher D., Berezuk D., Fritsch M., Löw-Baselli A., Vartian N., Bobrovsky R., Borislava G., Pavlova, Pöllabauer E., Kistner O., and Barrett N. 2008 A Clinical Trial of a Whole-Virus H5N 1 Vaccine Derived from Cell Culture for the Baxter H5N1 Pandemic Influenza Vaccine Clinical Study Team. N. Engl. J. Med. 358;24. Pp. 2573-2584.

Instituto de Medicina Tropical "Pedro Kouri". 2003. Manual de procedimientos para el diagnostico de laboratorio de la infecciones respiratyorias agudas de etiologia viral. Organización panaméricana de la salud. Organización mundial de la salud.

Kistner O., Howard K., Spruth M., Wodal W., Brühl P., Gerencer M., Crowe B., Savidis H., Livey I., Reiter M., Mayerhofer I., Tauer C., Grillberger L., Mundt G., Falkner F., and P. Noel. 2007 Cell culture (Vero) derived whole virus (H5N1) vaccine based on wild-type virus strain induces cross-protective immune responses. Baxter AG, Biomedical Research Center, Uferstrasse, Austria. Vaccine. 2007 10; 25(32). P. 6028-6036.

Reina J., Fernández V., Blanco I. y Munar M. 1997. Comparison of Madin-Darby Canine Kidney Cells (MDCK) with a Green Monkey Continuous Cell Line (Vero) and Human Lung Embryonated Cells (MRC-5) in the Isolation of Influenza A Virus from Nasopharyngeal Aspirates by Shell Vial Culture. American Society for Microbiology Vol. 35, No. 7. P. 1900-1901.

Ryan John. Ph.D. Introduction to animal cell culture. Coming Incorporated Life Sciences 900 Chelmsford St. Lowell, Bulletin Technical. MA 01851. P. 1-24.

Ryan John. Ph.D. Subculturing Monolayer cell cultures Protocol Corning Incorporated Life Sciences 900 Chelmsford St. Lowell, MA 01851. P. 1-5. Sheets R. 2000 History and Characterization of the Vero Cell Line. Vaccines and Related Biological Products Advisory Committee Meeting to be held.

All publications and patent applications cited in this specification are incorporated herein by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claim.

## Claims

1. An influenza virus vaccine comprising a influenza virus which has at least:
a) a hemagglutinin (HA) gene segment comprising an amino acid sequence of SEQ ID NO: 34 derived from virus I isolate of pandemic influenza A H1/N1 influenza virus, and
b) a neuraminidase (NA) gene segment comprising an amino acid sequence of SEQ ID NO: 39 derived from virus I isolate of pandemic influenza A H1/N1 influenza virus.

2. The vaccine of claim 1, wherein the HA gene segment comprises a amino acid sequence encoded by the nucleotide sequence of segment 4 of virus 1 (SEQ ID NO: 4).

3. The vaccine of claim 1, wherein the HA gene segment comprises a clade 7 specific HA sequence of pandemic influenza A H1/N1 influenza virus.

4. The vaccine of claim 1, wherein the HA gene segment comprises a mutation at D222 residue of wild type pandemic influenza A H1/N1 influenza virus.

5. The vaccine of claim 4, wherein the HA gene segment comprises a D222E mutation.

6. The vaccine of claim 4, wherein the HA gene segment comprises a S203T mutation relative to wild type pandemic influenza A H1/N1 influenza virus.

7. The vaccine of claim 4, wherein the HA gene segment further comprises one or more of P83S and I321V mutations.

8. The vaccine of claim 1, wherein the NA gene segment comprises a amino acid sequence encoded by the nucleotide sequence of segment 6 of virus 1 (SEQ ID NO: 6).

9. The vaccine of claim 1, wherein the NA gene segment comprises a Y275H mutation relative to wild type pandemic influenza A H1/N1 influenza virus.

10. The vaccine of claim 9, wherein the NA gene segment further comprises one or more of I106V, D248N mutations.

11. The vaccine of claim 1, further comprising:
c) a matrix (MA) gene segment encoding an M1 peptide comprising an amino acid sequence of SEQ ID NO: 50 derived from virus 1 isolate of pandemic influenza A H1/N1 influenza virus.

12. The vaccine of claim 11, wherein the MA gene segment comprises a amino acid sequence encoded by the nucleotide sequence of segment 7 of virus 1 (SEQ ID NO: 7).

13. The vaccine of claim 11, wherein the matrix (MA) gene segment encoding an M2 peptide comprising an amino acid sequence of SEQ ID NO: 51 derived from virus 1 isolate of pandemic influenza A H1/N1 influenza virus.

14. The vaccine of claim 11, wherein the M1 peptide comprises a A198P mutation.

15. A method for immunizing a patient against influenza virus comprising the step of administering an influenza vaccine according to any of claims 1-14 to the patient.

16. A method for immunizing a patient against a hypervirulent influenza virus comprising the step of administering an influenza vaccine according to any of claims 1-14 to the patient.

17. A method for immunizing a patient against a oseltamivir-resistant influenza virus comprising the step of administering an influenza vaccine according to any of claims 1-14 to the patient.

18. A vaccine according to any one of claims I to 14, wherein the vaccine is a reassortant vaccine.

19. A vaccine according to any claim 18, in which the reassortant influenza virus is obtained by classical reassortment.

20. A vaccine according to any one of claims 1 to 14, which is a live attenuated vaccine or an inactivated vaccine.

21. A vaccine according to claim 20, which is formulated for oral or intranasal administration.

22. A method of preparing a vaccine for immunization of a subject against a hypervirulent influenza virus strain, comprising the step of mixing a reassortant influenza virus according to any one of claims 1 to 14 with a carrier, and optionally an adjuvant.
